# EUROPÄISCHE PATENTSCHRIFT

(11) **EP 3 345 983 B1**
(45) Veröffentlichungstag und Bekanntmachung des Hinweises auf die Patenterteilung: **26.08.2020**
(21) Anmeldenummer: 18156284.4
(22) Anmeldetag: 26.06.2008
(51) Int. Cl.: C09K 11/06, C07F 9/572, C07F 9/655, C07F 9/6553, C07F 9/6568, C07D 333/76, C07F 7/08, C07D 307/91

(54) **VERBINDUNGEN ENTHALTEND MINDESTENS EINE DISILYLVERBINDUNG AUSGEWÄHLT AUS DISILYLCARBAZOLEN, DISILYLDIBENZOFURANEN, DISILYLDIBENZOTHIOPHENEN, DISILYLDIBENZOPHOSPHOLEN, DISILYLDIBENZOTHIOPHEN-S-OXIDEN UND DISILYLDIBENZOTHIOPHEN-S, S-DIOXIDEN**
COMPOUNDS CONTAINING AT LEAST ONE DISILYL COMPOUND SELECTED FROM DISILYLCARBAZOLES, DISILYLDIBENZOFURANS, DISILYLDIBENZOTHIOPHENES, DISILYLDIBENZOPHOSPHOLES, DISILYLDIBENZOTHIOPHENE S-OXIDES AND DISILYLDIBENZOTHIOPHENE S, S-DIOXIDES
MATERIAUX CONTENANT AU MOINS UN COMPOSÉ DISILYLE SÉLECTIONNÉ PARMI LES DISILYLCARBAZOLES, LES DISILYLDIBENZOFURANNES, LES DISILYLDIBENZOTHIOPHÈNES, LES DISILYLDIBENZOPHOSPHOLES, LES DISILYLDIBENZOTHIOPHÈNE-S-OXYDES ET LES DISILYLDIBENZOTHIOPHÈNE-S,S-DIOXYDES

(30) Priorität: 05.07.2007 EP 07111824; 26.03.2008 EP 08153306
(43) Veröffentlichungstag der Anmeldung: 11.07.2018
(62) Teilanmeldung aus: 08774381.1
(73) Patentinhaber: UDC Ireland Limited, Ballycoolin, Dublin 15 (IE)
(72) Erfinder: LANGER, Nicolle, 64646 Heppenheim (DE); KAHLE, Klaus, 67069 Ludwigshafen (DE); LENNARTZ, Christian, 67105 Schifferstadt (DE); MOLT, Oliver, 69493 Hirschberg (DE); FUCHS, Evelyn, 68199 Mannheim (DE); RUDOLPH, Jens, 67547 Worms (DE); SCHILDKNECHT, Christian, 68305 Mannheim (DE); WATANABE, Soichi, 68161 Mannheim (DE); WAGENBLAST, Gerhard, 67157 Wachenheim (DE)
(74) Vertreter: Hansen, Norbert

(56) Entgegenhaltungen:
- WO-A-2007/142083
- US-A1- 2005 238 919
- MOHAMMED BOUACHRINE ET AL: "REACTIVITY OF SILYL MONOMERS FOR THE OXIDATIVE POLYMERIZATION OF PHENYLENE UNITS", PHOSPHORUS, SULFUR AND SILICON AND THE RELATED ELEMENTS, Bd. 152, Nr. 1, 1. September 1999 (1999-09-01), Seiten 265-278, XP055461262, US ISSN: 1042-6507, DOI: 10.1080/10426509908031635

## Beschreibung

Die vorliegende Erfindung betrifft eine Verbindung der allgemeinen Formel (I). Offenbart wird weiterhin eine organische Leuchtdiode, enthaltend eine Anode An und eine Kathode Ka und eine zwischen der Anode An und der Kathode Ka angeordnete Lichtemittierende Schicht E sowie gegebenenfalls mindestens eine weitere Schicht, wobei die Licht-emittierende Schicht E und/oder die mindestens eine weitere Schicht mindestens eine Verbindung ausgewählt aus Disilylcarbazolen, Disilyldibenzofuranen, Disilyldibenzothiophenen, Disilyldibenzophospholen, Disilyldibenzothiophen-S-oxiden und Disilyldibenzothiophen-S,S-dioxiden enthält, eine Licht-emittierende Schicht enthaltend mindestens eine der vorstehend genannten Verbindungen, die Verwendung der vorstehend genannten Verbindungen als Matrixmaterial, Loch-/Excitonenblockermaterial, Elektronen-/Excitonenblockermaterial, Loch-Injektionsmaterial, Elektronen-Injektionsmaterial, Lochleitermaterial und/oder Elektronenleitermaterial sowie eine Vorrichtung ausgewählt aus der Gruppe bestehend aus stationären Bildschirmen, mobilen Bildschirmen und Beleuchtungseinheiten enthaltend mindestens eine organische Leuchtdiode.

In organischen Leuchtdioden (OLED) wird die Eigenschaft von Materialien ausgenutzt, Licht zu emittieren, wenn sie durch elektrischen Strom angeregt werden. OLEDs sind insbesondere interessant als Alternative zu Kathodenstrahlröhren und zu Flüssigkristalldisplays zur Herstellung von Flachbildschirmen. Aufgrund der sehr kompakten Bauweise und des intrinsisch niedrigen Stromverbrauchs eignen sich Vorrichtungen enthaltend OLEDs insbesondere für mobile Anwendungen, zum Beispiel für Anwendungen in Handys, Laptops, usw. sowie zur Beleuchtung.

Die Grundprinzipien der Funktionsweise von OLEDs sowie geeignete Aufbauten (Schichten) von OLEDs sind dem Fachmann bekannt und zum Beispiel in WO 2005/113704 und der darin zitierten Literatur genannt. Als Licht-emittierende Materialien (Emitter) können neben fluoreszierenden Materialien (Fluoreszenz-Emitter) phosphoreszierende Materialien (Phosphoreszenz-Emitter) eingesetzt werden. Bei den Phosphoreszenz-Emittern handelt es sich üblicherweise um metallorganische Komplexe, die im Gegensatz zu den Fluoreszenz-Emittern, die eine Singulett-Emission zeigen, eine Triplett-Emission zeigen (Triplett-Emitter) (M. A. Baldow et al., Appl. Phys. Lett. 1999, 75, 4 bis 6). Aus quantenmechanischen Gründen ist bei der Verwendung der Triplett-Emitter (Phosphoreszenz-Emitter) eine bis zu vierfache Quanten-, Energie- und Leistungseffizienz möglich. Um die Vorteile des Einsatzes der metallorganischen Triplett-Emitter (Phosphoreszenz-Emitter) in die Praxis umzusetzen, ist es erforderlich, Device-Kompositionen bereitzustellen, die eine hohe operative Lebensdauer, eine gute Effizienz, eine hohe Stabilität gegenüber Temperaturbelastung und eine niedrige Einsatz- und Betriebsspannung aufweisen.

Solche Device-Kompositionen können zum Beispiel spezielle Matrixmaterialien enthalten, in denen der eigentliche Lichtemitter in verteilter Form vorliegt. Des Weiteren können die Kompositionen Blockermaterialien enthalten, wobei Loch-, Excitionen- und/oder Elektronenblocker in den Device-Kompositionen vorliegen können. Daneben oder alternativ können die Device-Kompositionen des Weiteren Loch-Injektionsmaterialien und/oder Elektronen-Injektionsmaterialien und/oder Lochleitermaterialien und/oder Elektronenleitermaterialien aufweisen. Dabei hat die Auswahl der vorstehend genannten Materialien, die in Kombination mit dem eigentlichen Lichtemitter eingesetzt werden, einen wesentlichen Einfluss unter anderem auf die Effizienz sowie die Lebensdauer der OLEDs.

Im Stand der Technik werden zahlreiche verschiedene Materialien für den Einsatz in den verschiedenen Schichten von OLEDs vorgeschlagen.

US 2005/0238919 A1 betrifft eine organische Leuchtdiode, die mindestens eine Arylverbindung, die zwei oder mehr Siliziumatome enthält. Bevorzugt werden die in US 2005/0238919 A1 genannten Materialien als Matrixmaterialien in der Licht-emittierenden Schicht eingesetzt. Organische Leuchtdioden, die Disilyldibenzofurane, Disilyldibenzothiophene, Disilyldibenzophosphole, Disilyldibenzothiophen-S-oxide und Disilyldibenzothiophen-S,S-dioxide enthalten oder Carbazole, die - bei Einsatz ausschließlich in der Licht-emittierenden Schicht oder gleichzeitig in der Licht-emittierenden Schicht und in der Lochleiterschicht - mindestens ein weiteres Heteroatom aufweisen, sind in US 2005/0238919 A1 nicht offenbart. Des Weiteren ist in US 2005/0238919 A1 eine große Anzahl unterschiedlich substituierter Silizium-enthaltender Verbindungen genannt, wobei in den Beispielen der vorliegenden Anmeldung jedoch lediglich Silizium enthaltende Verbindungen der Formeln (1-1) und (1-2), die die folgenden Formeln aufweisen 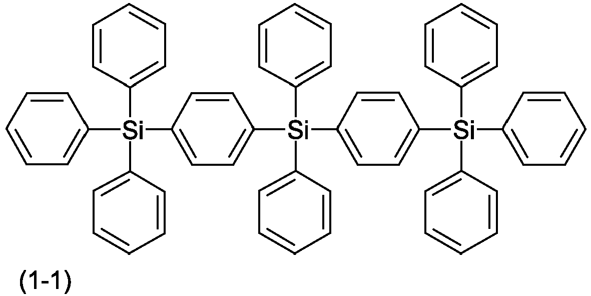 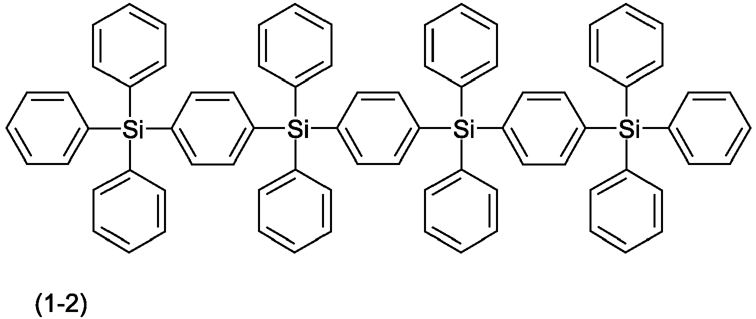 eingesetzt werden.

In Tsai et al., Adv. Mater., 2006, Vol. 18, Nr. 9, Seiten 1216 bis 1220 sind blaues Licht emittierende organische Leuchtdioden offenbart, die 9-(4-Tertbutylphenyl)-3,6-bis(triphenylsilyl)carbazol als Matrixmaterial enthalten.

Yang et al., Angew. Chem. 2007, 119, 2470 bis 2473 betrifft blaues Licht emittierende heteroleptische Iridium(III)komplexe, die für den Einsatz in phosphoreszierenden OLEDs geeignet sind. Als Lochtransportmaterial und Excitonenblockermaterial, das zwischen Lochleiter- und Emitterschicht liegt, wird in den Beispielen gemäß Yang et al. 9-(4-Tertbutylphenyl)-3,6-bis(triphenylsilyl)carbazol neben 9-(4-Tertbutylphenyl)-3,6-bis(triphenylsilyl)carbazol als Matrixmaterial eingesetzt.

JP 2004253298 A betrifft weißes Licht emittierende organische Leuchtdioden, worin substituierte Triphenylsilane als Matrixmaterialien eingesetzt werden.

WO 2007/142083 A1 offenbart eine organische Elektrolumineszenzvorrichtung, die eine Verbindung mit einer spezifischen Struktur mit einem Heteroatom umfasst, und eine organische Elektrolumineszenzvorrichtung, die eine Kathode, eine Anode und eine organische Dünnfilmschicht umfasst.

Mohammed et al, "Reactivity of Silyl Monomers for the Oxidative Polymerization of Phenylene Units", Phosphorus, Sulfur, and Silicon and the Related Elements, 152:1, 265-278, offenbart die oxidative Kupplung von Silylmonomeren, welche Phenyleneinheiten enthalten.

Aufgabe der vorliegenden Anmeldung gegenüber dem Stand der Technik ist es daher, neuartige Device-Kompositionen für OLEDs bereitzustellen, die die neuartigen Materialien zur Verbesserung der Performance der OLED enthalten. Die für die neuartigen Device-Kompositionen geeigneten Materialien sollen leicht zugänglich sein und in Kombination mit dem (den) Emitter(n) gute Effizienzen und gute Lebensdauern in OLEDs bewirken.

Diese Aufgabe wird gelöst durch die Bereitstellung von Verbindungen wie in Anspruch 1 definert.

Offenbart wird weiterhin eine organische Leuchtdiode enthaltend eine Anode An und eine Kathode Ka und eine zwischen der Anode An und der Kathode Ka angeordnete Licht-emittierende Schicht E
sowie gegebenenfalls mindestens eine weitere Schicht ausgewählt aus der Gruppe bestehend aus: mindestens einer Blockschicht für Elektronen/Excitonen, mindestens einer Blockschicht für Löcher/Excitonen, mindestens einer Loch-Injektionsschicht, mindestens einer Lochleiterschicht, mindestens einer Elektroneninjektionsschicht und mindestens einer Elektronenleiterschicht,
dadurch gekennzeichnet, dass die organische Leuchtdiode mindestens eine Verbindung der allgemeinen Formel I enthält, die in der Licht-emittierenden Schicht E und/oder in der mindestens einen weiteren Schicht vorliegt, 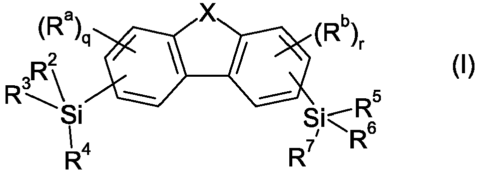 worin bedeuten:
- X: NR¹, S, O, PR¹, SO₂ oder SO;
- R¹: substituiertes oder unsubstituiertes C₁-C₂₀-Alkyl, substituiertes oder unsubstituiertes C₆-C₃₀-Aryl oder substituiertes oder unsubstituiertes Heteroaryl mit 5 bis 30 Ringatomen;
- R², R³, R⁴, R⁵, R⁶, R⁷: unabhängig voneinander substituiertes oder unsubstituiertes C₁-C₂₀-Alkyl oder substituiertes oder unsubstituiertes C₆-C₃₀-Aryl oder eine Struktur der allgemeinen Formel (c) 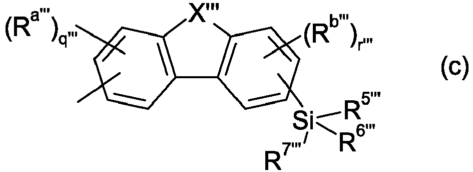
- R^{a}, R^{b}: unabhängig voneinander substituiertes oder unsubstituiertes C₁-C₂₀-Alkyl, substituiertes oder unsubstituiertes C₆-C₃₀-Aryl, substituiertes oder unsubstituiertes Heteroaryl mit 5 bis 30 Ringatomen oder ein Substituent mit Donor- oder Akzeptorwirkung ausgewählt aus der Gruppe bestehend aus: C₁-C₂₀-Alkoxy, C₆-C₃₀-Aryloxy, C₁-C₂₀-Alkylthio, C₆-C₃₀-Arylthio, SiR¹⁴R¹⁵R¹⁶, Halogenresten, halogenierten C₁-C₂₀-Alkylresten, Carbonyl (-CO(R¹⁴)), Carbonylthio (- C = O (SR¹⁴)), Carbonyloxy (- C = O(OR¹⁴)), Oxycarbonyl (- OC = O(R¹⁴)), Thiocarbonyl (- SC = O(R¹⁴)), Amino (-NR¹⁴R¹⁵), OH, Pseudohalogenresten, Amido (- C = O (NR¹⁴)), -NR¹⁴C = O (R¹⁵), Phosphonat (- P(O) (OR¹⁴)₂, Phosphat (-OP(O) (OR¹⁴)₂), Phosphin (-PR¹⁴R¹⁵), Phosphinoxid (-P(O)R¹⁴₂), Sulfat (-OS(O)₂OR¹⁴), Sulfoxid (-S(O)R¹⁴), Sulfonat (-S(O)₂OR¹⁴), Sulfonyl (-S(O)₂R¹⁴, Sulfonamid (-S(O)₂NR¹⁴R¹⁵), NO₂, Boronsäureestern (-OB(OR¹⁴)₂), Imino (-C = NR¹⁴R¹⁵)), Boranresten, Stannanresten, Hydrazinresten, Hydrazonresten, Oximresten, Nitroso-Gruppen, Diazo-Gruppen, Vinylgruppen, Sulfoximinen, Alanen, Germanen, Boroximen und Borazinen;
- R¹⁴, R¹⁵, R¹⁶: unabhängig voneinander substituiertes oder unsubstituiertes C₁-C₂₀-Alkyl oder substituiertes oder unsubstituiertes C₆-C₃₀-Aryl;
- q,r: unabhängig voneinander 0, 1, 2 oder 3; wobei in dem Fall, wenn q bzw. r 0 bedeuten, alle substituierbaren Positionen des Arylrests mit Wasserstoff substituiert sind,
wobei die Reste und Indices in der Gruppe der Formel (c) X"', R⁵''', R⁶''', R⁷''', R^{a}''', R^{b}''', q''' und r''' unabhängig voneinander die für die Reste und Indices der Verbindungen der allgemeinen Formel (I) X, R⁵, R⁶, R⁷, R^{a}, R^{b}, q und r genannten Bedeutungen aufweisen;
wobei

in dem Fall, wenn die Verbindung der allgemeinen Formel (I) ausschließlich in der Licht-emittierenden Schicht oder in der Licht-emittierenden Schicht und in der Lochleiterschicht vorliegt und die Gruppe X NR¹ bedeutet, mindestens einer der Reste R¹ bis R⁷, R^{a} oder R^{b} in den Verbindungen der Formel (I) mindestens ein Heteroatom enthält.

Die Verbindungen der allgemeinen Formel (I) sind leicht zugänglich und weisen, sowohl bei Einsatz als Matrixmaterialien in der Licht-emittierenden Schicht E als auch bei Einsatz in mindestens einer der weiteren Schichten einer OLED gute Effizienzen bei Einsatz in OLEDs auf und sind zur Bereitstellung von OLEDs mit langer Lebensdauer geeignet.

In Abhängigkeit von ihrem Substitutionsmuster können die Verbindungen der Formel (I) entweder als Matrix in der Licht-emittierenden Schicht E, als Loch-/Excitonenblocker, als Elektronen-/Excitonenblocker, als Loch-Injektionsmaterialien, als Elektronen-Injektionsmaterialien, als Lochleiter und/oder als Elektronenleiter eingesetzt werden. Entsprechende Schichten von OLEDs sind dem Fachmann bekannt und zum Beispiel in WO 2005/113704 oder WO 2005/019373 genannt.

### Aufbau der OLED

Die offenbarte organische Leuchtdiode (OLED) weist den folgenden Aufbau auf:
eine Anode (An) und eine Kathode (Ka) und eine zwischen der Anode (An) und der Kathode (Ka) angeordnete Licht-emittierende Schicht E sowie gegebenenfalls mindestens eine weitere Schicht ausgewählt aus der Gruppe bestehend aus: mindestens einer Blockschicht für Elektronen/Excitonen, mindestens einer Blockschicht für Löcher/Excitonen, mindestens einer Loch-Injektionsschicht, mindestens einer Lochleiterschicht, mindestens einer Elektroneninjektionsschicht und mindestens einer Elektronenleiterschicht.

Es ist des Weiteren möglich, dass mehrere der vorstehend genannten Funktionen Elektronen-/Excitonenblocker, Loch-/Excitonenblocker, Loch-Injektion, Lochleitung, Elektroneninjektion, Elektronenleitung) in einer Schicht vereint sind und z. B. von einem einzigen in dieser Schicht vorliegenden Material übernommen werden. Beispielsweise kann ein in der Lochleiterschicht eingesetztes Material in einer Ausführungsform gleichzeitig Excitonen und/oder Elektronen blocken.

Des Weiteren können die einzelnen der vorstehend genannten Schichten der OLED wiederum aus 2 oder mehreren Schichten aufgebaut sein. Beispielsweise kann die Lochleiterschicht aus einer Schicht aufgebaut sein, in die aus der Elektrode Löcher injiziert werden, und einer Schicht, die die Löcher von der Loch injizierenden Schicht weg in die Licht-emittierende Schicht transportiert. Die Elektronenleitungsschicht kann ebenfalls aus mehreren Schichten bestehen, zum Beispiel einer Schicht, worin Elektronen durch die Elektrode injiziert werden, und einer Schicht, die aus der Elektronen-Injektionsschicht Elektronen erhält und in die Licht-emittierende Schicht transportiert. Diese genannten Schichten werden jeweils nach Faktoren wie Energieniveau, Temperaturresistenz und Ladungsträgerbeweglichkeit, sowie Energiedifferenz der genannten Schichten mit den organischen Schichten oder den Metallelektroden ausgewählt. Der Fachmann ist in der Lage, den Aufbau der OLEDs so zu wählen, dass er optimal an die erfindungsgemäß als Emittersubstanzen verwendeten organischen Verbindungen angepasst ist.

Um besonders effiziente OLEDs zu erhalten, sollte z. B. das HOMO (höchstes besetztes Molekülorbital) der Lochleiterschicht mit der Arbeitsfunktion der Anode angeglichen sein, und das LUMO (niedrigstes unbesetztes Molekülorbital) der Elektronenleiterschicht sollte mit der Arbeitsfunktion der Kathode angeglichen sei, soweit die vorstehend genannten Schichten in den OLEDs vorliegen.

Die offenbarte OLED kann z. B. - in einer bevorzugten Ausführungsform - aus den folgenden Schichten aufgebaut sein:
1. Anode
2. Lochleiterschicht
3. Licht-emittierende Schicht
4. Blockschicht für Löcher/Excitonen
5. Elektronenleiterschicht
6. Kathode

Es sind auch von dem vorstehend genannten Aufbau verschiedene Schichtenfolgen möglich, die dem Fachmann bekannt sind. Beispielsweise ist es möglich, dass das OLED nicht alle der genannten Schichten aufweist, zum Beispiel ist eine OLED mit den Schichten (1) (Anode), (3) (Licht-emittierende Schicht) und (6) (Kathode) ebenfalls geeignet, wobei die Funktionen der Schichten (2) (Lochleiterschicht) und (4) (Blockschicht für Löcher/Excitonen) und (5) (Elektronenleiterschicht) durch die angrenzenden Schichten übernommen werden. OLEDs, die die Schichten (1), (2), (3) und (6) bzw. die Schichten (1), (3), (4), (5) und (6) aufweisen, sind ebenfalls geeignet. Des Weiteren können die OLEDs zwischen der Anode (1) und der Lochleiterschicht (2) eine Blockschicht für Elektronen/Excitonen aufweisen.

Die Anode (1) ist eine Elektrode, die positive Ladungsträger bereitstellt. Sie kann zum Beispiel aus Materialien aufgebaut sein, die ein Metall, eine Mischung verschiedener Metalle, eine Metalllegierung, ein Metalloxid oder eine Mischung verschiedener Metalloxide enthält. Alternativ kann die Anode ein leitendes Polymer sein. Geeignete Metalle umfassen die Metalle der Gruppen Ib, IVa, Va und VIa des Periodensystems der Elemente sowie die Übergangsmetalle der Gruppe VIIIa. Wenn die Anode lichtdurchlässig sein soll, werden im Allgemeinen gemischte Metalloxide der Gruppen IIb, IIIb und IVb des Periodensystems der Elemente (alte IUPAC-Version) eingesetzt, zum Beispiel Indium-Zinn-Oxid (ITO). Es ist ebenfalls möglich, dass die Anode (1) ein organisches Material, zum Beispiel Polyanilin enthält, wie beispielsweise in Nature, Vol. 357, Seiten 477 bis 479 (11. Juni 1992) beschrieben ist. Zumindest entweder die Anode oder die Kathode sollten mindestens teilweise transparent sein, um das gebildete Licht auskoppeln zu können. Bevorzugt wird als Material für die Anode (1) ITO eingesetzt. Geeignete Lochleitermaterialien für die Schicht (2) der OLEDs sind zum Beispiel in Kirk-Othmer Encyclopedia of Chemical Technologie, 4. Auflage, Vol. 18, Seiten 837 bis 860, 1996 offenbart. Sowohl Löcher transportierende Moleküle als auch Polymere können als Lochtransportmaterial eingesetzt werden. Üblicherweise eingesetzte Löcher transportierende Moleküle sind ausgewählt aus der Gruppe bestehend aus tris-[N-(1-naphthyl)-N-(phenylamino)]triphenylamin (1-NaphDATA), 4,4'-Bis[N-(1-naphthyl)-N-phenyl-amino]biphenyl (a-NPD), N,N'-Diphenyl-N,N'-bis(3-methylphenyl)-[1,1'-biphenyl]-4,4'-diamin (TPD), 1,1-Bis[(di-4-tolylamino)phenyl]cyclohexan (TAPC), N,N'-Bis(4-methylphenyl)-N,N'-Bis(4-ethylphenyl)-[1,1'-(3,3'-dimethyl)biphenyl]-4,4'-diamin (ETPD), Tetrakis-(3-methylphenyl)-N,N,N',N'-2,5-phenylendiamin (PDA), α-Phenyl-4-N,N-diphenylaminostyrol (TPS), p-(Diethylamino)-benzaldehyddiphenylhydrazon (DEH), Triphenylamin (TPA), Bis[4-(N,N-diethylamino)-2-methylphenyl)(4-methyl-phenyl)methan (MPMP), 1-Phenyl-3-[p-(diethylamino)styryl]-5-[p-(diethylamino)phenyl]pyrazolin (PPR oder DEASP), 1,2-trans-Bis(9H-carbazol-9-yl)cyclobutan (DCZB), N,N,N',N'-Tetrakis(4-methylphenyl)-(1,1'-biphenyl)-4,4'-diamin (TTB), 4,4',4"-tris(N,N-Diphenylamino)triphenylamin (TDTA), Porphyrinverbindungen und Phthalocyaninen wie Kupferphthalocyanine. Üblicherweise eingesetzte Löcher transportierende Polymere sind ausgewählt aus der Gruppe bestehend aus Polyvinylcarbazolen, (Phenylmethyl)polysilanen und Polyanilinen. Es ist ebenfalls möglich, Löcher transportierende Polymere durch Dotieren Löcher transportierender Moleküle in Polymere wie Polystyrol und Polycarbonat zu erhalten. Geeignete Löcher transportierende Moleküle sind die bereits vorstehend genannten Moleküle.

Weiterhin können - in einer Ausführungsform - Carben-Komplexe als Lochleitermaterialien eingesetzt werden, wobei die Bandlücke des mindestens einen Lochleitermaterials im Allgemeinen größer ist als die Bandlücke des eingesetzten Emittermaterials. Dabei ist unter Bandlücke im Sinne der vorliegenden Anmeldung die Triplett-Energie zu verstehen. Geeignete Carben-Komplexe sind z.B. Carben-Komplexe, wie sie in WO 2005/019373 A2, WO 2006/056418 A2 und WO 2005/113704 beschrieben sind.

Die Licht-emittierende Schicht (3) enthält mindestens ein Emittermaterial. Dabei kann es sich grundsätzlich um einen Fluoreszenz oder Phosphoreszenzemitter handeln, wobei geeignete Emittermaterialien dem Fachmann bekannt sind. Bevorzugt handelt es sich bei dem mindestens einen Emittermaterial um einen Phosphoreszenzemitter. Die bevorzugt eingesetzten Phosphoreszenzmitter-Verbindungen basieren auf Metallkomplexen, wobei insbesondere die Komplexe der Metalle Ru, Rh, Ir, Pd und Pt, vor allem die Komplexe des Ir Bedeutung erlangt haben. Die erfindungsgemäß verwendeten Verbindungen der Formel I sind besonders für den Einsatz gemeinsam mit solchen Metallkomplexen geeignet. Dabei werden die Verbindungen der Formel (I) in einer bevorzugten Ausführungsform als Matrixmaterialien und/oder Loch/Excitonen- und/oder Elektronen/Excitonen-blockermaterialien eingesetzt. Insbesondere sind sie für die Verwendung als Matrixmaterialien und/oder Loch/Excitonen- und/oder Elektronen/Excitonen-blockermaterialien zusammen mit Komplexen des Ru, Rh, Ir, Pd und Pt, besonders bevorzugt für die Verwendung zusammen mit Komplexen des Ir geeignet.

Geeignete Metallkomplexe zur Verwendung in den OLEDs sind z.B. in den Schriften WO 02/60910 A1, US 2001/0015432 A1, US 2001/0019782 A1, US 2002/0055014 A1, US 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1 und WO 2006/056418 beschrieben.

Weitere geeignete Metallkomplexe sind die kommerziell erhältlichen Metallkomplexe Tris(2-phenylpyridin)iridium(III), Iridium(III)tris(2-(4-tolyl)pyridinato-N,C^{2'}), Iridium(III)tris(1-phenylisochinolin), Iridium(III)bis(2-2'-benzo-thienyl)pyridinato-N,C^{3'})(acetylacetonat), Iridium(III)bis(2-(4,6-difluorophenyl)pyridinato-N,C²)picolinat, Iridium(III)bis(1-phenylisochinolin)(acetylacetaonat), Iridium(III)bis(di-benzo[f,h]chinoxalin)(acetylacetaonat), Iridium(III)bis(2-methyldi-benzo[f,h]chinoxalin)(acetylacetonat) und Tris(3-methyl-1-phenyl-4-trimethyl-acetyl-5-pyrazolin)terbium(III).

Des Weiteren sind die folgenden kommerziell erhältlichen Materialien geeignet: Tris(dibenzoylacetonato)-mono(phenanthrolin)-europium(III), Tris(dibenzoylmethan)-mono(phenanthrolin)europium(III), Tris(dibenzoylmethan)-mono(5-aminophenanthrolin)-europium(III), Tris(di-2-naphthoylmethan)-mono(phenanthrolin)-europium(III), Tris(4-bromobenzoylmethan)-mono(phenanthrolin)-europium(III), Tris(di-biphenylmethan)-mono(phenanthrolin)-europium(III), Tris(dibenzoylmethan)-mono(4,7-diphenylphenanthrolin)-europium(III), Tris(dibenzoylmethan)-mono(4,7-di-methylphenanthrolin)-europium(III), Tris(dibenzoylmethan)-mono(4,7-dimethyl-phenanthrolin-disulfonsäure)europium(III)-dinatriumsalz, Tris[di(4-(2-(2-ethoxy-ethoxy)ethoxy)benzoylmethan)]mono-(phenanthrolin)-europium(III) und Tris[di[4-(2-(2-ethoxyethoxy)ethoxy)benzoylmethan)]mono-(5-aminophenanthrolin )-europium(III).

Besonders bevorzugte Triplett-Emitter sind Carbenkomplexe. In einer bevorzugten Ausführungsform der vorliegenden Erfindung werden die Verbindungen der Formel (I) in der Licht-emittierenden Schicht als Matrixmaterial gemeinsam mit Carbenkomplexen als Triplett-Emitter eingesetzt. Geeignete Carbenkomplexe sind dem Fachmann bekannt und in einigen der vorstehend genannten Anmeldungen und nachstehend genannt. In einer weiteren bevorzugten Ausführungsform werden die Verbindungen der Formel (I) als Loch/Excitonen-Bockermaterial gemeinsam mit Carbenkomplexen als Triplett-Emitter eingesetzt. Die Verbindungen der Formel (I) können des Weiteren sowohl als Matrixmaterialien als auch als Loch/Excitonenblockermaterialien gemeinsam mit Carbenkomplexen als Triplett-Emitter eingesetzt werden.

Geeignete Metallkomplexe zur Verwendung zusammen mit den Verbindungen der Formel I als Matrixmaterialien und/oder Loch/Excitonen- und/oder Elektronen/ExcitonenBlockermaterialien in OLEDs sind somit z.B. auch Carben-Komplexe, wie sie in WO 2005/019373 A2, WO 2006/056418 A2 und WO 2005/113704 beschrieben sind.

Die Blockschicht für Löcher/Excitonen (4) kann üblicherweise in OLEDs eingesetzte Lochblockermaterialien aufweisen wie 2,9-Dimethyl-4,7-diphenyl-1,10-phenanthrolin (Bathocuproin, (BCP)), Bis-(2-methyl-8-chinolinato)-4-phenyl-phenylato)-aluminium(III) (BAIq), Phenothiazin-S,S-dioxidderivate und 1,3,5-tris(N-Phenyl-2-benzylimidazol)-benzol) (TPBI), wobei TPBI auch als Elektronen-leitendes Material geeignet ist. In einer weiteren Ausführungsform können Verbindungen, die aromatische oder heteroaromatische über Carbonyl-Gruppen enthaltende Gruppen verbundene Ringe enthalten, wie sie in WO2006/100298 offenbart sind, als Blockschicht für Löcher/Excitonen (4) oder als Matrixmaterialien in der Licht-emittierenden Schicht (3) eingesetzt werden.

Des Weiteren kann die Blockschicht für Löcher/Excitonen eine Verbindung der allgemeinen Formel (I) aufweisen, wobei bevorzugt als Lochblocker-/Excitonen-blockermaterialien geeignete Verbindungen der Formel (I) nachstehend genannt sind.

Offenbart wird eine OLED umfassend die Schichten (1) Anode, (2) Lochleiterschicht, (3) Licht-emittierende Schicht, (4) Blockschicht für Löcher/Excitonen, (5) Elektronenleiterschicht und (6) Kathode, sowie gegebenenfalls weitere Schichten, wobei die Blockschicht für Löcher/Excitonen mindestens eine Verbindung der Formel (I) enthält.

Offenbart wird ferner eine OLED umfassend die Schichten (1) Anode, (2) Lochleiterschicht, (3) Licht-emittierende Schicht, (4) Blockschicht für Löcher/Excitonen, (5) Elektronenleiterschicht und (6) Kathode, sowie gegebenenfalls weitere Schichten, wobei die Licht-emittierende Schicht (3) mindestens mindestens eine Verbindung der Formel (I) und die Blockschicht für Löcher/Excitonen mindestens eine Verbindung der Formel (I) enthält.

Offenbart wird ferner eine OLED umfassend die Schichten (1) Anode, (2) Lochleiterschicht und/oder (2') Blockschicht für Elektronen/Excitonen (die OLED kann sowohl die Schichten (2) und (2') als auch entweder die Schicht (2) oder die Schicht (2') enthalten), (3) Licht-emittierende Schicht, (4) Blockschicht für Löcher/Excitonen, (5) Elektronenleiterschicht und (6) Kathode, sowie gegebenenfalls weitere Schichten, wobei die die Blockschicht für Elektronen/Excitonen und/oder die Lochleiterschicht und gegebenenfalls die Licht-emittierende Schicht (3) mindestens eine Verbindung der Formel (I) enthält.

Geeignete Elektronenleitermaterialien für die Schicht (5) der OLEDs umfassen mit oxinoiden Verbindungen chelatisierte Metalle wie 2,2', 2"-(1,3,5-phenylen)tris-[1-phenyl-1H-benzimidazol] (TPBI), Tris(8-chinolinolato)aluminium (Alq₃), Verbindungen auf Phenanthrolinbasis wie 2,9-Dimethyl-4,7-diphenyl-1,10-phenanthrolin (DDPA = BCP) oder 4,7-Diphenyl-1,10-phenanthrolin (DPA) und Azolverbindungen wie 2-(4-Biphenylyl)-5-(4-t-butylphenyl)-1,3,4-oxadiazol (PBD) und 3-(4-Biphenylyl)-4-phenyl-5-(4-t-butylphenyl)-1,2,4-triazol (TAZ). Dabei kann die Schicht (5) sowohl zur Erleichterung des Elektronentransports dienen als auch als Pufferschicht oder als Sperrschicht, um ein Quenchen des Excitons an den Grenzflächen der Schichten der OLED zu vermeiden. Vorzugsweise verbessert die Schicht (5) die Beweglichkeit der Elektronen und reduziert ein Quenchen des Excitons. In einer bevorzugten Ausführungsform wird TPBI als Elektronenleitermaterial eingesetzt.

Von den vorstehend als Lochleitermaterialien und Elektronenleitermaterialien genannten Materialien können einige mehrere Funktionen erfüllen. Zum Beispiel sind einige der Elektronen leitenden Materialien gleichzeitig Löcher blockende Materialien, wenn sie ein tief liegendes HOMO aufweisen. Diese können z. B. in der Blockschicht für Löcher/Excitonen (4) eingesetzt werden. Es ist jedoch ebenfalls möglich, dass die Funktion als Loch/Excitonenblocker von der Schicht (5) mit übernommen wird, so dass die Schicht (4) entfallen kann.

Die Ladungstransportschichten können auch elektronisch dotiert sein, um die Transporteigenschaften der eingesetzten Materialien zu verbessern, um einerseits die Schichtdicken großzügiger zu gestalten (Vermeidung von Pinholes/Kurzschlüssen) und um andererseits die Betriebsspannung des Devices zu minimieren. Beispielsweise können die Lochleitermaterialien mit Elektronenakzeptoren dotiert werden, zum Beispiel können Phthalocyanine bzw. Arylamine wie TPD oder TDTA mit Tetrafluortetracyanchinodimethan (F4-TCNQ) dotiert werden. Die Elektronenleitermaterialien können zum Beispiel mit Alkalimetallen dotiert werden, beispielsweise Alq₃ mit Lithium. Die elektronische Dotierung ist dem Fachmann bekannt und zum Beispiel in W. Gao, A. Kahn, J. Appl. Phys., Vol. 94, No. 1, 1 July 2003 (p-dotierte organische Schichten); A. G. Werner, F. Li, K. Harada, M. Pfeiffer, T. Fritz, K. Leo. Appl. Phys. Lett., Vol. 82, No. 25, 23 June 2003 und Pfeiffer et al., Organic Electronics 2003, 4, 89 - 103 offenbart.

Die Kathode (6) ist eine Elektrode, die zur Einführung von Elektronen oder negativen Ladungsträgern dient. Geeignete Materialien für die Kathode sind ausgewählt aus der Gruppe bestehend aus Alkalimetallen der Gruppe Ia, zum Beispiel Li, Cs, Erdalkalimetallen der Gruppe IIa, zum Beispiel Calcium, Barium oder Magnesium, Metallen der Gruppe IIb des Periodensystems der Elemente (alte IUPAC-Version), umfassend die Lanthaniden und Aktiniden, zum Beispiel Samarium. Des Weiteren können auch Metalle wie Aluminium oder Indium, sowie Kombinationen aller genannten Metalle eingesetzt werden. Weiterhin können Lithium enthaltende organometallische Verbindungen oder LiF zwischen der organischen Schicht und der Kathode aufgebracht werden, um die Betriebsspannung (Operating Voltage) zu vermindern.

Die OLED kann zusätzlich weitere Schichten enthalten, die dem Fachmann bekannt sind. Beispielsweise kann zwischen der Schicht (2) und der Licht emittierenden Schicht (3) eine Schicht aufgebracht sein, die den Transport der positiven Ladung erleichtert und/oder die Bänderlücke der Schichten aneinander anpasst. Alternativ kann diese weitere Schicht als Schutzschicht dienen. In analoger Weise können zusätzliche Schichten zwischen der Licht emittierenden Schicht (3) und der Schicht (4) vorhanden sein, um den Transport der negativen Ladung zu erleichtern und/oder die Bänderlücke zwischen den Schichten aneinander anzupassen. Alternativ kann diese Schicht als Schutzschicht dienen.

In einem Aspekt enthält die OLED zusätzlich zu den Schichten (1) bis (6) mindestens eine der im Folgenden genannten weiteren Schichten:
- eine Loch-Injektionsschicht zwischen der Anode (1) und der Löcher-transportierenden Schicht (2);
- eine Blockschicht für Elektronen zwischen der Löcher-transportierenden Schicht (2) und der Licht-emittierenden Schicht (3);
- eine Elektronen-Injektionsschicht zwischen der Elektronen-transportierenden Schicht (5) und der Kathode (6).

Dem Fachmann ist bekannt, wie er (zum Beispiel auf Basis von elektrochemischen Untersuchungen) geeignete Materialien auswählen muss. Geeignete Materialien für die einzelnen Schichten sind dem Fachmann bekannt und z.B. in WO 00/70655 offenbart.

Des Weiteren ist es möglich, dass einige der in der OLED eingesetzten Schichten oberflächenbehandelt sind, um die Effizienz des Ladungsträgertransports zu erhöhen. Die Auswahl der Materialien für jede der genannten Schichten ist bevorzugt dadurch bestimmt, eine OLED mit einer hohen Effizienz und Lebensdauer zu erhalten.

Die Herstellung der OLED kann nach dem Fachmann bekannten Methoden erfolgen. Im Allgemeinen wird die OLED durch aufeinander folgende Dampfabscheidung (Vapor deposition) der einzelnen Schichten auf ein geeignetes Substrat hergestellt. Geeignete Substrate sind zum Beispiel Glas, anorganische Halbleiter oder Polymerfilme. Zur Dampfabscheidung können übliche Techniken eingesetzt werden wie thermische Verdampfung, Chemical Vapor Deposition (CVD), Physical Vapor Deposition (PVD) und andere. In einem alternativen Verfahren können die organischen Schichten der OLED aus Lösungen oder Dispersionen in geeigneten Lösungsmitteln aufgetragen werden, wobei dem Fachmann bekannte Beschichtungstechniken angewendet werden.

Im Allgemeinen haben die verschiedenen Schichten folgende Dicken: Anode (1) 50 bis 500 nm, bevorzugt 100 bis 200 nm; Löcher-leitende Schicht (2) 5 bis 100 nm, bevorzugt 20 bis 80 nm, Licht-emittierende Schicht (3) 1 bis 100 nm, bevorzugt 10 bis 80 nm, Blockschicht für Löcher/Excitonen (4) 2 bis 100 nm, bevorzugt 5 bis 50 nm, Elektronen-leitende Schicht (5) 5 bis 100 nm, bevorzugt 20 bis 80 nm, Kathode (6) 20 bis 1000 nm, bevorzugt 30 bis 500 nm. Die relative Lage der Rekombinationszone von Löchern und Elektronen in den OLEDs in Bezug zur Kathode und somit das Emissionsspektrum der OLED können u. a. durch die relative Dicke jeder Schicht beeinflusst werden. Das bedeutet, die Dicke der Elektronentransportschicht sollte bevorzugt so gewählt werden, dass die Lage der Rekombinationszone auf die optische Resonatoreigenschaft der Diode und damit auf die Emissionswellenlänge des Emitters abgestimmt ist. Das Verhältnis der Schichtdicken der einzelnen Schichten in der OLED ist von den eingesetzten Materialien abhängig. Die Schichtdicken von gegebenenfalls eingesetzten zusätzlichen Schichten sind dem Fachmann bekannt. Es ist möglich, dass die Elektronen-leitende Schicht und/oder die Löcher leitende Schicht größere Dicken als die angegebenen Schichtdicken aufweisen, wenn sie elektrisch dotiert sind.

Die Licht-emittierende Schicht E und/oder enthält mindestens eine der weiteren in der OLED gegebenenfalls vorliegenden Schichten mindestens eine Verbindung der allgemeinen Formel (I). Während die mindestens eine Verbindung der allgemeinen Formel (I) in der Licht-emittierenden Schicht E als Matrixmaterial vorliegt, kann die mindestens eine Verbindung der allgemeinen Formel (I) in der mindestens einen weiteren Schicht der OLED jeweils allein oder gemeinsam mit mindestens einem der weiteren für die entsprechenden Schichten geeigneten vorstehend genannten Materialien eingesetzt werden.

Unter substituiertem oder unsubstituiertem C₁-C₂₀-Alkyl sind Alkylreste mit 1 bis 20 Kohlenstoffatomen zu verstehen. Bevorzugt sind C₁- bis C₁₀-Alkylreste, besonders bevorzugt C₁- bis C₆-Alkylreste. Die Alkylreste können sowohl geradkettig als auch verzweigt oder cyclisch sein, wobei die Alkylreste im Falle von cyclischen Alkylresten mindestens 3 Kohlenstoffatome aufweisen. Des Weiteren können die Alkylreste mit einem oder mehreren Substituenten ausgewählt aus der Gruppe bestehend aus C₁-C₂₀-Alkoxy, Halogen, bevorzugt F, und C₆-C₃₀-Aryl, das wiederum substituiert oder unsubstituiert sein kann, substituiert sein. Geeignete Arylsubstituenten sowie geeignete Alkoxy- und Halogensubstituenten sind nachstehend genannt. Beispiele für geeignete Alkylgruppen sind Methyl, Ethyl, Propyl, Butyl, Pentyl, Hexyl, Heptyl und Octyl sowie mit C₆-C₃₀-Aryl-, C₁-C₂₀-Alkoxy- und/oder Halogen, insbesondere F, substituierte Derivate der genannten Alkylgruppen, zum Beispiel CF₃. Dabei sind sowohl die n-Isomere der genannten Reste als auch verzweigte Isomere wie Isopropyl, Isobutyl, Isopentyl, sec-Butyl, tert-Butyl, Neopentyl, 3,3-Dimethylbutyl, 3-Ethylhexyl usw. mit umfasst. Bevorzugte Alkylgruppen sind Methyl, Ethyl, tert-Butyl und CF₃.

Beispiele für geeignete cyclische Alkylgruppen, die ebenfalls unsubstituiert oder mit den vorstehend bezüglich der Alkylgruppen genannten Resten substituiert sein können, sind Cyclopropyl, Cyclobutyl, Cyclopentyl, Cyclohexyl, Cycloheptyl, Cyclooctyl, Cyclononyl und Cyclodecyl. Gegebenenfalls kann es sich auch um polycyclische Ringsysteme handeln, wie Decalinyl, Norbornyl, Bornanyl oder Adamantyl.

Geeignete C₁-C₂₀-Alkoxy- und C₁-C₂₀-Alkylthiogruppen leiten sich entsprechend von den vorstehend genannten C₁-C₂₀-Alkylresten ab. Beispielsweise sind hier zu nennen OCH₃, OC₂H₅, OC₃H₇, OC₄H₉ und OC₈H₁₇ sowie SCH₃, SC₂H₅, SC₃H₇, SC₄H₉ und SC₈H₁₇. Dabei sind unter C₃H₇, C₄H₉ und C₈H₁₇ sowohl die n-Isomere als auch verzweigte Isomere wie iso-Propyl, iso-Butyl, sec-Butyl, tert-Butyl und 2-Ethylhexyl umfasst. Besonders bevorzugte Alkoxy- oder Alkylthio-Gruppen sind Methoxy, Ethoxy, n-Octyloxy, 2-Ethylhexyloxy und SCH₃.

Geeignete Halogenreste oder Halogensubstituenten im Sinne der vorliegenden Anmeldung sind Fluor, Chlor, Brom und lod, bevorzugt Fluor, Chlor und Brom, besonders bevorzugt Fluor und Chlor, ganz besonders bevorzugt Fluor.

Geeignete Pseudohalogenreste im Sinne der vorliegenden Anmeldung sind CN, SCN, OCN, N₃ und SeCN zu verstehen, wobei CN und SCN bevorzugt sind. Ganz besonders bevorzugt ist CN.

Als C₆-C₃₀-Aryl werden in der vorliegenden Erfindung Reste bezeichnet, die von monocyclischen, bicyclischen oder tricyclischen Aromaten abgeleitet sind, die keine Ringheteroatome enthalten. Sofern es sich nicht um monocyclische Systeme handelt, ist bei der Bezeichnung Aryl für den zweiten Ring auch die gesättigte Form (Perhydroform) oder die teilweise ungesättigte Form (beispielsweise die Dihydroform oder Tetrahyroform), sofern die jeweiligen Formen bekannt und stabil sind, möglich. Das heißt, die Bezeichnung Aryl umfasst in der vorliegenden Erfindung beispielsweise auch bicyclische oder tricyclische Reste, in denen sowohl beide als auch alle drei Reste aromatisch sind, als auch bicyclische oder tricyclische Reste, in denen nur ein Ring aromatisch ist, sowie tricyclische Reste, worin zwei Ringe aromatisch sind. Beispiele für Aryl sind: Phenyl, Naphthyl, Indanyl, 1,2-Dihydronaphthenyl, 1,4-Dihydronaphthenyl, Indenyl, Anthracenyl, Phenanthrenyl oder 1,2,3,4-Tetrahydronaphthyl. Besonders bevorzugt sind C₆-C₁₀-Arylreste, zum Beispiel Phenyl oder Naphthyl, ganz besonders bevorzugt C₆-Arylreste, zum Beispiel Phenyl.

Die C₆-C₃₀-Arylreste können unsubstituiert sein oder mit einem oder mehreren weiteren Resten substituiert sein. Geeignete weitere Reste sind ausgewählt aus der Gruppe bestehend aus C₁-C₂₀-Alkyl, C₆-C₃₀-Aryl oder Substituenten mit Donor- oder Akzeptorwirkung, wobei geeignete Substituenten mit Donor- oder Akzeptorwirkung nachstehend genannt sind. Bevorzugt sind die C₆-C₃₀-Arylreste unsubstituiert oder mit einer oder mehreren C₁-C₂₀-Alkoxygruppen, CN, CF₃, F oder Aminogruppen (NR¹⁴R¹⁵, wobei geeignete Reste R¹⁴ und R¹⁵ vorstehend genannt sind), substituiert. Weitere bevorzugte Substitutionen der C₆-C₃₀-Arylreste sind abhängig von dem Einsatzzweck der Verbindungen der allgemeinen Formel (I) und sind nachstehend genannt.
Geeignete C₆-C₃₀-Aryloxy-, C₆-C₃₀-Alkylthioreste leiten sich entsprechend von den vorstehend genannten C₆-C₃₀-Arylresten ab. Besonders bevorzugt sind Phenoxy und Phenylthio.

Unter unsubstituiertem oder substituiertem Heteroaryl mit 5 bis 30 Ringatomen sind monocyclische, bicyclische oder tricyclische Heteroaromaten zu verstehen, die sich zum Teil vom vorstehend genannten Aryl ableiten lassen, in dem im Aryl-Grundgerüst mindestens ein Kohlenstoffatom durch ein Heteroatom ersetzt ist. Bevorzugte Heteroatome sind N, O und S. Besonders bevorzugt weisen die Heteroarylreste 5 bis 13 Ringatome auf. Insbesondere bevorzugt ist das Grundgerüst der Heteroarylreste ausgewählt aus Systemen wie Pyridin und fünfgliedrigen Heteroaromaten wie Thiophen, Pyrrol, Imidazol oder Furan. Diese Grundgerüste können gegebenenfalls mit einem oder zwei sechsgliedrigen aromatischen Resten anelliert sein. Geeignete anellierte Heteroaromaten sind Carbazolyl, Benzimidazolyl, Benzofuryl, Dibenzofuryl oder Dibenzothiophenyl. Das Grundgerüst kann an einer, mehreren oder allen substituierbaren Positionen substituiert sein, wobei geeignete Substituenten dieselben sind, die bereits unter der Definition von C₆-C₃₀-Aryl genannt wurden. Bevorzugt sind die Heteroarylreste jedoch unsubstituiert. Geeignete Heteroarylreste sind zum Beispiel Pyridin-2-yl, Pyridin-3-yl, Pyridin-4-yl, Thiophen-2-yl, Thiophen-3-yl, Pyrrol-2-yl, Pyrrol-3-yl, Furan-2-yl, Furan-3-yl und Imidazol-2-yl sowie die entsprechenden benzanellierten Reste, insbesondere Carbazolyl, Benzimidazolyl, Benzofuryl, Dibenzofuryl oder Dibenzothiophenyl.

Unter Gruppen mit Donor- oder Akzeptorwirkung sind im Sinne der vorliegenden Anmeldung die folgenden Gruppen zu verstehen:
C₁-C₂₀-Alkoxy, C₆-C₃₀-Aryloxy, C₁-C₂₀-Alkylthio, C₆-C₃₀-Arylthio, SiR¹⁴R¹⁵R¹⁶, Halogenresten, halogenierten C₁-C₂₀-Alkylresten, Carbonyl (-CO(R¹⁴)), Carbonylthio (- C = O (SR¹⁴)), Carbonyloxy (- C = O(OR¹⁴)), Oxycarbonyl (- OC = O(R¹⁴)), Thiocarbonyl (- SC = O(R¹⁴)), Amino (-NR¹⁴R¹⁵), OH, Pseudohalogenresten, Amido (- C = O (NR¹⁴)), - NR¹⁴C = O (R¹⁵), Phosphonat (- P(O) (OR¹⁴)₂, Phosphat (-OP(O) (OR¹⁴)₂), Phosphin (-PR¹⁴R¹⁵), Phosphinoxid (-P(O)R¹⁴₂), Sulfat (-OS(O)₂OR¹⁴), Sulfoxid (-S(O)R¹⁴), Sulfonat (-S(O)₂OR¹⁴), Sulfonyl (-S(O)₂R¹⁴), Sulfonamid (-S(O)₂NR¹⁴R¹⁵), NO₂, Boronsäureestern (-OB(OR¹⁴)₂), Imino (-C = NR¹⁴R¹⁵)), Boranresten, Stannanresten, Hydrazinresten, Hydrazonresten, Oximresten, Nitroso-Gruppen, Diazo-Gruppen, Vinylgruppen, (=Sulfonat) und Boronsäuregruppen, Sulfoximine, Alane, Germane, Boroxime und Borazine.

Bevorzugte Substituenten mit Donor- oder Akzeptorwirkung sind ausgewählt aus der Gruppe bestehend aus:
C₁- bis C₂₀-Alkoxy, bevorzugt C₁-C₆-Alkoxy, besonders bevorzugt Ethoxy oder Methoxy; C₆-C₃₀-Aryloxy, bevorzugt C₆-C₁₀-Aryloxy, besonders bevorzugt Phenyloxy; SiR¹⁴R¹⁵R¹⁶,wobei R¹⁴,R¹⁵ und R¹⁶ bevorzugt unabhängig voneinander substituiertes oder unsubstituiertes Alkyl oder substituiertes oder unsubstituiertes Phenyl bedeuten; besonders bevorzugt ist mindenstens einer der Reste R¹⁴,R¹⁵ oder R¹⁶ substituiertes oder unsubstituiertes Phenyl, ganz besonders bevorzugt ist mindestens einer der Reste R¹⁴,R¹⁵ und R¹⁶ substituiertes Phenyl, wobei geeignete Substituenten vorstehend genannt sind; Halogenresten, bevorzugt F, Cl, Br, besonders bevorzugt F oder Cl, ganz besonders bevorzugt F, halogenierten C₁-C₂₀-Alkylresten, bevorzugt halogenierten C₁-C₆-Alkylresten, ganz besonders bevorzugt fluorierten C₁-C₆-Alkylresten, z. B. CF₃, CH₂F, CHF₂ oder C₂F₅; Amino, bevorzugt Dimethylamino, Diethylamino oder Diphenylamino; OH, Pseudohalogenresten, bevorzugt CN, SCN oder OCN, besonders bevorzugt CN, -C(O)OC₁-C₄-Alkyl, bevorzugt -C(O)OMe, P(O)R₂, bevorzugt P(O)Ph₂ oder SO₂R₂, bevorzugt SO₂Ph.

Ganz besonders bevorzugte Substituenten mit Donor- oder Akzeptorwirkung sind ausgewählt aus der Gruppe bestehend aus Methoxy, Phenyloxy, halogeniertem C₁-C₄-Alkyl, bevorzugt CF₃, CH₂F, CHF₂, C₂F₅, Halogen, bevorzugt F, CN, SiR¹⁴R¹⁵R¹⁶, wobei geeignete Reste R¹⁴, R¹⁵ und R¹⁶ bereits genannt sind, Diphenylamino, -C(O)OC₁-C₄-Alkyl, bevorzugt -C(O)OMe, P(O)Ph₂, SO₂Ph.

Durch die vorstehend genannten Gruppen mit Donor- oder Akzeptorwirkung soll nicht ausgeschlossen werden, dass auch weitere der vorstehend genannten Reste und Gruppen eine Donor- oder Akzeptorwirkung aufweisen können. Beispielsweise handelt es sich bei den vorstehend genannten Heteroarylresten ebenfalls um Gruppen mit Donor- oder Akzeptorwirkung und bei den C₁-C₂₀-Alkylresten handelt es sich um Gruppen mit Donorwirkung.

Die in den vorstehend genannten Gruppen mit Donor- oder Akzeptorwirkung erwähnten Reste R¹⁴, R¹⁵ und R¹⁶ haben die bereits vorstehend erwähnten Bedeutungen, d. h. R¹⁴, R¹⁵, R¹⁶ bedeuten unabhängig voneinander:
Substituiertes oder unsubstituiertes C₁-C₂₀-Alkyl oder substituiertes oder unsubstituiertes C₆-C₃₀-Aryl, wobei geeignete und bevorzugte Alkyl- und Arylreste vorstehend genannt sind. Besonders bevorzugt bedeuten die Reste R¹⁴, R¹⁵ und R¹⁶ C₁-C₆-Alkyl, z. B. Methyl, Ethyl oder i-Propyl, Phenyl. In einer bevorzugten Ausführungsform - im Falle von SiR¹⁴R¹⁵R¹⁶ - bedeuten R¹⁴,R¹⁵ und R¹⁶ bevorzugt unabhängig voneinander substituiertes oder unsubstituiertes C₁-C₂₀-Alkyl oder substituiertes oder unsubstituiertes Phenyl; besonders bevorzugt ist mindestens einer der Reste R¹⁴,R¹⁵ oder R¹⁶ substituiertes oder unsubstituiertes Phenyl, ganz besonders bevorzugt ist mindestens einer der Reste R¹⁴,R¹⁵ und R¹⁶ substituiertes Phenyl, wobei geeignete Substituenten vorstehend genannt sind.

Die in der vorliegenden Anmeldung des Weiteren verwendeten Ausdrücke elektronenschiebende Substituenten und elektronenziehende Substituenten sind Substituenten mit Donorwirkung (elektronenschiebende Substituenten) bzw. Substituenten mit Akzeptorwirkung (elektronenziehende Substituenten). Geeignete elektronenschiebende und elektronenziehende Substituenten sind somit die vorstehend bereits bezüglich der Substituenten mit Donor- oder Akzeptorwirkung genannten Substituenten.

### Verbindungen der Formel (I)

Bei den Verbindungen der Formel (I) handelt es sich um Disilylverbindungen, worin die Reste und Indices die folgenden Bedeutungen aufweisen:
- X: NR¹, S, O, PR¹, SO₂ oder SO, bevorzugt NR¹, S, O;
- R¹: substituiertes oder unsubstituiertes C₁-C₂₀-Alkyl, substituiertes oder unsubstituiertes C₆-C₃₀-Aryl oder substituiertes oder unsubstituiertes Heteroaryl mit 5 bis 30 Ringatomen; bevorzugt substituiertes oder unsubstituiertes C₁-C₂₀-Alkyl oder substituiertes oder unsubstituiertes C₆-C₃₀-Aryl, besonders bevorzugt substituiertes oder unsubstituiertes C₆-C₁₀-Aryl oder unsubstituiertes C₁-C₂₀-Alkyl, ganz besonders bevorzugt substituiertes oder unsubstituiertes Phenyl, wobei geeignete Substituenten vorstehend genannt sind;
- R², R³, R⁴, R⁵, R⁶, R⁷: unabhängig voneinander substituiertes oder unsubstituiertes C₁-C₂₀-Alkyl oder substituiertes oder unsubstituiertes C₆-C₃₀-Aryl oder eine Struktur der allgemeinen Formel (c); bevorzugt bedeutet mindestens einer der Reste R², R³ oder R⁴ und/oder mindestens einer der Reste R⁵, R⁶ oder R⁷ substituiertes oder unsubstituiertes C₆-C₃₀-Aryl, besonders bevorzugt substituiertes oder unsubstituiertes C₆-C₁₀-Aryl, ganz besonders bevorzugt substituiertes oder unsubstituiertes Phenyl, wobei geeignete Substituenten vorstehend genannt sind, und/oder einer der Reste R², R³ oder R⁴ und/oder einer der Reste R⁵, R⁶ oder R⁷ ist ein Rest der Struktur (c);
- R^{a}, R^{b}: unabhängig voneinander substituiertes oder unsubstituiertes C₁-C₂₀-Alkyl, substituiertes oder unsubstituiertes C₆-C₃₀-Aryl, subsituiertes oder unsubstituiertes Heteroaryl mit 5 bis 30 Ringatomen, oder ein Substituent mit Donor- oder Akzeptorwirkung, wobei geeignete und bevorzugte Substituenten mit Donor- oder Akzeptorwirkung vorstehend genannt sind;
- R¹⁴, R¹⁵, R¹⁶: unabhängig voneinander substituiertes oder unsubstituiertes C₁-C₂₀-Alkyl oder substituiertes oder unsubstituiertes C₆-C₃₀-Aryl, bevorzugt substituiertes oder unsubstituiertes C₁-C₆-Alkyl oder substituiertes oder unsubstituiertes C₆-C₁₀-Aryl, wobei R¹⁴,R¹⁵ und R¹⁶ besonders bevorzugt unabhängig voneinander substituiertes oder unsubstituiertes C₁-C₂₀-Alkyl oder substituiertes oder unsubstituiertes Phenyl bedeuten; besonders bevorzugt ist mindestens einer der Reste R¹⁴,R¹⁵ oder R¹⁶ substituiertes oder unsubstituiertes Phenyl, ganz besonders bevorzugt ist mindestens einer der Reste R¹⁴,R¹⁵ und R¹⁶ substituiertes Phenyl, wobei geeignete Substituenten vorstehend genannt sind;
- q,r: unabhängig voneinander 0, 1, 2 oder 3, wobei, wenn q bzw. r 0 sind, alle substituierbaren Positionen des Arylrests Wasserstoffatome tragen, bevorzugt 0.

Offenbart wird ferner eine organische Leuchtdiode, worin in dem Fall, wenn die Verbindung der allgemeinen Formel (I) ausschließlich in der Licht-emittierenden Schicht oder in der Licht-emittierenden Schicht und in der Lochleiterschicht vorliegt und die Gruppe X NR¹ bedeutet, mindestens einer der Reste R¹ bis R⁷, R^{a} oder R^{b} in den Verbindungen der Formel (I) mindestens ein Heteroatom enthält. Bevorzugte Heteroatome sind N, Si, Halogen, insbesondere F oder Cl, O, S oder P. Das Heteroatom kann in Form eines Substituenten an mindestens einem der Reste R¹ bis R⁷, R^{a} oder R^{b} bzw. in Form eines Teils eines Substituenten vorliegen oder in dem Grundgerüst mindestens eines der Reste R¹ bis R⁷, R^{a} oder R^{b} vorliegen. Geeignete Substituenten oder Grundgerüste sind dem Fachmann bekannt und unter den Definitionen der Reste R¹ bis R⁷, R^{a} oder R^{b} genannt.

Eine Aspekt betrifft eine organische Leuchtdiode, worin mindestens einer der Reste R², R³ oder R⁴ und/oder mindestens einer der Reste R⁵, R⁶ oder R⁷ in den Verbindungen der Formel (I) substituiertes oder unsubstituiertes C₆-C₃₀-Aryl bedeuten. Bevorzugte Arylreste und deren Substituenten wurden bereits vorstehend genannt.

Offenbart wird ferner eine organische Leuchtdiode, worin die Verbindung der allgemeinen Formel (I) eine 3,6-Disilyl-substituierte Verbindung der allgemeinen Formel (Ia) ist: 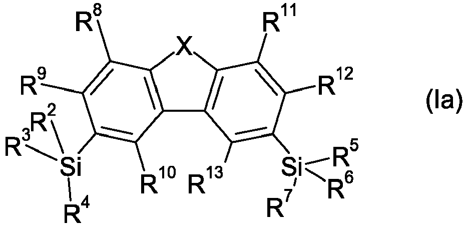 worin bedeuten:
- X: NR¹, S, O, PR¹, SO₂ oder SO; bevorzugt NR¹, S oder O; besonders bevorzugt NR¹;
- R¹: substituiertes oder unsubstituiertes C₁-C₂₀-Alkyl, substituiertes oder unsubstituiertes C₆-C₃₀-Aryl oder substituiertes oder unsubstituiertes Heteroaryl mit 5 bis 30 Ringatomen; bevorzugt substituiertes oder unsubstituiertes C₆-C₃₀-Aryl oder substituiertes oder unsubstituiertes C₁-C₂₀-Alkyl, besonders bevorzugt substituiertes oder unsubstituiertes C₆-C₁₀-Aryl oder unsubstituiertes C₁-C₂₀-Alkyl, ganz besonders bevorzugt substituiertes oder unsubstituiertes Phenyl, wobei geeignete Substituenten vorstehend genannt sind;
- R², R³, R⁴, R⁵, R⁶, R⁷: unabhängig voneinander substituiertes oder unsubstituiertes C₁-C₂₀-Alkyl oder substituiertes oder unsubstituiertes C₆-C₃₀-Aryl oder eine Struktur der allgemeinen Formel (c); bevorzugt bedeutet mindestens einer der Reste R², R³ oder R⁴ und/oder mindestens einer der Reste R⁵, R⁶ oder R⁷ substituiertes oder unsubstituiertes C₆-C₃₀-Aryl, besonders bevorzugt substituiertes oder unsubstituiertes C₆-C₁₀-Aryl, ganz besonders bevorzugt substituiertes oder unsubstituiertes Phenyl, wobei geeignete Substituenten vorstehend genannt sind, und/oder einer der Reste R², R³ oder R⁴ und/oder einer der Reste R⁵, R⁶ oder R⁷ ist ein Rest der Struktur (c);
- R⁸, R⁹, R¹⁰, R¹¹, R¹², R¹³: unabhängig voneinander Wasserstoff oder die für R^{a} und R^{b} genannten Bedeutungen, das heißt unabhängig voneinander substituiertes oder unsubstituiertes C₁-C₂₀-Alkyl, substituiertes oder unsubstituiertes C₆-C₃₀-Aryl, substituiertes oder unsubstituiertes Heteroaryl mit 5 bis 30 Ringatomen oder ein Substituent mit Donor- oder Akzeptorwirkung, wobei geeignete Substituenten mit Donor- oder Akzeptorwirkung vorstehend genannt sind; bevorzugt Wasserstoff, substituiertes oder unsubstituiertes C₁-C₆-Alkyl, substituiertes oder unsubstituiertes C₆-C₁₀-Aryl oder SiR¹⁴R¹⁵R¹⁶; besonders bevorzugt Wasserstoff, Methyl, Ethyl, Phenyl, CF₃ oder SiR¹⁴R¹⁵R¹⁶ wobei R¹⁴,R¹⁵ und R¹⁶ bevorzugt unabhängig voneinander substituiertes oder unsubstituiertes C₁-C₂₀-Alkyl oder substituiertes oder unsubstituiertes Phenyl bedeuten; besonders bevorzugt ist mindestens einer der Reste R¹⁴,R¹⁵ oder R¹⁶ substituiertes oder unsubstituiertes Phenyl, ganz besonders bevorzugt ist mindestens einer der Reste R¹⁴,R¹⁵ und R¹⁶ substituiertes Phenyl, wobei geeignete Substituenten vorstehend genannt sind;
und die weiteren Reste und Indices R¹⁴, R¹⁵, R¹⁶ die vorstehend genannten Bedeutungen aufweisen;
wobei
in dem Fall, wenn die Verbindung der allgemeinen Formel (I) ausschließlich in der Licht-emittierenden Schicht oder in der Licht-emittierenden Schicht und in der Lochleiterschicht vorliegt und die Gruppe X NR¹ bedeutet, mindestens einer der Reste R¹ bis R⁷, R^{a} oder R^{b} in den Verbindungen der Formel (I) mindestens ein Heteroatom enthält.

In einem Aspekt weisen die in den organischen Leuchtdioden eingesetzten Verbindungen der Formel (I) für die Reste R¹ bis R⁷, R^{a} und R^{b} sowie die Gruppe X die folgenden Bedeutungen auf:
- X: NR¹;
- R¹: substituiertes oder unsubstituiertes C₆-C₃₀-Aryl oder substituiertes oder unsubstituiertes Heteroaryl mit 5 bis 30 Ringatomen, bevorzugt substituiertes oder unsubstituiertes C₆-C₁₀-Aryl, besonders bevorzugt substituiertes oder unsubstituiertes Phenyl, wobei geeignete Substituenten vorstehend genannt sind;
- R², R³, R⁴, R⁵, R⁶, R⁷: unabhängig voneinander substiuiertes oder unsubstituiertes C₁-C₂₀-Alkyl oder substituiertes oder unsubstituiertes C₆-C₃₀-Aryl, oder eine Struktur der allgemeinen Formel (c), bevorzugt unabhängig voneinander substituiertes oder unsubstituiertes C₁-C₆-Alkyl oder substituiertes oder unsubstituiertes C₆-C₁₀-Aryl, besonders bevorzugt substituiertes oder unsubstituiertes C₁-C₆-Alkyl oder substituiertes oder unsubstituiertes Phenyl; wobei in einer Ausführungsform mindestens einer der Reste R², R³ oder R⁴ und/oder mindestens einer der Reste R⁵, R⁶ oder R⁷ substituiertes oder unsubstituiertes C₆-C₃₀-Aryl, bevorzugt substituiertes oder unsubstituiertes C₆-C₁₀-Aryl, besonders bevorzugt substituiertes oder unsubstituiertes Phenyl, bedeutet; wobei bevorzugte Substituenten vorstehend genannt sind;
- R⁸, R⁹, R¹⁰, R¹¹, R¹², R¹³: unabhängig voneinander Wasserstoff oder die für R^{a} und R^{b} genannten Bedeutungen, das heißt unabhängig voneinander substituiertes oder unsubstituiertes C₁-C₂₀-Alkyl, substituiertes oder unsubstituiertes C₆-C₃₀-Aryl, substituiertes oder unsubstituiertes Heteroaryl mit 5 bis 30 Ringatomen oder ein Substituent mit Donor- oder Akzeptorwirkung, wobei geeignete Substituenten mit Donor- oder Akzeptorwirkung bereits vorstehend genannt sind; bevorzugt Wasserstoff, substituiertes oder unsubstituiertes C₁-C₆-Alkyl, substituiertes oder unsubstituiertes C₆-C₁₀-Aryl oder SiR¹⁴R¹⁵R¹⁶; besonders bevorzugt Wasserstoff, Methyl, Ethyl, Phenyl, CF₃ oder SiR¹⁴R¹⁵R¹⁶;
- R¹⁴, R¹⁵, R¹⁶: unabhängig voneinander substituiertes oder unsubstituiertes C₁-C₂₀-Alkyl oder substituiertes oder unsubstituiertes C₆-C₃₀-Aryl, bevorzugt substituiertes oder unsubstituiertes C₁-C₆-Alkyl oder substituiertes oder unsubstituiertes C₆-C₁₀-Aryl, wobei R¹⁴, R¹⁵ und R¹⁶ besonders bevorzugt unabhängig voneinander substituiertes oder unsubstituiertes C₁-C₂₀-Alkyl,oder substituiertes oder unsubstituiertes Phenyl bedeuten; besonders bevorzugt ist mindenstens einer der Reste R¹⁴,R¹⁵ oder R¹⁶ substituiertes oder unsubstituiertes Phenyl, ganz besonders bevorzugt ist mindenstens einer der Reste R¹⁴,R¹⁵ und R¹⁶ substituiertes Phenyl, wobei geeignete Substituenten vorstehend genannt sind;
wobei
in dem Fall, wenn die Verbindung der allgemeinen Formel (I) ausschließlich in der Licht-emittierenden Schicht oder in der Licht-emittierenden Schicht und in der Lochleiterschicht vorliegt, mindestens einer der Reste R¹ bis R⁷, R^{a} oder R^{b} in den Verbindungen der Formel (I) mindestens ein Heteroatom enthält.

Bevorzugte Verbindungen der Formel (I) für den Einsatz in den OLEDs sind nachstehend aufgeführt:
i) Bevorzugte Verbindungen der Formel (I), worin X NR¹ oder PR¹ bedeutet (im Folgenden sind die bevorzugten Carbazolderivate (X = NR¹) dargestellt). Von der vorliegenden Erfindung sind ebenfalls solche Verbindungen umfasst, worin das N in den nachfolgenden Formeln durch P ersetzt ist (X = PR¹):
   ia) Verbindungen der Formel (I), die besonders bevorzugt als Matrix und/oder Elektronen/Excitonenblocker geeignet sind: 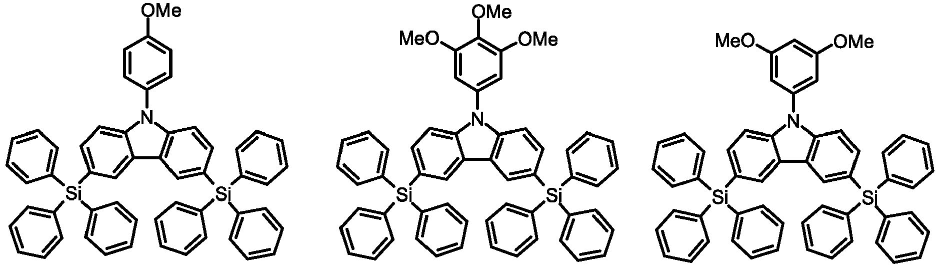 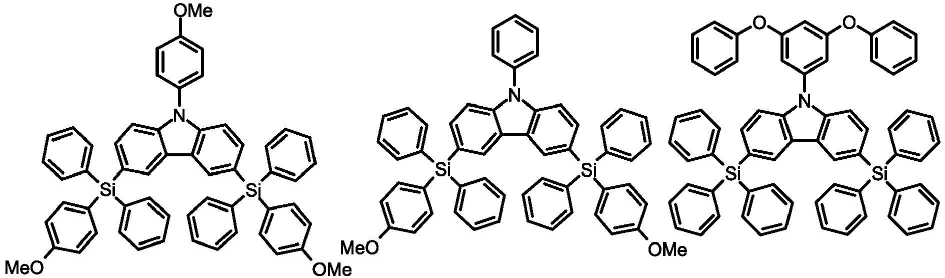 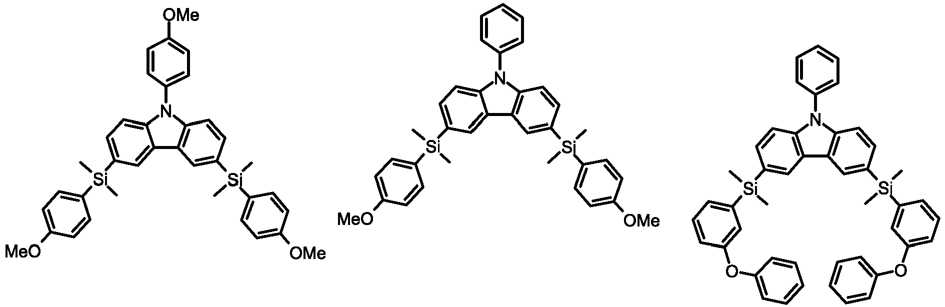 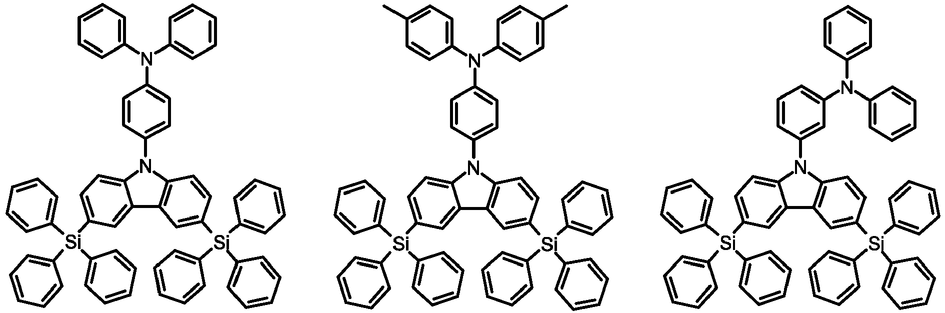 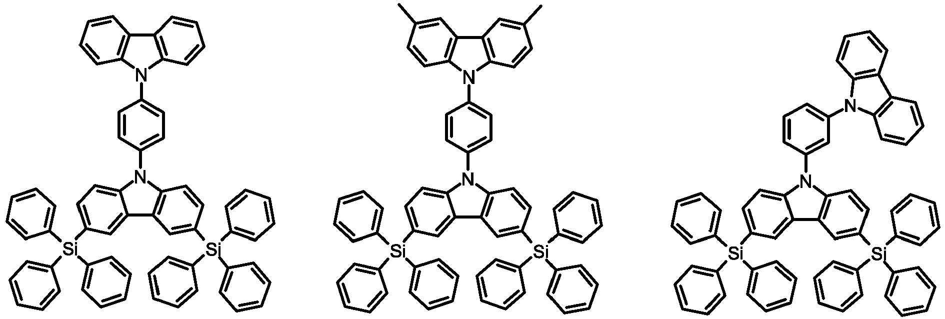 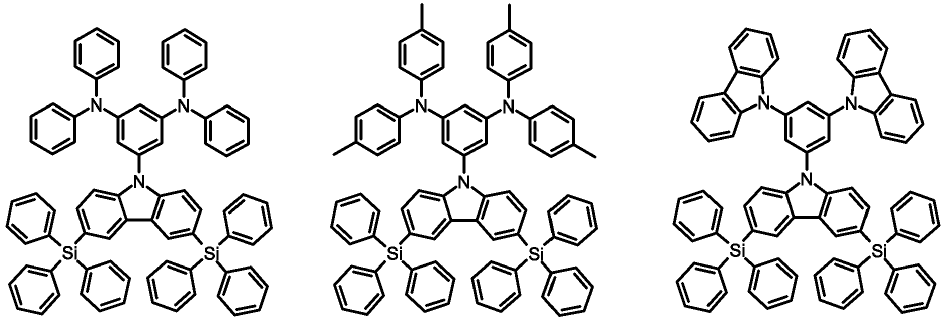 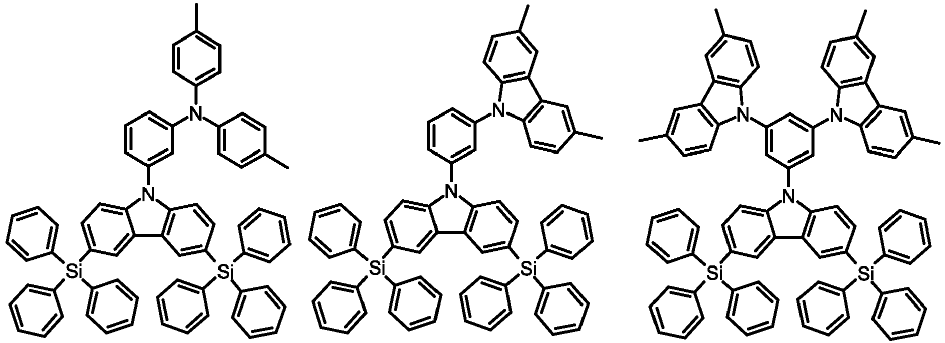
   ib) Verbindungen der Formel (I), die besonders bevorzugt als Matrix und/oder Loch/Excitonenblocker geeignet sind: 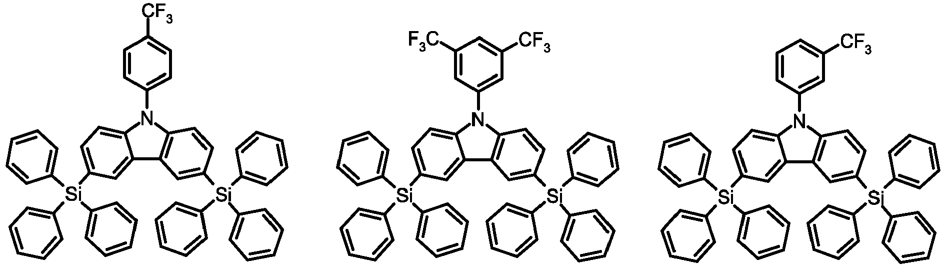 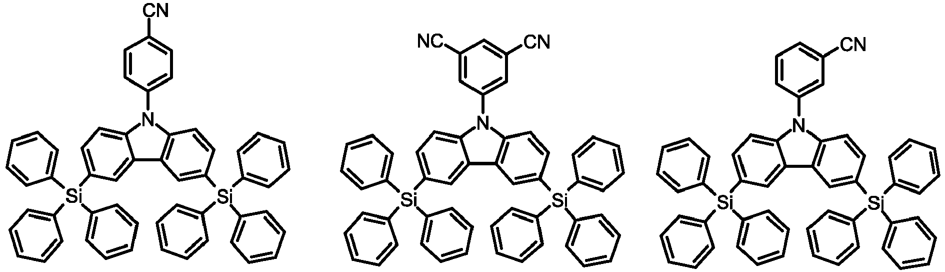 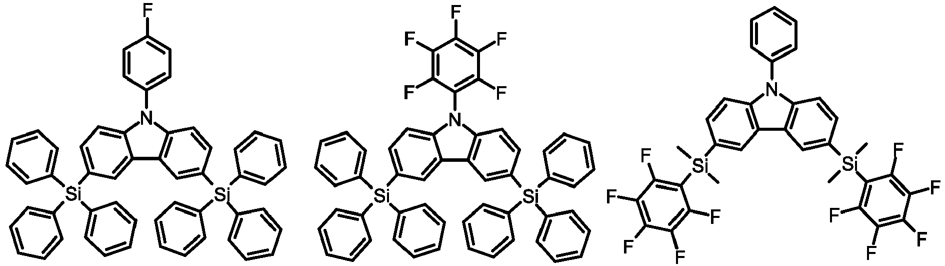 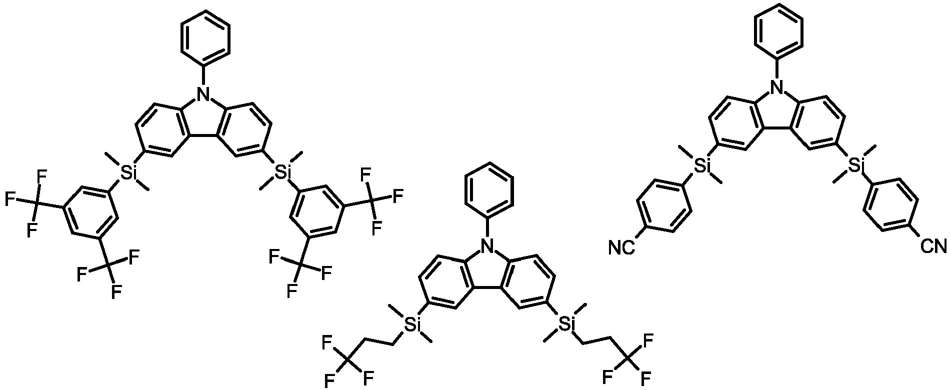 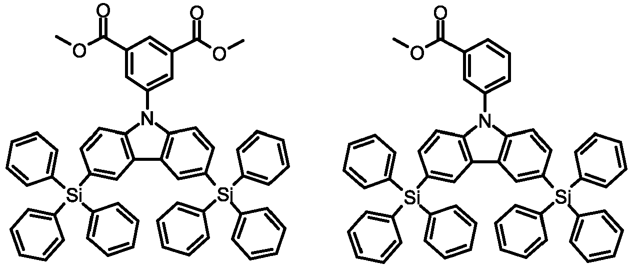 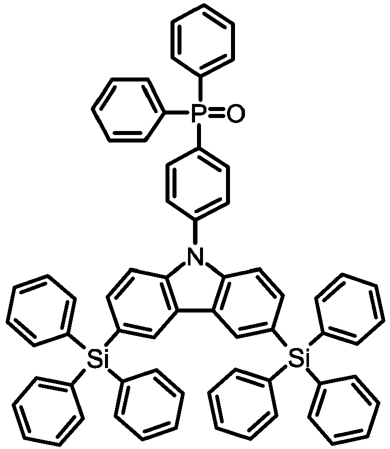 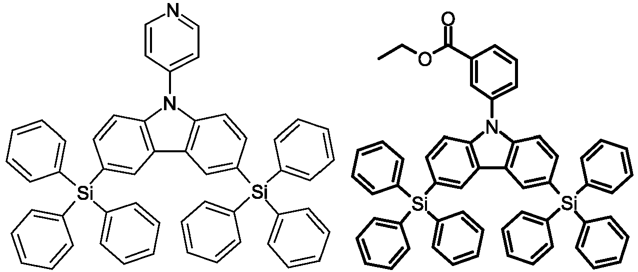 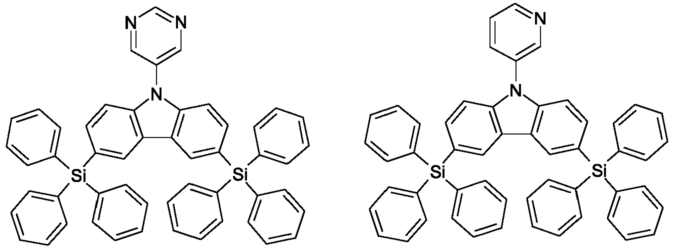 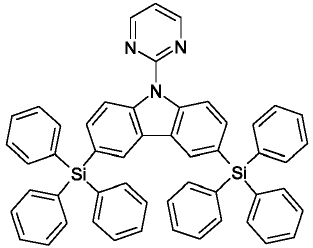 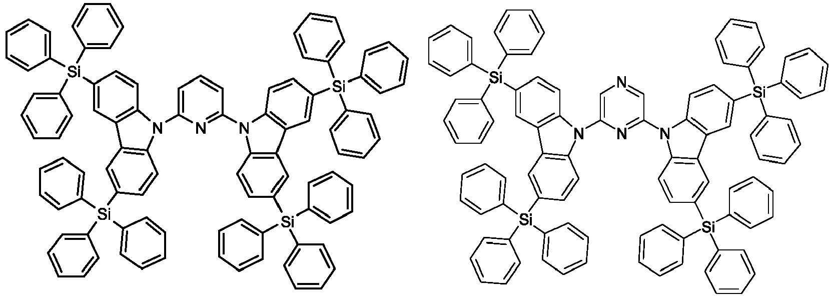 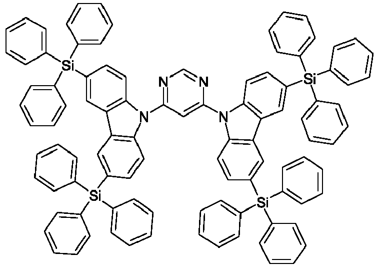 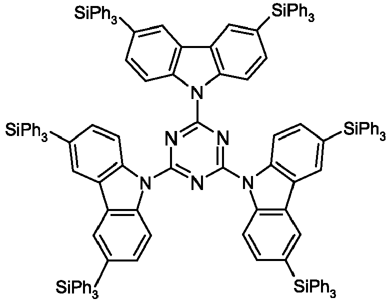
   ic) Verbindungen der Formel (I), die besonders bevorzugt als Matrix geeignet sind: 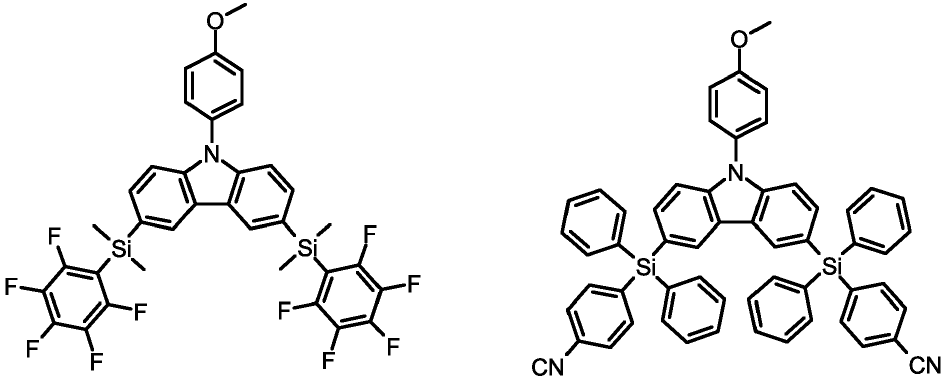
   id) weitere bevorzugte Verbindungen der Formel (I), die besonders bevorzugt als Matrix und/oder Loch/Excitonenblocker und/oder Elektronen/Excitonenblocker geeignet eingesetzt werden. 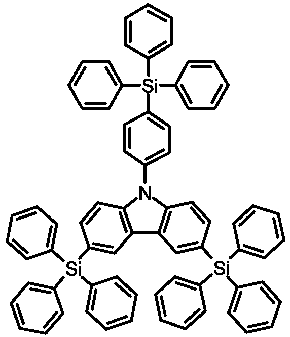 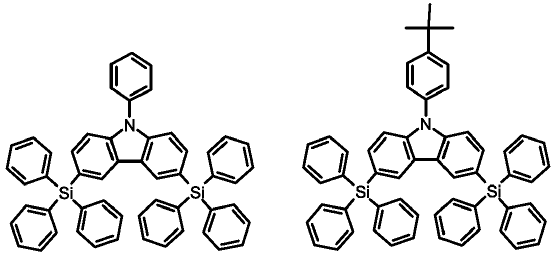 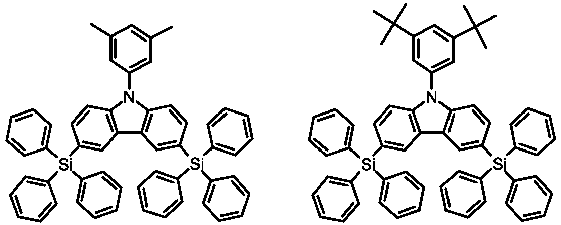 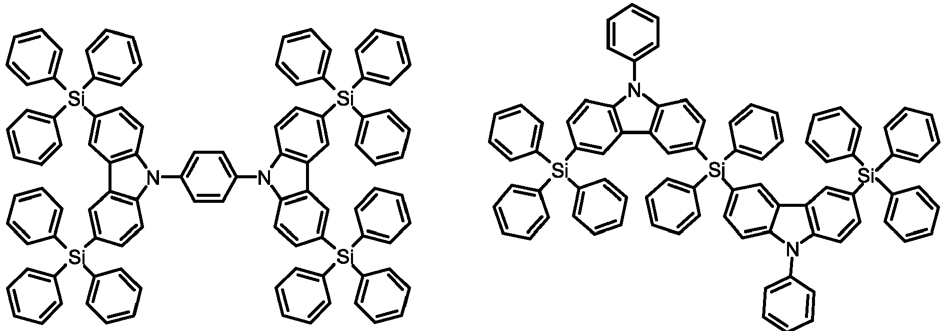 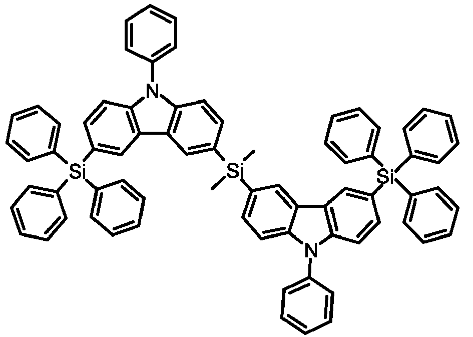 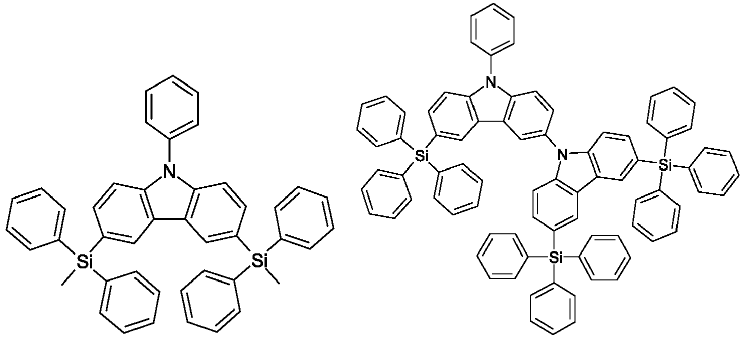 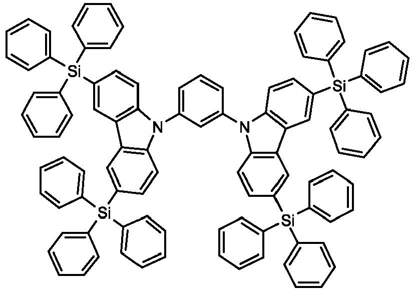 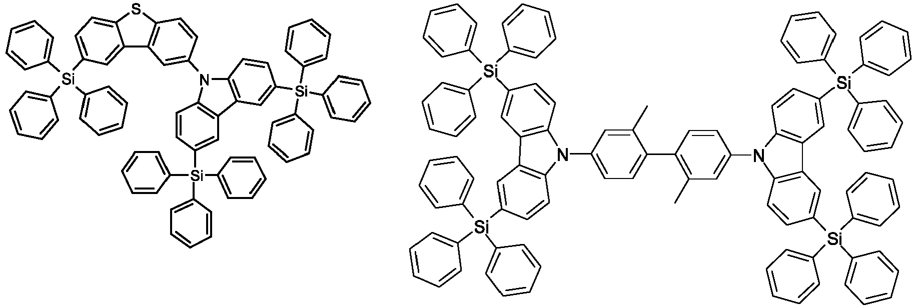 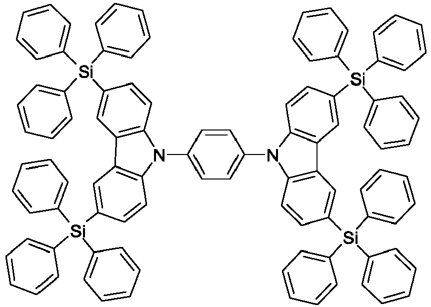
ii) Bevorzugte Verbindungen der Formel (I), worin X O, S, SO, SO₂ bedeutet:
   iia) Verbindungen der Formel (I), die besonders bevorzugt als Matrix und/oder Elektronen/Excitonenblocker geeignet sind: 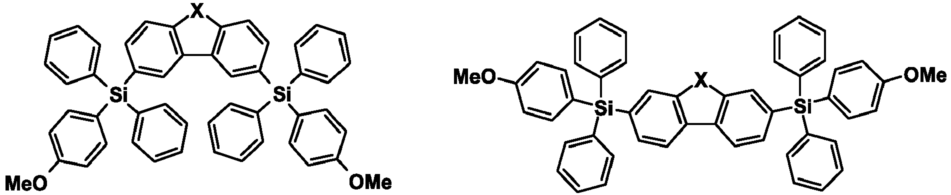 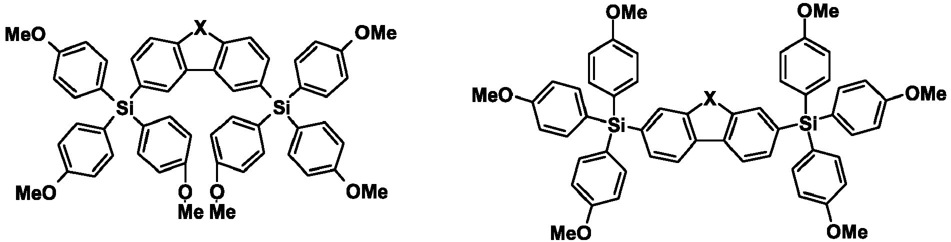 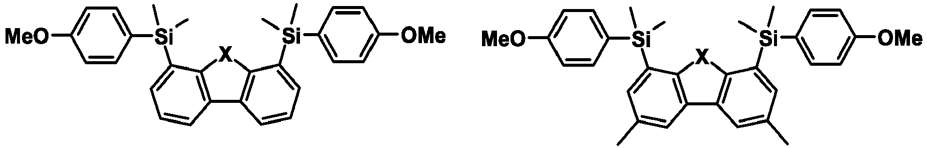 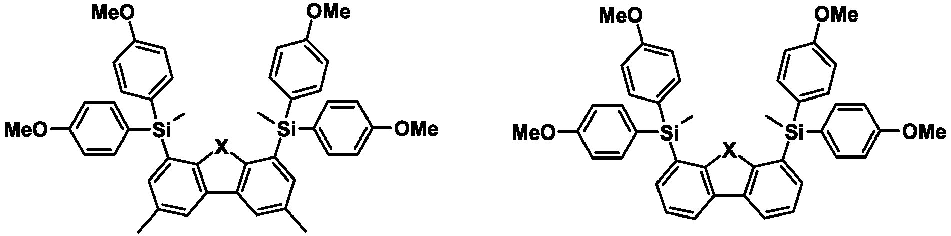
   iib) Verbindungen der Formel (I), die besonders bevorzugt als Matrix und/oder Loch/Excitonenblocker geeignet sind:

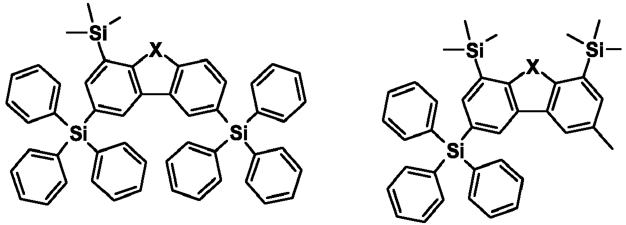

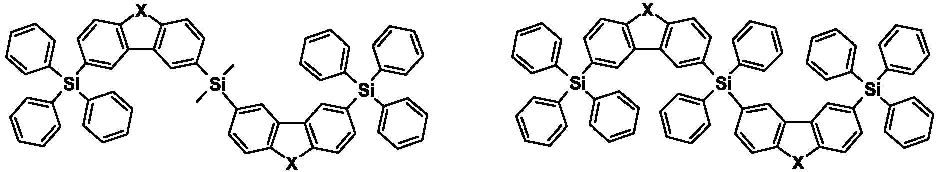

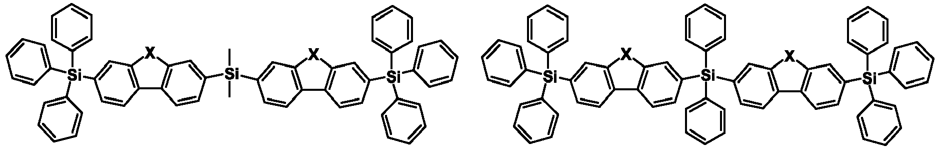

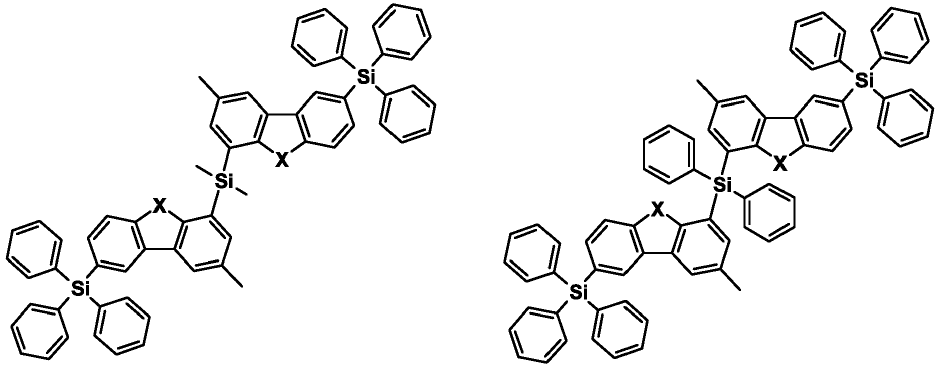

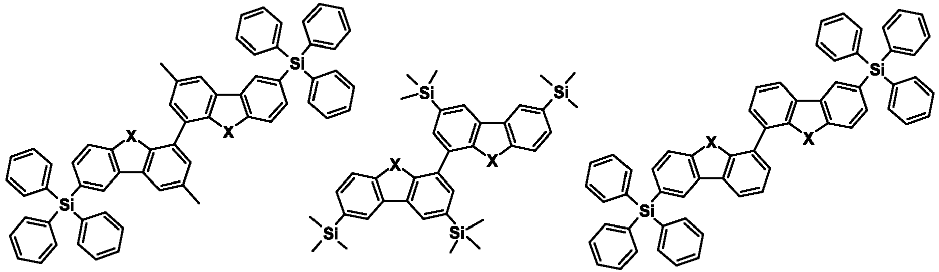

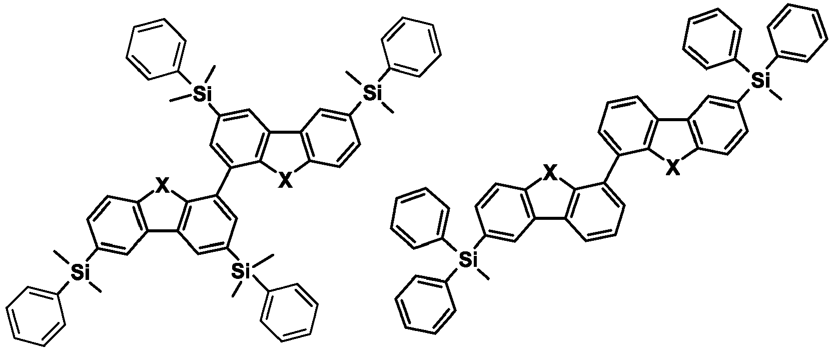

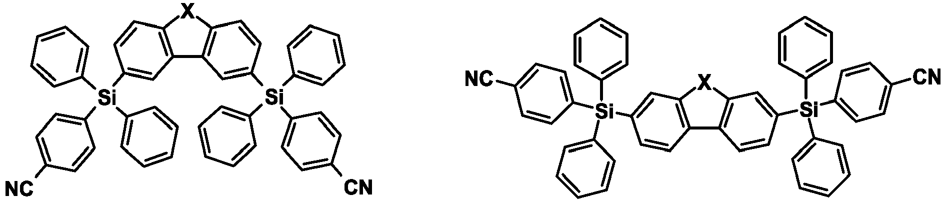

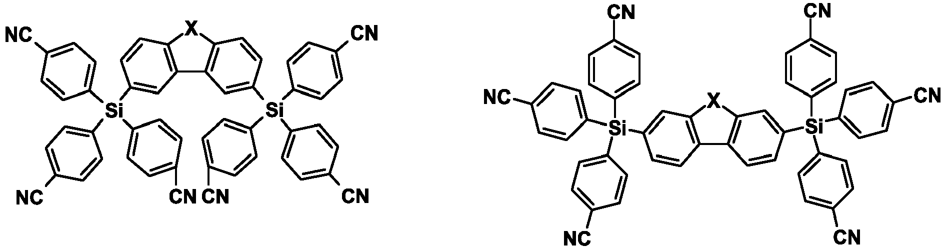

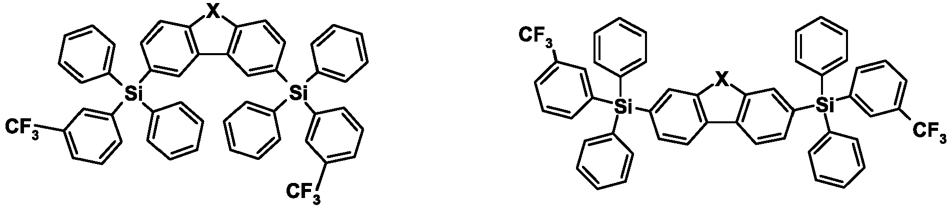

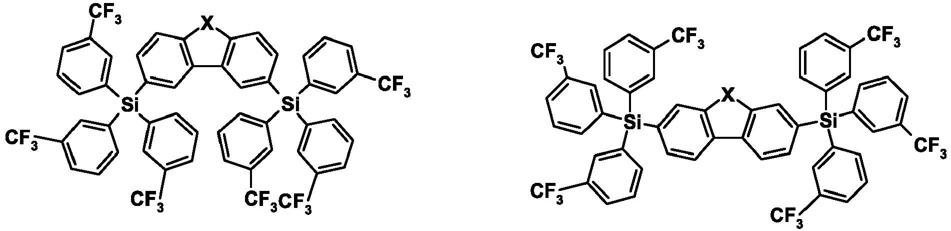

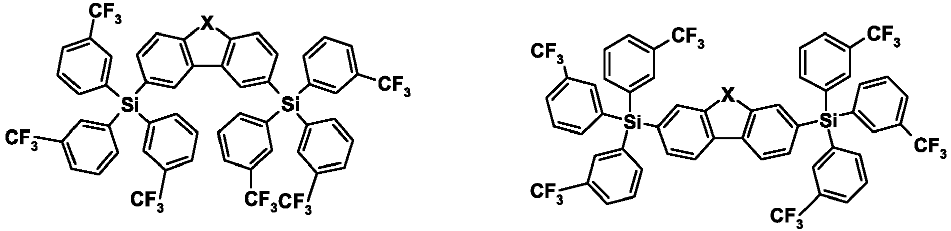

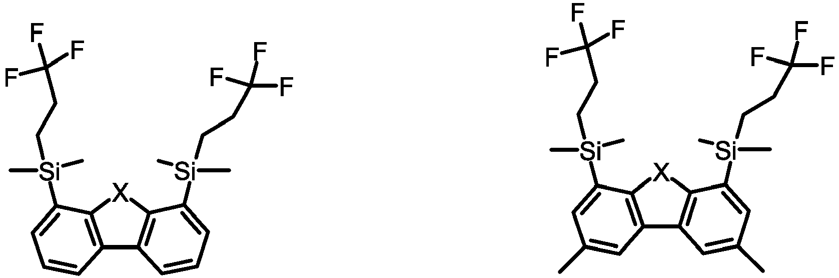

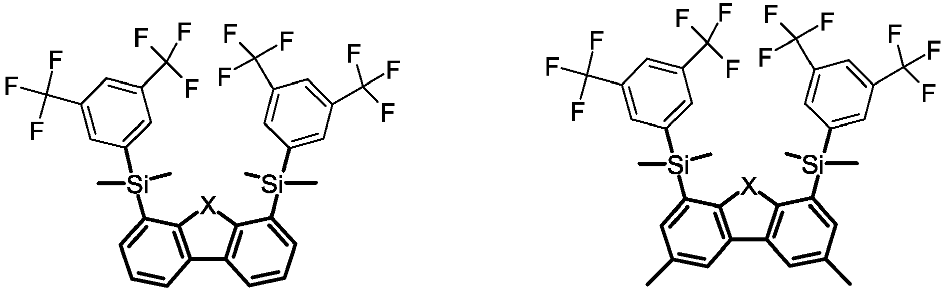

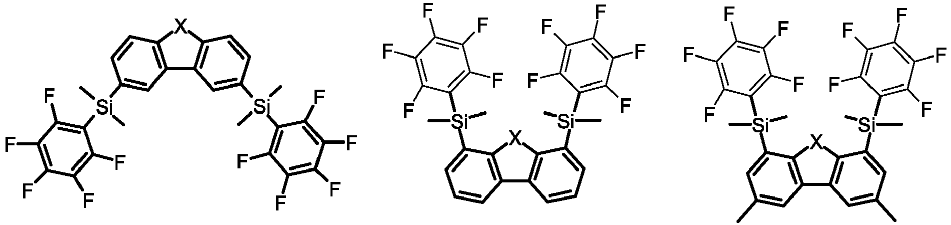

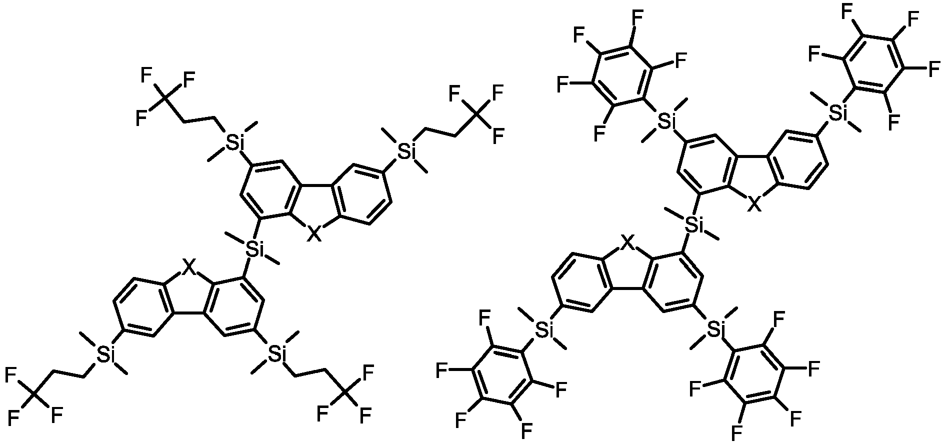

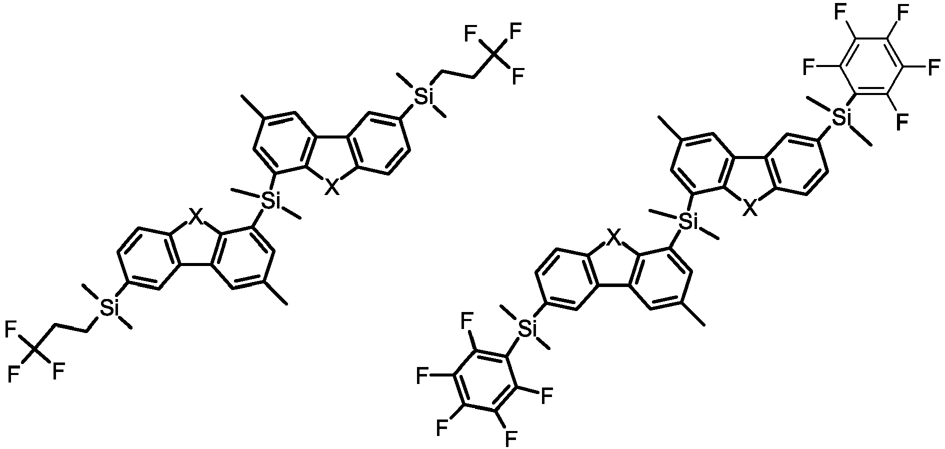

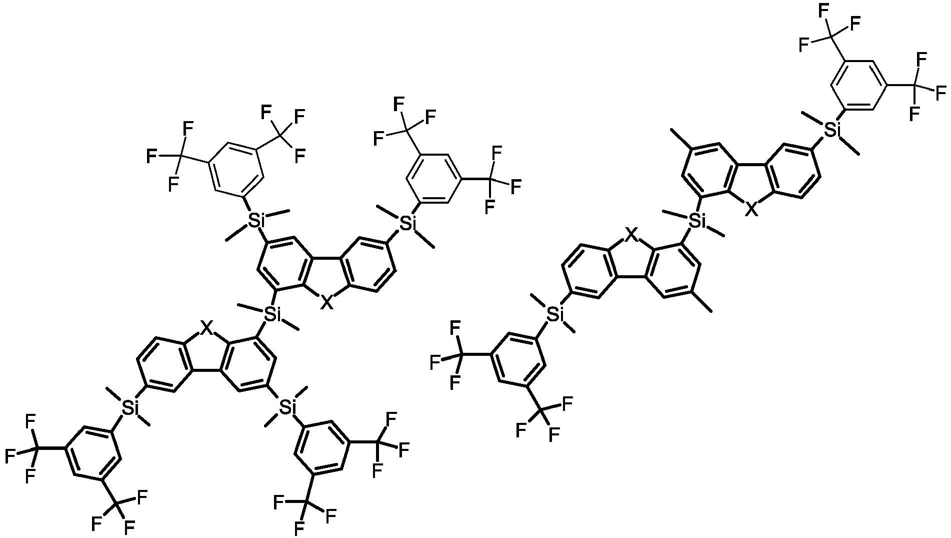

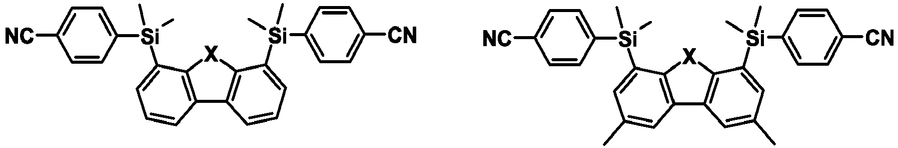

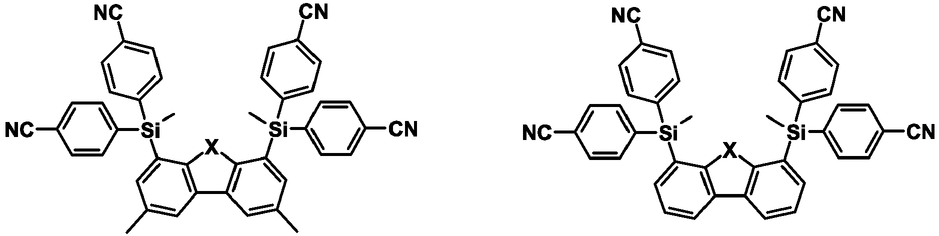

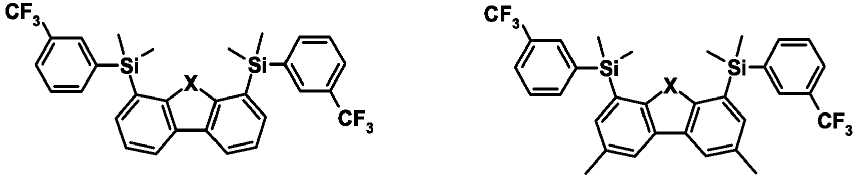

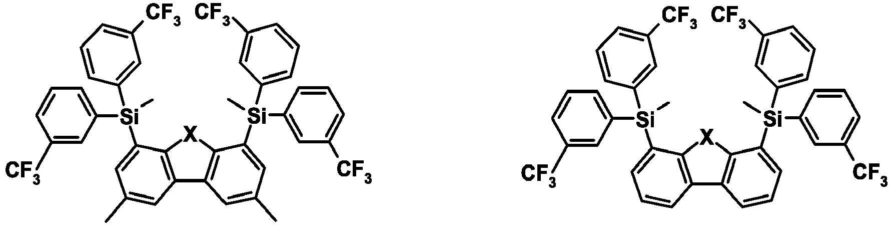

X in den unter iia) und iib) genannten Formeln bedeutet bevorzugt O, S, SO oder SO₂.

Offenbart wird ferner eine organische Leuchtdiode, in der eine Verbindung der Formel (I) eingesetzt wird, worin der Rest R¹, und/oder mindestens einer der Reste aus der Gruppe R², R³ und R⁴ und/oder mindestens einer der Reste aus der Gruppe R⁵, R⁶ und R⁷ unabhängig voneinander substituiertes oder unsubstituiertes C₆-Aryl der folgenden Formel bedeuten: 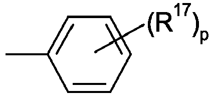 worin bedeuten
- p: 0, 1, 2, 3, 4 oder 5, bevorzugt 0, 1, 2 oder 3, besonders bevorzugt 0, 1 oder 2;
- R¹⁷: Wasserstoff, substituiertes oder unsubstituiertes C₁-C₂₀-Alkyl, substituiertes oder unsubstituiertes C₆-C₃₀-Aryl, substituiertes oder unsubstituiertes Heteroaryl mit 5 bis 30 Ringatomen, ein Substituent mit Donor- oder Akzeptorwirkung, wobei geeignete Substituenten mit Donor- oder Akzeptorwirkung vorstehend genannt sind, oder ein Rest der allgemeinen Formel a oder b 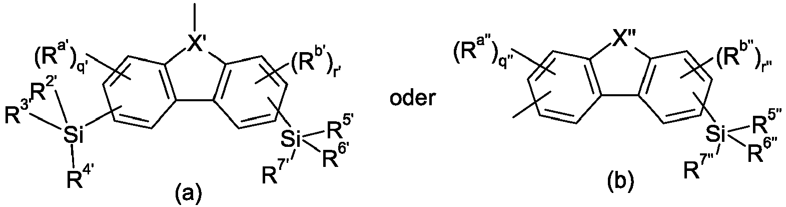
worin
X' N oder P bedeutet, und
die Reste und Indizes X", R^{2'}, R^{3'}, R^{4'}, R^{5'}, R^{5"}, R^{6'}, R^{6"}, R^{7'}, R^{7"}, R^{a'}, R^{a"}, R^{b'}, R^{b"}, q', q", r' und r" unabhängig voneinander für die Reste und Indizes X, R², R³, R⁴, R⁵, R⁶, R⁷, R^{a}, R^{b}, q und r genannten Bedeutungen aufweisen;
oder
einer der Reste R², R³ oder R⁴ und/oder einer der Reste R⁵, R⁶ oder R⁷ ein Rest der allgemeinen Formel c ist 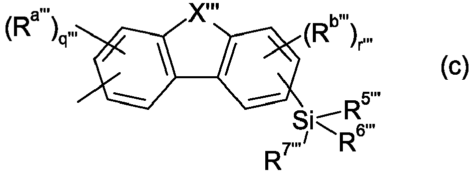
worin die Reste und Indizes X"', R^{5'''}, R^{6'''}, R^{7'''}, R^{a'''}, R^{b'''}, q''' und r''' unabhängig voneinander die für die Reste und Indizes X, R⁵, R⁶, R⁷, R^{a}, R^{b}, q und r genannten Bedeutungen aufweisen.

Bevorzugte Reste R¹⁷ sind ausgewählt aus der Gruppe bestehend aus Wasserstoff, substituiertem oder unsubstituiertem C₁-C₆-Alkyl, substituiertem oder unsubstituiertem C₆-C₁₀-Aryl, substituiertem oder unsubstituiertem Heteroaryl mit 5 bis 13 Ringatomen, bevorzugt Carbazolyl, einem Substituenten mit Donor- oder Akzeptorwirkung ausgewählt aus der Gruppe bestehend aus C₁- bis C₂₀-Alkoxy, bevorzugt C₁-C₆-Alkoxy, besonders bevorzugt Ethoxy oder Methoxy; C₆-C₃₀-Aryloxy, bevorzugt C₆-C₁₀-Aryloxy, besonders bevorzugt Phenyloxy; SiR¹⁴R¹⁵R¹⁶; Halogenresten, bevorzugt F, Cl, Br, besonders bevorzugt F oder Cl, ganz besonders bevorzugt F, halogenierten C₁-C₂₀-Alkylresten, bevorzugt halogenierten C₁-C₆-Alkylresten, ganz besonders bevorzugt fluorierten C₁-C₆-Alkylresten, z. B. CF₃, CH₂F, CHF₂ oder C₂F₅; Amino, bevorzugt Dimethylamino, Diethylamino oder Diphenylamino, besonders bevorzugt Diphenylamino; OH, Pseudohalogenresten, bevorzugt CN, SCN oder OCN, besonders bevorzugt CN; C(O)OC₁-C₄-Alkyl, bevorzugt -C(O)OMe, P(O)Ph₂, SO₂Ph, wobei R¹⁴, R¹⁵ und R¹⁶ unabhängig voneinander substituiertes oder unsubstituiertes C₁- bis C₆-Alkyl oder substituiertes oder unsubstituiertes C₆- bis C₁₀-Aryl bedeuten und - im Falle von SiR¹⁴R¹⁵R¹⁶ - bevorzugt unabhängig voneinander substituiertes oder unsubstituiertes C₁-C₂₀-Alkyl oder substituiertes oder unsubstituiertes Phenyl bedeuten; besonders bevorzugt ist mindenstens einer der Reste R¹⁴,R¹⁵ oder R¹⁶ substituiertes oder unsubstituiertes Phenyl, ganz besonders bevorzugt ist mindenstens einer der Reste R¹⁴,R¹⁵ und R¹⁶ substituiertes Phenyl, wobei geeignete Substituenten vorstehend genannt sind. Besonders bevorzugt sind die Reste R¹⁷ unabhängig voneinander ausgewählt aus der Gruppe bestehend aus Methoxy, Phenyloxy, unsubstituiertem C₁-C₄-Alkyl, bevorzugt Methyl, halogeniertem C₁-C₄-Alkyl, bevorzugt CF₃, CHF₂, CH₂F, C₂F₅, CN, Halogen, bevorzugt F, -C(O)O-C₁-C₄-Alkyl, bevorzugt -C(O)OMe, P(O)Ph₂, und substituiertem oder unsubstituiertem Heteroaryl mit 5 bis 13 Ringatomen, bevorzugt Carbazolyl, wobei in dem Fall, wenn die Verbindung der allgemeinen Formel (I) ausschließlich in der Licht-emittierenden Schicht oder in der Licht-emittierenden Schicht und in der Lochleiterschicht vorliegt und die Gruppe X NR¹ bedeutet, mindestens einer der Reste R¹ bis R⁷, R^{a} oder R^{b} in den Verbindungen der Formel (I) mindestens ein Heteroatom enthält.

In einer weiteren Ausführungsform der vorliegenden Erfindung bedeuten die Indizes r und q in den Verbindungen der Formel (I) 0, d. h. alle substituierbaren Positionen der Arylgruppen tragen Wasserstoffatome. Für alle anderen Reste und Indizes gelten die vorstehend genannten Bedeutungen.

Die erfindungsgemäßen Verbindungen der Formel (I) können in verschiedenen Schichten der organischen Leuchtdiode eingesetzt werden, wobei geeignete und bevorzugte Schichtfolgen in den OLEDs vorstehend genannt sind.

Ein Aspekt betrifft organische Leuchtdioden, worin die Verbindungen der Formel (I) in der Licht-emittierenden Schicht E als Matrix eingesetzt werden, wobei in dem Fall, wenn die Verbindung der allgemeinen Formel (I) ausschließlich in der Licht-emittierenden Schicht oder in der Licht-emittierenden Schicht und in der Lochleiterschicht vorliegt und die Gruppe X NR¹ bedeutet, mindestens einer der Reste R¹ bis R⁷, R^{a} oder R^{b} in den Verbindungen der Formel (I) mindestens ein Heteroatom enthält.

Offenbart wird ferner eine organische Leuchtdiode, worin die Verbindungen der Formel (I) in der Blockschicht für Elektronen als Elektronen/Excitonenblocker und/oder in der Loch-Injektionsschicht und/oder in der Lochleiterschicht eingesetzt werden, wobei in dem Fall, wenn die Verbindung der allgemeinen Formel (I) in der Licht-emittierenden Schicht und in der Lochleiterschicht vorliegt und die Gruppe X NR¹ bedeutet, mindestens einer der Reste R¹ bis R⁷, R^{a} oder R^{b} in den Verbindungen der Formel (I) mindestens ein Heteroatom enthält. Es ist ebenfalls möglich, dass die Verbindungen der Formel (I) des Weiteren in der Licht-emittierenden Schicht E und/oder einer oder mehreren der nachstehend genannten Schichten vorliegen.

Offenbart wird ferner eine organische Leuchtdiode, worin die Verbindungen der Formel (I) in der Blockschicht für Löcher als Loch/Excitonenblocker und/oder in der Elektronen-Injektionsschicht und/oder in der Elektronenleiterschicht eingesetzt werden. Es ist ebenfalls möglich, dass die Verbindungen der Formel (I) in der Licht-emittierenden Schicht E und/oder einer oder mehreren der vorstehend genannten Schichten vorliegen.

In Abhängigkeit davon, in welcher Schicht die Verbindungen der Formel (I) eingesetzt werden, weisen die Verbindungen der Formel (I) unterschiedliche bevorzugte Reste R¹, R², R³, R⁴, R⁵, R⁶, R⁷, R^{a} und R^{b} und unterschiedliche Gruppen X auf. Neben der Funktion der Schicht, worin die Verbindungen der Formel (I) in der OLED eingesetzt werden können, sind die Reste R¹ bis R⁷, R^{a} und R^{b} und die Gruppe X der Verbindungen der Formel (I) des Weiteren von den elektronischen Eigenschaften (relative Lagen der HOMOs und LUMOs) der jeweiligen in der OLED eingesetzten Schichten abhängig. Somit ist es möglich, durch geeignete Substitution der Verbindungen der Formel (I) die HOMO und LUMO-Orbitallagen an die weiteren in der OLED eingesetzten Schichten anzupassen und so eine hohe Stabilität der OLED und damit eine lange operative Lebensdauer und gute Effizienzen zu erreichen.

Die Grundsätze betreffend die relativen Lagen von HOMO und LUMO in den einzelnen Schichten einer OLED sind dem Fachmann bekannt. Im Folgenden sind die Grundsätze beispielhaft bezüglich der Eigenschaften der Blockschicht für Elektronen und der Blockschicht für Löcher im Verhältnis zur Licht-emittierenden Schicht aufgeführt:
Das LUMO der Blockschicht für Elektronen liegt energetisch höher als das LUMO der in der Licht-emittierenden Schicht eingesetzten Materialien (sowohl des Emittermaterials auch gegebenenfalls eingesetzter Matrixmaterialien). Je größer die energetische Differenz der LUMOs der Blockschicht für Elektronen und der Materialien in der Lichtemittierenden Schicht ist, desto besser sind die Elektronen- und/oder Excitonen-blockierenden Eigenschaften der Blockschicht für Elektronen. Geeignete Substitutionsmuster der als Elektronen- und/oder Excitonenblockermaterialien geeigneten Verbindungen der Formel (I) sind somit unter anderem abhängig von den elektronischen Eigenschaften (insbesondere der Lage des LUMOs) der in der Licht-emittierenden Schicht eingesetzten Materialien.

Das HOMO der Blockschicht für Elektronen liegt energetisch höher als die HOMOs der in der Licht-emittierenden Schicht vorliegenden Materialien (sowohl der Emittermaterialien als auch der gegebenenfalls vorliegenden Matrixmaterialien). Je größer die energetische Differenz der HOMOs der Blockschicht für Löcher und der in der Licht-emittierenden Schicht vorliegenden Materialien ist, desto besser sind die Loch- und/oder Excitonen-blockierenden Eigenschaften der Blockschicht für Löcher. Geeignete Substitutionsmuster der als Loch- und/oder Excitonenblockermaterialien geeigneten Verbindungen der Formel (I) sind somit unter anderem abhängig von den elektronischen Eigenschaften (insbesondere der Lage der HOMOs) der in der Licht-emittierenden Schicht vorliegenden Materialien.

Vergleichbare Überlegungen betreffend die relative Lage der HOMOs und LUMOs der unterschiedlichen in der OLED eingesetzten Schichten gelten für die weiteren gegebenenfalls in der OLED eingesetzten Schichten und sind dem Fachmann bekannt.

Bevorzugt geeignete Reste R¹, R², R³, R⁴, R⁵, R⁶, R⁷, R^{a} und R^{b} der Verbindungen der Formel (I) in Abhängigkeit von deren Einsatz in verschiedenen Schichten der OLED sind nachstehend genannt. Es wird darauf hingewiesen, dass auch andere als die nachstehend genannten bevorzugten Substitutionen der Verbindungen der Formel (I) zum Einsatz in den verschiedenen Schichten - in Abhängigkeit von den elektronischen Eigenschaften der weiteren Schichten der OLED, insbesondere in Abhängigkeit von den elektronischen Eigenschaften der Licht-emittierenden Schicht - grundsätzlich geeignet sein können.

*Verbindungen der allgemeinen Formel (I), die insbesondere für den Einsatz in der Licht-emittierenden Schicht E als Matrixmaterialien sowie für den Einsatz in der Blockschicht für Elektronen, der Loch-Injektionsschicht und*/*oder der Lochleiterschicht geeignet sind*

Eine Aspekt betrifft eine organische Leuchtdiode, worin die Verbindungen der Formel (I) in einer Blockschicht für Elektronen, in einer Loch-Injektionsschicht und/oder in einer Lochleiterschicht und/oder in der Licht-emittierenden Schicht E als Matrixmaterialien eingesetzt werden.

Bevorzugte Verbindungen der Formel (I), die in mindestens einer der vorstehend genannten Schichten eingesetzt werden können, weisen mindestens einen Rest R¹, R², R³, R⁴, R⁵, R⁶ oder R⁷ auf, der substituiertes oder unsubstituiertes C₁-C₂₀-Alkyl, Heteroaryl mit 5 bis 30 Ringatomen, mit mindestens einem Substituenten mit Donorwirkung substituiertes C₆-C₃₀-Aryl oder mit Heteroaryl mit 5 bis 30 Ringatomen substituiertes C₆-C₃₀-Aryl oder ein Substituent mit Donorwirkung oder, im Falle von R², R³, R⁴, R⁵, R⁶ oder R⁷, Wasserstoff ist, wobei in dem Fall, wenn die Verbindung der allgemeinen Formel (I) ausschließlich in der Licht-emittierenden Schicht oder in der Licht-emittierenden Schicht und in der Lochleiterschicht vorliegt und die Gruppe X NR¹ bedeutet, mindestens einer der Reste R¹ bis R⁷, R^{a} oder R^{b} in den Verbindungen der Formel (I) mindestens ein Heteroatom enthält.

Geeignete Substituenten mit Donorwirkung (elektronenschiebende Reste) sind ausgewählt aus der Gruppe bestehend aus substituiertem oder unsubstituiertem C₁-C₆-Alkyl, bevorzugt Methyl, substituiertem oder unsubstituiertem C₆-C₁₀-Aryl, substituiertem oder unsubstituiertem elektronenreichem Heteroaryl mit fünf bis 30 Ringatomen, bevorzugt ausgewählt aus der Gruppe bestehend aus Carbazolyl, Pyrrolyl, Imidazolyl, Pyrazolyl, Triazolyl, Oxazolyl, Thiophenyl, bevorzugt Carbazolyl, Pyrrolyl und Thiophenyl, besonders bevorzugt Carbazolyl, C₁-C₂₀-Alkoxy, bevorzugt C₁-C₆-Alkoxy, besonders bevorzugt Methoxy und Ethoxy, C₆-C₃₀-Aryloxy, bevorzugt C₆-C₁₀-Aryloxy, besonders bevorzugt Phenyloxy, C₁-C₂₀₋Alkylthio, bevorzugt C₁-C₆-Alkylthio, besonders bevorzugt -SCH₃, C₆-C₃₀-Arylthio, bevorzugt C₆-C₁₀-Arylthio, besonders bevorzugt -SPh, F, SiR¹⁴R¹⁵R¹⁶, wobei R¹⁴, R¹⁵ und R¹⁶ bevorzugt donor-substituierte Phenylgruppen bedeuten, Amino (-NR¹⁴R¹⁵), bevorzugt Diphenylamino, Phosphin (-PR¹⁴R¹⁵), Hydrazinresten, OH, Donor-substituierten Vinylgruppen, wobei R¹⁴, R¹⁵ und R¹⁶ die vorstehend genannten Bedeutungen aufweisen und bevorzugt Donor-substituierte Phenylgruppen bedeuten.

Ganz besonders bevorzugte Substituenten mit Donorwirkung sind ausgewählt aus der Gruppe bestehend aus Diphenylamino, Carbazolyl, Methoxy, Phenoxy, wobei Methoxy und Carbazolyl insbesondere ganz besonders bevorzugt sind.

Besonders bevorzugt handelt es sich bei dem mindestens einen Rest, der in den vorstehend genannten Schichten eingesetzt wird, um einen mit mindestens einem Substituenten mit Donorwirkung und/oder mindestens einem Heteroarylrest mit 5 bis 30 Ringatomen substituierten C₆-Arylrest der Formel (d) 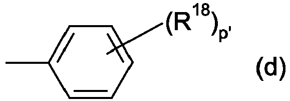 worin bedeuten:
- p': 1, 2, 3, 4 oder 5, bevorzugt 1, 2 oder 3, besonders bevorzugt 1 oder 2, und
- R¹⁸: jeweils unabhängig voneinander substituiertes oder unsubstituiertes C₁-C₆-Alkyl, bevorzugt Methyl, substituiertes oder unsubstituiertes C₆-C₁₀-Aryl, C₁-C₂₀-Alkoxy, bevorzugt C₁-C₆-Alkoxy, besonders bevorzugt Methoxy und Ethoxy, C₆-C₃₀-Aryloxy, bevorzugt C₆-C₁₀-Aryloxy, besonders bevorzugt Phenyloxy, C₁-C₂₀-Alkylthio, bevorzugt C₁-C₆-Alkylthio, besonders bevorzugt -SCH₃, C₆-C₃₀-Arylthio, bevorzugt C₆-C₁₀-Arylthio, besonders bevorzugt -SPh, SiR¹⁴R¹⁵R¹⁶, wobei R¹⁴, R¹⁵ und R¹⁶ die vorstehend genannten Bedeutungen aufweisen und bevorzugt Donorsubstituierte Phenylgruppen bedeuten, Amino (-NR¹⁴R¹⁵), bevorzugt Diphenylamino, Amido (-NR¹⁴ (C = O (R¹⁵)), Phosphin (-PR¹⁴R¹⁵), Hydrazinreste, OH, Donor-substituierte Vinylgruppen, wobei R¹⁴, R¹⁵ und R¹⁶ die vorstehend genannten Bedeutungen aufweisen und bevorzugt Donor-substituierte Phenylgruppen bedeuten, oder R¹⁸ bedeutet substituiertes oder unsubstituiertes elektronenreiches Heteroaryl mit fünf bis 30 Ringatomen, bevorzugt ausgewählt aus der Gruppe bestehend aus Carbazolyl, Pyrrolyl, Imidazolyl, Pyrazolyl, Triazolyl, Oxazolyl, Thiophenyl, besonders bevorzugt Carbazolyl, Pyrrolyl und Thiophenyl.

Bevorzugte Gruppen R¹⁸ sind ausgewählt aus der Gruppe bestehend aus Methoxy, Ethoxy, Phenoxy, wobei Methoxy insbesondere ganz besonders bevorzugt ist, Carbazolyl, Pyrrolyl, Imidazolyl, Pyrazolyl, Triazolyl, Oxazolyl und Thiophenyl, wobei Methoxy, Phenyloxy, Carbazolyl und NR¹⁴R¹⁵ wobei R¹⁴ und R¹⁵ Phenyl oder Tolyl bedeuten, ganz besonders bevorzugt sind.

Besonders bevorzugt weisen die in den vorstehend genannten Schichten eingesetzten Verbindungen der Formel (I) mindestens einen Rest R¹, R², R³, R⁴, R⁵, R⁶ oder R⁷ auf, ausgewählt aus der Gruppe bestehend aus

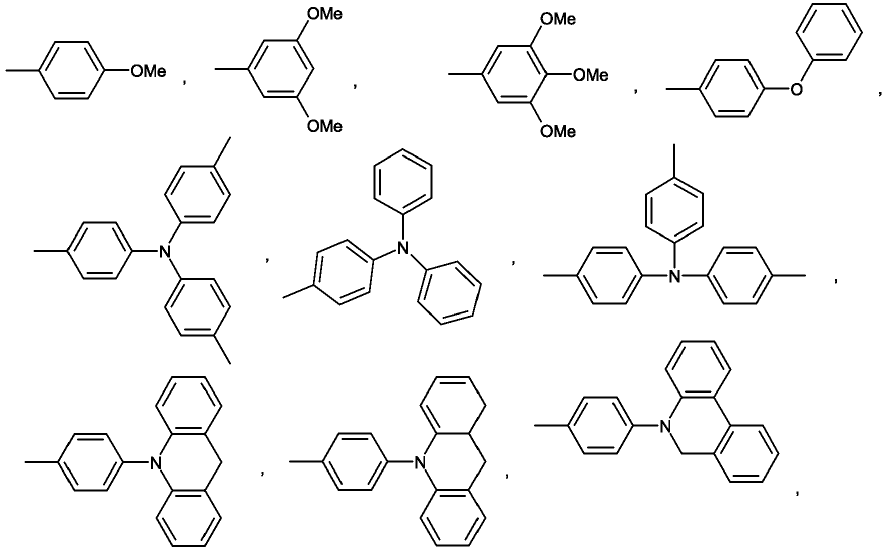

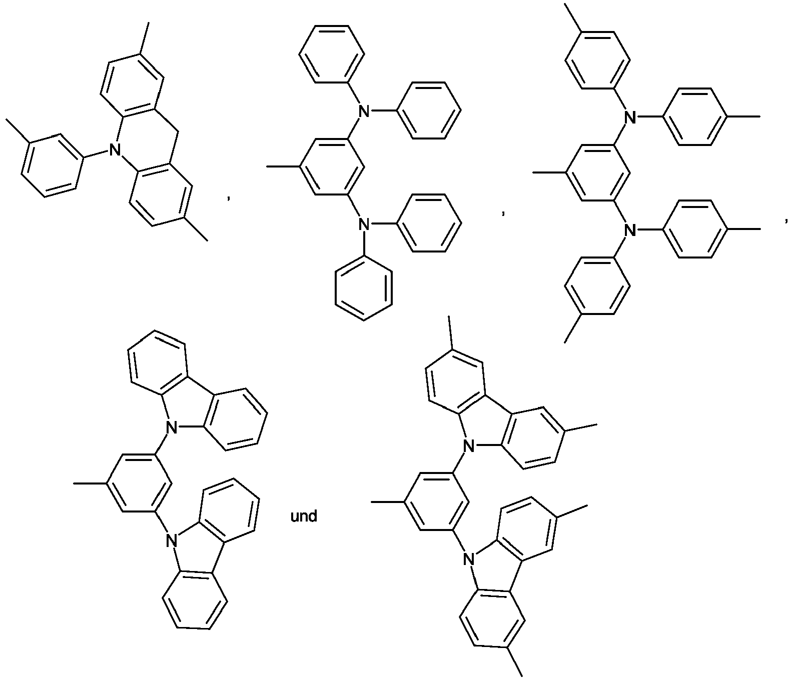

In einer bevorzugten Ausführungsform ist mindestens der Rest R¹ ein mit mindestens einem Substituenten mit Donorwirkung und/oder mindestens einem Heteroarylrest mit 5 bis 30 Ringatomen substituierter C₆-Arylrest der Formel (d);
und die Reste R², R³, R⁴, R⁵, R⁶ und R⁷ bedeuten bevorzugt Phenyl, Methyl oder mit Methoxy oder Phenyloxy substituiertes Phenyl.

### Einsatz der Verbindungen der Formel (I) in der Licht-emittierenden Schicht E als Matrixmaterial und/oder in einer Blockschicht für Löcher, einer Elektronen-Injektionsschicht und/oder einer Elektronenleiterschicht

Ein weiterer Gegenstand betrifft eine organische Leuchtdiode, worin in mindestens einer der Schichten ausgewählt aus Licht-emittierender Schicht E, Blockschicht für Löcher, Elektronen-Injektionsschicht und Elektronenleiterschicht mindestens eine Verbindung der Formel (I) vorliegt.

Bevorzugte in den vorstehend genannten Schichten eingesetzte Verbindungen der Formel (I) weisen mindestens einen Rest R¹, R², R³, R⁴, R⁵, R⁶ oder R⁷ auf, der mit mindestens einem Substituenten mit Akzeptorwirkung (elektronenziehender Rest) substituiertes C₁- bis C₂₀-Alkyl, mit mindestens einem Substituenten mit Akzeptorwirkung substituiertes C₆-C₃₀-Aryl, mit mindestens einem Heteroarylrest mit 5 bis 30 Ringatomen substituiertes C₆-C₃₀-Aryl oder ein Substituent mit Akzeptorwirkung ist.

Geeignete Substituenten mit Akzeptorwirkung (elektronenziehende Reste) sind ausgewählt aus der Gruppe bestehend aus elektronenarmen Heteroarylen mit 5 bis 30 Ringatomen, Carbonyl (-CO(R¹⁴)), Carbonylthio (-C = O (SR¹⁴)), Carbonyloxy (-C = O (OR¹⁴)), Oxycarbonyl (-OC = O (R¹⁴)), Thiocarbonyl (-SC = O(R¹⁴)), OH, Halogen, mit Halogen substituiertem C₁-C₂₀-Alkyl, Pseudohalogenresten, Amido (-C = O (NR¹⁴), Phosphonat (-P(O) (OR¹⁴)₂), Phosphat (-OP (O) (OR¹⁴)₂), Phosphinoxid (-P(O)R¹⁴R¹⁵), Sulfonyl (-S(O)₂R¹⁴), Sulfonat (-S(O)₂OR¹⁴) , Sulfat (-OS(O)₂OR¹⁴), Sulfoxid (-S(O)R¹⁴), Sulfonamid (-S(O)₂NR¹⁴R¹⁵), NO₂, Boronsäureestern (-OB(OR¹⁴)₂, Imino (-C = NR¹⁴R¹⁵)), Hydrazonresten, Oximresten, Nitroso-Gruppen, Diazo-Gruppen, Sulfoximinen, SiR¹⁴R¹⁵R¹⁶, , Boranresten, Stannanresten, akzeptorsubstituierten Vinylgruppen, Boroxine und Borazinen, wobei R¹⁴, R¹⁵ und R¹⁶ substituiertes oder unsubstituiertes C₁-C₂₀-Alkyl, bevorzugt substituiertes oder unsubstituiertes C₁-C₆-Alkyl, oder substituiertes oder unsubstituiertes C₆-C₃₀-Aryl, bevorzugt substituiertes oder unsubstituiertes C₆-C₁₀-Aryl bedeuten.

Bevorzugte Substituenten mit Akzeptorwirkung sind ausgewählt aus der Gruppe bestehend aus Halogen, bevorzugt F, mit Halogen substituiertem Alkyl, bevorzugt CF₃, CH₂F, CHF₂, C₂F₅, C₃F₃H₄, Pseudohalogen, bevorzugt CN, Carbonyloxy (-C = O (OR¹⁴)), bevorzugt -C = O (OCH₃), Phosphinoxid, bevorzugt P(O)Ph₂ und Sulfonyl, bevorzugt S(O)₂Ph₂.

Besonders bevorzugt handelt es sich bei dem mindestens einen Rest, der in den vorstehend genannten Schichten eingesetzt wird, um einen substituierten C₆-Arylrest der Formel (e) 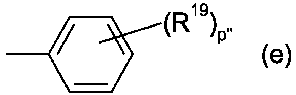 worin bedeuten:
- p": 1, 2, 3, 4 oder 5, bevorzugt 1, 2 oder 3, besonders bevorzugt 1 oder 2; und
- R¹⁹: Carbonyl (-CO(R¹⁴)), Carbonylthio (-C = O (SR¹⁴)), Carbonyloxy (-C = O (OR¹⁴)), Oxycarbonyl (-OC = O (R¹⁴)), Thiocarbonyl (-SC = O(R¹⁴)), OH, Halogen, mit Halogen substituiertes C₁-C₂₀-Alkyl, Pseudohalogenreste, Amido (-C = O (NR¹⁴), Phosphonat (-P(O) (OR¹⁴)₂), Phosphat (-OP (O) (OR¹⁴)₂), Phosphinoxid (-P(O)R¹⁴R¹⁵), Sulfonyl (-S(O)₂R¹⁴), Sulfonat (-S(O)₂OR¹⁴), Sulfat (-OS(O)₂OR¹⁴), Sulfoxid (-S(O)R¹⁴), Sulfonamid (-S(O)₂NR¹⁴R¹⁵), NO₂, Boronsäureester (-OB(OR¹⁴)₂, Imino (-C = NR¹⁴R¹⁵)), Hydrazonreste, Oximreste, Nitroso-Gruppen, Diazo-Gruppen, Sulfoximine, SiR¹⁴R¹⁵R¹⁶, und, Boranreste, Stannanreste, akzeptorsubstituierte Vinylgruppen, Boroxine und Borazine, wobei R¹⁴, R¹⁵ und R¹⁶ substituiertes oder unsubstituiertes C₁-C₂₀-Alkyl, bevorzugt substituiertes oder unsubstituiertes C₁-C₆-Alkyl, oder substituiertes oder unsubstituiertes C₆-C₃₀-Aryl, bevorzugt substituiertes oder unsubstituiertes C₆-C₁₀-Aryl bedeuten; bevorzugt Halogen, bevorzugt F, mit Halogen substituiertem Alkyl, bevorzugt CF₃, CH₂F, CHF₂, C₂F₅, C₃F₃H₄, Pseudohalogen, bevorzugt CN, Carbonyloxy (-C = O (OR¹⁴)), bevorzugt -C = O (OCH₃), Phosphinoxid, bevorzugt P(O)Ph₂ und Sulfonyl, bevorzugt S(O)₂Ph₂; oder R¹⁹ bedeutet substituiertes oder unsubstituiertes elektronenarmes Heteroaryl mit fünf bis 30 Ringatomen, bevorzugt ausgewählt aus der Gruppe bestehend aus Pyridin, Pyrimidin und Triazin.

Besonders bevorzugt weisen die in den vorstehend genannten Schichten eingesetzten Verbindungen der Formel (I) mindestens einen Rest R¹, R², R³, R⁴, R⁵, R⁶ oder R⁷ auf, ausgewählt aus der Gruppe bestehend aus:

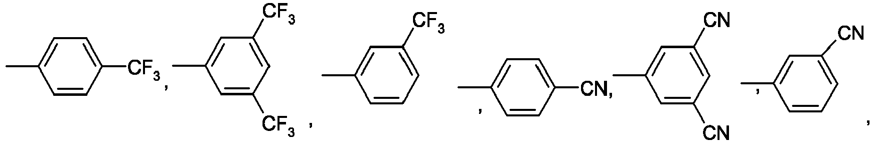

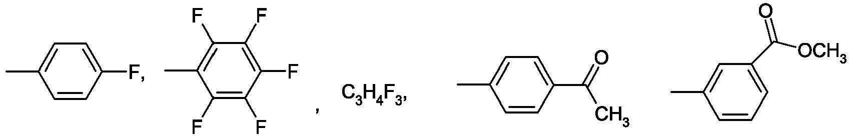

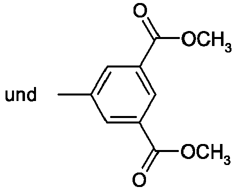

### Herstellung der erfindungsgemäßen Verbindungen der Formel (I)

Die Verbindungen der Formel (I) können grundsätzlich nach dem Fachmann bekann-ten Verfahren hergestellt werden, z. B. können Carbazole der Formel (I) (X = NR¹) thermisch oder photochemisch durch oxidativen Ringschluss aus Diphenylamin (oder geeignet substituierten Derivaten davon) und gegebenenfalls nachfolgende Substituti-on, z. B. am Stickstoff, hergestellt werden. Des Weiteren können die Carbazole der Formel (I) ausgehend von den geeignet substituierten Tetrahydrocarbazolen durch Oxidation erhalten werden. Eine typische Carbazol-Synthese ist die Borsche-DrechselCyclisierung (Borsche, Ann., 359, 49 (1908); Drechsel, J. prakt. Chem., [2], 38, 69, 1888). Die vorstehend erwähnten Tetrahydrocarbozole können gemäß dem Fachmann bekannten Verfahren hergestellt werden, z. B. durch Kondensation von ggf. geeignet substituiertem Phenylhydrazin mit ggf. geeignet substituiertem Cyclohexanon, wobei das entsprechende Imin erhalten wird. In einem anschließenden Schritt erfolgt eine Säure katalysierte Umlagerungs- und Ringschlussreaktion, wobei das entsprechende Tetrahydrocarbozol erhalten wird. Es ist ebenfalls möglich, die Herstellung des Imins und die Umlagerungs- und Ringschlussreaktion in einem Schritt durchzuführen. Dieses wird - wie vorstehend erwähnt - zu dem gewünschten Carbazol oxidiert.

Die Herstellung der Verbindungen der Formel (I) erfolgt bevorzugt ausgehend von dem ensprechenden Grundgerüst der Formel (II): 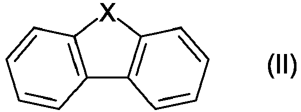 wobei X NR¹, SO, SO₂, S, O oder PR¹ oder NH oder PH oder PPh bedeutet. Geignete Grundgerüste der Formel (II) sind entweder kommerziell erhältlich (insbesondere in den Fällen, wenn X SO₂, S, O, NH oder PPh bedeutet) oder nach dem Fachmann bekannten Verfahren (X= PH oder SO) herstellbar.

Die Reste R¹ können in dem Fall, dass X NH oder PH bedeutet, vor oder nach dem Einbringen der Reste R^{a}, R^{b}, SiR²R³R⁴ und SiR⁵R⁶R⁷, soweit die Reste R^{a} und R^{b} in den Verbindungen der Formel (I) vorliegen, bzw. zur Einführung der Reste R^{a}, R^{b}, SiR²R³R⁴ und SiR⁵R⁶R⁷ geeigneten Vorläuferverbindungen, eingebracht werden. Somit sind drei Varianten - im Fall von X = NR¹ und PR¹ - möglich:

### Variante a)

ia) Herstellung einer zur Einführung der Reste R^{a}, R^{b}, SiR²R³R⁴ und SiR⁵R⁶R⁷ geeig-neten Vorläuferverbindung,
iia) Einbringen des Rests R¹,
iiia) Einbringen der Reste R^{a}, R^{b}, soweit vorhanden, sowie der Reste SiR²R³R⁴ und SiR⁵R⁶R⁷.

### Variante b)

Variante b) ist insbesondere dann bevorzugt, wenn R¹ substituiertes oder unsubstitu-iertes C₁-C₂₀-Alkyl oder C₆-C₃₀-Aryl oder C₁-C₂₀-alkylsubstituiertes C₆-C₃₀-Aryl ist.
ib) Einbringen des Rests R¹,
iib) Herstellung einer zur Einführung der Reste R^{a}, R^{b}, SiR²R³R⁴ und SiR⁵R⁶R⁷ geeig-neten Vorläuferverbindung,
iiib) Einbringen der Reste R^{a}, R^{b}, soweit vorhanden, sowie der Reste SiR²R³R⁴ und SiR⁵R⁶R⁷.

### Variante c)

ic) Herstellung einer zur Einführung der Reste R^{a}, R^{b}, SiR²R³R⁴ und SiR⁵R⁶R⁷ geeig-neten Vorläuferverbindung,
iic) Einbringen der Reste R^{a}, R^{b}, soweit vorhanden, sowie der Reste SiR²R³R⁴ und SiR⁵R⁶R⁷,
iiic) Einbringen des Rests R¹.

In dem Fall, dass X in Formel (I) NR¹, SO, SO₂, S, O oder PR¹ bedeutet, entfällt der Schritt "Einbringen des Rests R¹", so dass das Verfahren die folgenden Schritte (*Vari-ante d*) umfasst:
id) Herstellung einer zur Einführung der Reste R^{a}, R^{b}, SiR²R³R⁴ und SiR⁵R⁶R⁷ geeig-neten Vorläuferverbindung,
iid) Einbringen der Reste R^{a}, R^{b}, soweit vorhanden, sowie der Reste SiR²R³R⁴ und SiR⁵R⁶R⁷.

### Schritt ia), iib), ic) und id)

Als Vorläuferverbindungen zur Einführung der Reste R^{a}, R^{b}, SiR²R³R⁴ und SiR⁵R⁶R⁷ sind insbesondere die entsprechenden halogenierten, bevorzugt bromierten Verbin-dungen der allgemeinen Formel (III) geeignet: 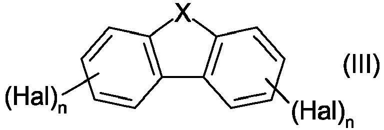 wobei Hal Halogen, bevorzugt Brom oder lod, besonders bevorzugt Brom, bedeutet, n jeweils 0, 1 oder 2 (wobei die Summe beider n in Formel (III) mindestens 1 ist), bevorzugt jeweils 1 ist und X in Schritt ia) und ic) X NH oder PH bedeutet, X in Schritt ib) NR¹ oder PR¹ bedeutet und X in Schritt id) NR¹, SO, SO₂, S, O oder PR¹ bedeutet.

Die Halogenierung kann gemäß dem Fachmann bekannten Verfahren durchgeführt werden. Bevorzugt erfolgt eine Bromierung oder lodierung in 3- und 6-Position des Grundgerüsts der Formel (II).

Besonders bevorzugt erfolgt die Bromierung mit Br₂ in Eisessig oder Chloroform bei tiefen Temperaturen, z.B. 0°C. Geeignete Verfahren sind z.B. für X= NPh in M. Park, J.R. Buck, C.J. Rizzo, Tetrahedron, 1998, 54, 12707-12714 und für X= S in W. Yang et al., J. Mater. Chem. 2003, 13, 1351 beschrieben. Des Weiteren sind einige bromierte Produkte der Formel (III) kommerziell erhältlich (FürX= NH und S).

### Schritt iia), ib) und iiic)

Die Einführung des Restes R¹ erfolgt nach dem Fachmann bekannten Verfahren.

Die Einführung des Restes erfolgt bevorzugt durch Umsetzung des Grundgerüsts der Formel (II) bzw. der Verbindung der Formel (III) mit einem Alkylhalogenid bzw. Arylhalogenid bzw. Heteroarylhalogenid der Formel R¹-Hal, wobei R¹ bereits vorstehend defi-niert wurde und Hal F, Cl, Br oder I, bevorzugt Br, I oder F, bedeutet.

Die Einführung des Restes R¹ wird im Allgemeinen in Anwesenheit einer Base durch-geführt. Geeignete Basen sind dem Fachmann bekannt und bevorzugt ausgewählt aus der Gruppe bestehend aus Alkali- und Erdalkalihydroxiden wie NaOH, KOH, Ca(OH)₂, Alkalihydriden wie NaH, KH, Alkaliamiden wie NaNH₂, Alkali- oder Erdalkalimetallcar-bonaten wie K₂CO₃ oder Cs₂CO₃,, und Alkalimetallalkoxiden wie NaOMe, NaOEt. Des Weiteren sind Gemische der vorstehend genannten Basen geeignet. Besonders bevor-zugt sind NaOH, KOH, K₂CO₃ oder NaH.

Die N-Alkylierung (zum Beispiel offenbart in M. Tosa et al., Heterocycl. Communicati-ons, Vol. 7, No. 3, 2001, S. 277 - 282) bzw. N-Arylierung oder N-Heteroarylierung (zum Beispiel (N-Arylierung) offenbart in H. Gilman und D. A. Shirley, J. Am. Chem. Soc. 66 (1944) 888; D. Li et al., Dyes and Pigments 49 (2001) 181 - 186) wird bevor-zugt in einem Lösungsmittel durchgeführt. Geeignete Lösungsmittel sind z.B. polare aprotische Lösungsmittel wie Dimethylsulfoxid, Dimethylformamid oder Alkohole. Es ist ebenfalls möglich, einen Überschuss des eingesetzten Alkyl- oder (Hetero)arylhaloge-nids als Lösungsmittel einzusetzen. Die Umsetzung kann des Weiteren in einem unpo-laren aprotischen Lösungsmittel, z. B. Toluol, durchgeführt werden, wenn ein Phasentransferkatalysator, z. B. tetra-n-Butylammoniumhydrogensulfat, anwesend ist (wie z. B. in I. Gozlan et al., J. Heterocycl. Chem. 21 (1984) 613 - 614 offenbart ist).

Die N-(Hetero)arylierung kann zum Beispiel durch Kupfer-katalysierte Kupplung der Verbindung der Formel (II) oder (III) mit einem (Hetero)arylhalogenid, z.B einem Ary-liodid erfolgen (Ullmann-Reaktion).

Bevorzugt erfolgt die Einführung des Restes R¹ durch Umsetzung der Verbindung der Formel (II) oder (III) mit einem Alkyl-, Aryl- oder Heteroarylfluorid in Anwesenheit von NaH in DMF (nucleophile Substitution) oder durch Umsetzung mit einem Alkyl-, Aryl- oder Heteroarylbromid oder-iodid unter Cu/Base (Ullmann, s.o) oder Pd Katalyse.

Das molare Verhältnis der Verbindung der Formel (II) oder (III) zu dem Alkylhalogenid bzw. (Hetero)arylhalogenid der Formel R¹-Hal beträgt im Allgemeinen 1 : 1 bis 1 : 15, bevorzugt 1 : 1 bis 1 : 6, besonders bevorzugt 1:4.

Die N-Alkylierung bzw. N-(Hetero)arylierung wird im Allgemeinen bei einer Temperatur von 0 bis 220 °C, bevorzugt 20 bis 200 °C durchgeführt. Die Reaktionsdauer beträgt im Allgemeinen 0,5 bis 48 h, bevorzugt 1 bis 24 h. Im Allgemeinen wird die N-Alkylierung bzw. N-Arylierung bei Normaldruck durchgeführt.

Das erhaltene Rohprodukt wird gemäß dem Fachmann bekannten Verfahren aufgear-beitet.

Bevorzugte Ausführungsformen des Schritts iia), ib) und iiic) sind nachstehend in allgemeiner Form am Beispiel von R¹ = substituiertes Phenyl (R = vorstehend genannter Substituent am Arylrest; q = 0, 1, 2, 3, 4 oder 5) dargestellt: 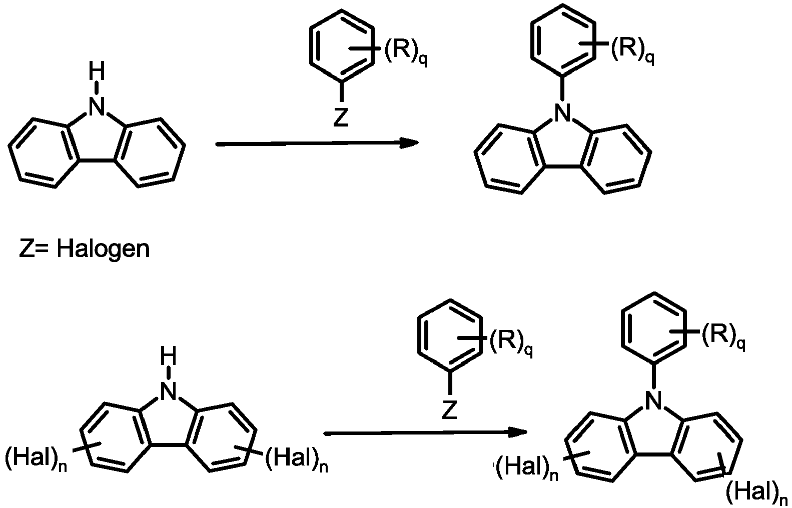
Z = Halogen
n=0, 1, 2

### Schritt iiia), iiib), iic) und iid)

Die Herstellung der gewünschten silylierten Verbindungen der Formel (I) erfolgt ausgehend von den halogenierten Vorläuferverbindungen der Formel (III) im Allgemeinen durch Halogen/Metallaustausch und anschließende Silylierung nach dem Fachmann bekannten Verfahren.

Bevorzugt erfolgt die Herstellung in einem ersten Schritt durch Halogen/Metallaus-tausch durch Umsetzung der halogenierten Verbindungen der Formel (III) mit Alkyllithiumverbindungen oder Mg bei Temperaturen von Allgemeinen -80°C bis + 80 °C, be-vorzugt bei -78°C bis 0°C (für Alkyllithiumverbinguen) oder 0°C bis 80°C (für Mg), be-sonders bevorzugt von 0°C bis 40°C (für Mg). Besonders bevorzugt werden Alkyllithiumverbindungen, insbesondere n-BuLi oder tert.-BuLi eingesetzt. Die Umsetzung er-folgt im Allgemeinen in einem Lösungsmittel, bevorzugt in THF (oder Ether, bevorzugt Diethylether. In einem direkt anschließenden zweiten Schritt erfolgt eine Silylierung zu den gewünschten Verbindungen der Formel (I), bevorzugt durch Umsetzung mit SiRₘ₋Cl₍₄₋ₘ₎ oder SiRₘ(OR')₍₄₋ₘ₎, wobei m 1, 2 oder 3 und R' C₁- bis C₆-Alkyl bedeutet. Die Silylierung wird im Allgemeinen in einem Lösungsmittel durchgeführt. Bevorzugte Lö-sungsmittel sind THF oder Ether, bevorzugt Diethylether. Im Allgemeinen erfolgt die Silylierung in direktem Anschluss an die Umsetzung im ersten Schritt, ohne Aufarbei-tung oder Isolierung des nach dem ersten Schritt erhaltenen Produktes Der Halo-gen/Metallaustausch und die anschließende Silylierung werden im Allgemeinen so oft wiederholt, bis alle n Halogenreste in der Verbindung der Formel (IV) durch Silylgruppen ersetzt sind. Tsai et al., Adv. Mater., 2006, Vol. 18, Nr. 9, Seiten 1216 bis 1220.

In dem Fall, wenn der Halogen/Metall-Austausch und die anschließende Silylierung an einer Verbindung der Formel (III) durchgeführt werden, worin X = NH oder PH bedeutet (Variante c), Schritt iic)), ist es erforderlich, die NH oder PH Gruppe durch eine Schutzgruppe zu schützen und im Anschluss an die Silylierung wieder zu entschützen.

Die Einbringung der Schutzgruppe erfolgt gemäß dem Fachmann bekannten Verfah-ren. Im Allgemeinen erfolgt zunächst eine Deprotonierung und anschließend die Ein-führung einer Schutzgruppe. Geeignete N-H und P-H Schutzgruppen sind dem Fach-mann bekannt, wobei Silyl-Schutzgruppen, insbesondere SiR₃ mit R = Alkyl oder Aryl, bevorzugt Methyl, Ethyl, Phenyl, für dieses Verfahren besonders geeignet sind. Die Deprotonierung erfolgt üblicherweise mit Basen, z. B. mit NaH, nBuLi oder tert.-BuLi.

Das Entschützen erfolgt ebenfalls gemäß dem Fachmann bekannten Verfahren. Ge-eignete Reagezien zum Entschützen richten sich nach den eingesetzten Schutzgrup-pen. Bei Einsatz von SiR₃ als Schutzgruppe erfolgt die Entschützung im Allgemeinen mit einer Säure oder TBAF (Tetrabutylammoniumfluorid).

Bevorzugte Ausführungsformen des Schritts iiia), iiib) und iid) sind nachstehend in allgemeiner Form dargestellt (X = NR¹, SO, SO₂, S, O oder PR¹): 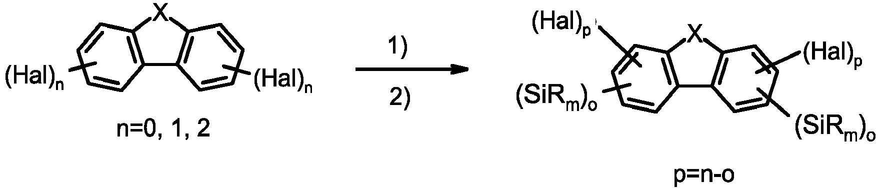
1) Halogen/Metall-Austausch
2) Silylierung, SiRₘ(Cl)₄₋ₘ oder SiRₘ(OR')₄₋ₘ

Wenn p= 1 oder 2, können Schritt 1 und 2 nochmals wiederholt werden, bis p=0
In Schritt iic) (X = NH oder PH) erfolgt im Allgemeinen vor dem Halogen/Metallaus-tausch mit anschließender Silylierung die Einführung einer Schutzgruppe. Eine bevor-zugte Ausführungsform des Schritts iic) ist nachstehend am Beispiel für X = NH darge-stellt:

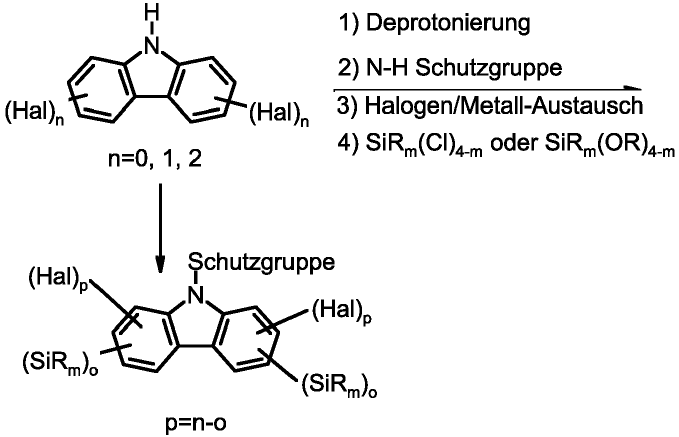

Wenn p= 1 oder 2, können Schritt 3 und 4 nochmals wiederholt werden, bis p=0

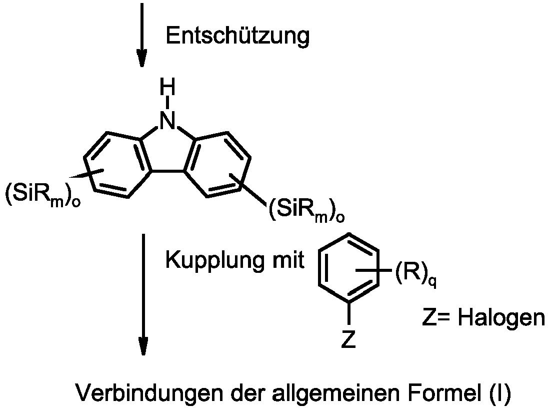

Durch Einsatz der erfindungsgemäßen Verbindungen der Formel (I) als Matrixmateriali-en in der Licht-emittierenden Schicht und/oder in mindestens einer weiteren Schicht der OLEDs ausgewählt aus Disilylcarbazolen, Disilyldibenzofuranen, Disilyldibenzothio-phenen, Disilyldibenzophospholen, Disilyldibenzothiophen-S-oxiden und Disilyldibenzo-thiophen-S,S-dioxiden können OLEDs mit hoher Effizienz und Lebensdauer erhalten werden. Die Effizienz der OLEDs kann des Weiteren durch Optimierung der anderen Schichten verbessert werden. Beispielsweise können hoch effiziente Kathoden wie Ca oder Ba, gegebenenfalls in Kombination mit einer Zwischenschicht aus LiF, eingesetzt werden. Geformte Substrate und neue Löcher-transpor-tierende Materialien, die eine Reduktion der Operationsspannung oder eine Erhöhung der Quanteneffizienz bewir-ken, sind ebenfalls in den OLEDs einsetzbar. Des Weiteren können zusätzliche Schichten in den OLEDs vorhanden sein, um die Energielevel der verschiedenen Schichten einzustellen und um Elektrolumineszenz zu erleichtern.

Die OLEDs können in allen Vorrichtungen eingesetzt werden, worin Elektrolumines-zenz nützlich ist. Geeignete Vorrichtungen sind bevorzugt ausgewählt aus stationären und mobilen Bildschirmen und Beleuchtungseinheiten. Stationäre Bildschirme sind z.B. Bildschirme von Computern, Fernsehern, Bildschirme in Druckern, Küchengeräten sowie Reklametafeln, Beleuchtungen und Hinweistafeln. Mobile Bildschirme sind z.B. Bildschirme in Handys, Laptops, Digitalkameras, Fahrzeugen sowie Zielanzeigen an Bussen und Bahnen.

Weiterhin können die Verbindungen der Formel (I) in OLEDs mit inverser Struktur ein-gesetzt werden. Bevorzugt werden die erfindungsgemäßen Verbindungen der Formel (I) in diesen inversen OLEDs wiederum als Matrixmaterialien in der Licht-emittierenden Schicht und/oder in mindestens einer weiteren Schicht der OLED eingesetzt. Der Auf-bau von inversen OLEDs und die üblicherweise darin eingesetzten Materialien sind dem Fachmann bekannt.

Ein Aspekt betrifft eine Licht-emittierende Schicht enthaltend mindestens eine Verbindung der Formel (I) gemäß der vorliegenden Erfindung und mindestens Emittermateri-al. Bevorzugte Verbindungen der Formel (I) und Emittermaterialien sind vorstehend genannt.

Im Allgemeinen beträgt der Anteil der mindestens einen Verbindung der Formel (I) in der Licht-emittierenden Schicht der OLED 10 bis 99 Gew.-%, bevorzugt 50 bis 99 Gew.-%, besonders bevorzugt 70 bis 97 Gew.-%. Der Anteil des mindestens einen Emitter-Materials in der Licht-emittierenden Schicht beträgt im Allgemeinen 1 bis 90 Gew.-%, bevorzugt 1 bis 50 Gew.-%, besonders bevorzugt 3 bis 30 Gew.-%, wobei die Anteile an der mindestens einen Verbindung der Formel (I) und dem mindestens einen Emittermaterial 100 Gew.-% ergeben. Es ist jedoch auch möglich, dass die Licht-emit-tierende Schicht neben der mindestens einen Verbindung der allgemeinen Formel (I) und dem mindestens einen Emittermaterial weitere Substanzen enthält, z. B. weiteres Verdünnungsmaterial, weiteres von den Verbindungen der Formel (I) verschiedenes Matrixmaterial, wobei z. B. weiteres Verdünnungsmaterial vorstehend genannt wurde.

Ein weiterer Gegenstand ist die Verwendung von Verbindungen der allgemeinen For-mel I gemäß der vorliegenden Anmeldung als Matrixmaterial, Loch-/Excitonenblocker-material und/oder Elektronen-/Excitonenblockermaterial und/oder Loch-Injektionsmate-rial und/oder Elektronen-Injektionsmaterial und/oder Lochleitermaterial und/oder Elek-tronenleitermaterial in einer organischen Leuchtdiode. Bevorzugt werden die Verbin-dungen der Formel (I) als Matrixmaterialien und/oder Loch/Excitonenblockermaterialien in der OLED verwendet.

Bevorzugte Verbindungen der Formel (I), bevorzugte Emittermaterialien sowie bevor-zugte Ausführungsformen betreffend den Einsatz von Verbindungen der Formel (I), die bestimmte Substituenten aufweisen, in speziellen Schichten der OLEDs sind vorste-hend genannt.

Des Weiteren betrifft die Offenbarung eine Vorrichtung ausgewählt aus der Gruppe bestehend aus stationären Bildschirmen wie Bildschirmen von Computern, Fernsehern, Bildschirmen in Druckern, Küchengeräten sowie Reklametafeln, Beleuchtungen, Hin-weistafeln und mobilen Bildschirmen wie Bildschirmen in Handys, Laptops, Digitalka-meras, Fahrzeugen sowie Zielanzeigen an Bussen und Bahnen und Beleuchtungsein-heiten enthaltend mindestens eine offenbarte organische Leuchtdiode oder mindestens eine offenbarte Licht-emittierende Schicht.

Die nachfolgenden Beispiele erläutern die Erfindung zusätzlich.

### Beispiele

### A Herstellungsbeispiele

### Beispiel 1

### Bromierung von N-substituierten Carbazolen

### Beispiel 1a: Synthese von 9-(3,5-Bis(trifluoromethyl)phenyl)-3,6-dibromo-carbazol

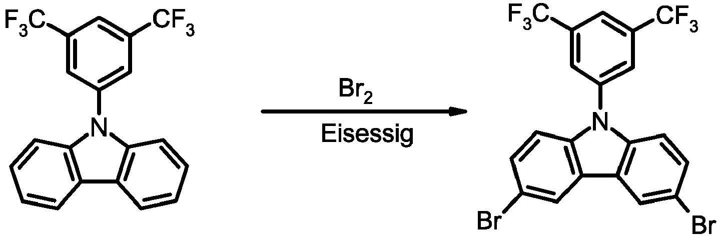

Eine Lösung von 9-(3,5-Bis(trifluoromethyl)phenyl)-9H-carbazol (2.7 g, 1 eq) in Eisessig (210 mL) wird langsam mit einer Lösung von Brom (2.3 g, 2.0 eq) in Eisessig (7 mL) versetzt. Nach 1h rühren, wird die Mischung mit Eiswasser (1000 mL) versetzt und 1 h gerührt. Der Niederschlag wird abfiltriert und mit Wasser gewaschen. Der Rückstand wird in Essigester umkristallisiert. Ausbeute 55%. ¹H-NMR (THF-d8, 400 MHz): *δ* = 7.3 (d, 2H), 7.6 (d, 2H), 8.2 (s, 1H), 8.3 (s, 2H), 8.4 (s, 2H).

### Beispiel 1b: Synthese von 9-(3-Cyanophenyl)-3,6-dibromo-carbazol

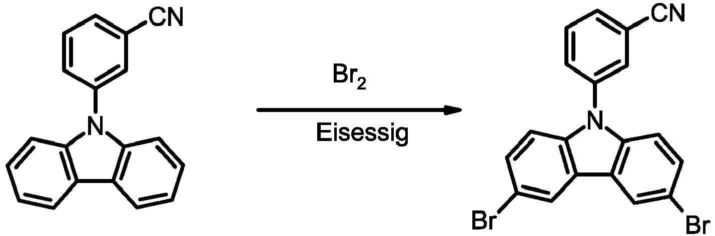

Eine Lösung von 9-(3-Cyanophenyl)-9H-carbazol (3.5 g, 1 eq) in Eisessig (150mL) wird langsam mit einer Lösung von Brom (4.1 g, 2.0 eq) in Eisessig (5 mL) versetzt. Nach 3h rühren, wird die Mischung mit Eiswasser (500 mL) versetzt und 1 h gerührt. Der Niederschlag wird abfiltriert und mit Wasser gewaschen. Der Rückstand wird in Ethanol umkristallisiert. Ausbeute 69%. ¹H-NMR (CDCl₃, 400 MHz): *δ*= 7.2 (d, 2H), 7.5 (d, 2H), 7.75 (m, 3H), 7.8 (s, 1H), 8.2 (s, 2H).

### Beispiel 2

### N-Arylierung von Carbazol (X in der allgemeinen Formel II bedeutet N-H)

Allgemeine Arbeitsvorschrift:

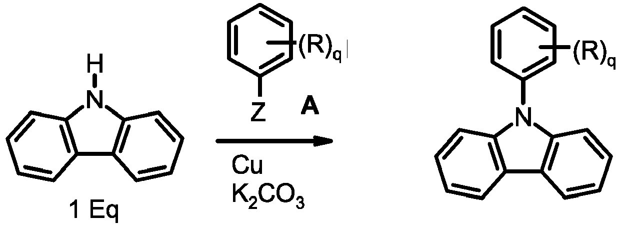

Carbazol (1 Eq), Phenylhalogenid **A,** Kaliumcarbonat und Kupferpulver werden auf 160-200 °C erhitzt und über Nacht bei dieser Temperatur gerührt. Nach dem Abkühlen auf Raumtemperatur wird mit Aceton oder Methylenchlorid extrahiert. Der Niederschlag wird abfiltriert und säulenchromatographisch (Kieselgel, Methylenchlorid/Cyclohexan) aufgereinigt.

In der nachfolgenden Tabelle sind die Daten verschiedener N-Arylierungen von Carbazol, die entsprechend der allgemeinen Vorschrift durchgeführt wurden, zusammengefasst:

| | | | | | | |
|---|---|---|---|---|---|---|
| 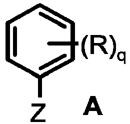 | Equiv. **A** | Equiv. K₂CO₃ | Mol% Cu | T (°C) | Ausbeute | Analytik |
| 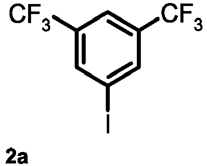 | 1.1 | 1.5 | 12 | 165 | 41 | ¹H-NMR (CDCl₃, 400 MHz): *δ*= 7.35 (m, 4H), 7.4 (m, 2H), 7.9 (s, 1H), 8.1 (s, 2H), 8.2 (d, 2H). MALDI-MS: m/z= 379 |
| 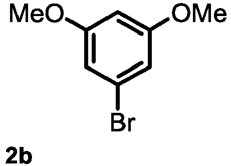 | 1.1 | 1.2 | 12.5 | 180 | 76 | ¹H-NMR (CDCl₃, 400 MHz): *δ*= 3.8 (s, 6H), 6.6 (s, 1H), 6.7 (s, 2H), 7.3 (dd, 2H), 7.4 (dd, 2H), 7.5 (d, 2H), 8.1 (d, 2H); MALDI-MS: m/z= 303 |
| 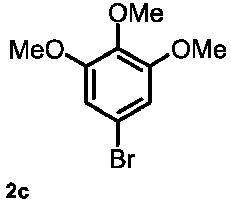 | 1.1 | 1.2 | 12.5 | 180-200 | 73 | ¹H-NMR (CDCl₃, 400 MHz): *δ*= 3.8 (s, 6H), 3.9 (s, 3H), 6.8 (s, 2H), 7.3 (m, 2H), 7.4 (quasi d, 4H), 8.2 (d, 2H). |
| 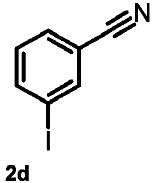 | 1.1 | 1.2 | 12.5 | 175 | 73 | ¹H-NMR (CDCl₃, 400 MHz): *δ*= 7.3 (m, 4H), 7.45 (dd, 2H), 7.7 (dd, 2H), 7.8 (dd, 1H), 7.9 (s, 1H), 8.2 (d, 2H). |

### Beispiel 3

### N-Arylierung von bromierten Carbazolderivaten (X in der allgemeinen Formel II bedeu-tet N-H)

Allgemeine Arbeitsvorschrift:

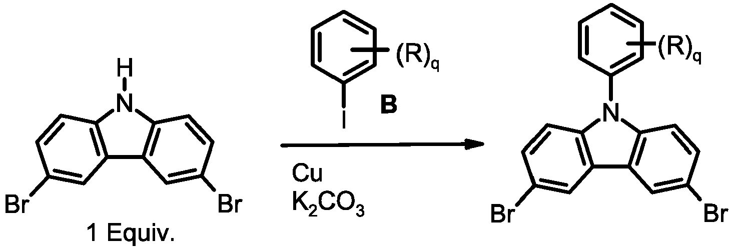

Carbazol (1 Eq), Phenyliodid **B,** Kaliumcarbonat und Kupferpulver werden auf 130-160 °C erhitzt und 48 h bei dieser Temperatur gerührt. Nach dem Abkühlen auf Raumtem-peratur wird mit Methylenchlorid extrahiert. Umkristallisation aus EtOH gibt das ge-wünschte Produkt.

In der nachfolgenden Tabelle sind die Daten verschiedener N-Arylierungen von bro-mierten Carbazolderivaten, die entsprechend der allgemeinen Vorschrift durchgeführt wurden, zusammengefasst:

| | | | | | | |
|---|---|---|---|---|---|---|
| 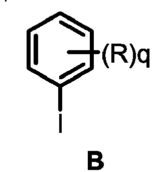 | Equiv. **B** | Equiv. K₂CO₃ | Mol% Cu | T (°C) | Ausbeute | Analytik |
| 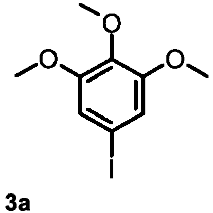 | 4 | 2.5 | 21 | 130 | 70 | ¹H-NMR (CDCl₃, 400 MHz): *δ*= 3.9 (s, 6H), 4.0 (s, 3H), 6.6 (s, 2H), 7.2 (d, 2H), 7.5 (d, 2H), 8.2 (s, 2H). |
| 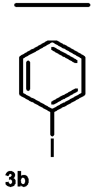 | 2.9 | 2.6 | 18 | 125 | 68 | ¹H-NMR (CD₂Cl₂, 400 MHz): *δ*= 1.4 (s, 9H), 7.25 (d, 2H), 7.37 (d, 2H), 7.47 (d, 2H), 7.6 (d, 2H), 8.2 (s, |
| | | | | | | 2H). |
| 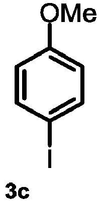 | 2 | 1.25 | 11 | 130 | 60 | ¹H-NMR (CDCl₃, 400 MHz): *δ*= 3.9 (s, 3H), 7.1 (d, 2H), 7.2 (d, 2H), 7.4 (d, 2H), 7.5 (d, 2H), 8.2 (s, 2H). |

### Beispiel 4

### Kupplung von bromierten N-arylierten Carbazolderivaten und bromierten Dibenzothio-phenderivaten mit Silylverbindungen

### Beispiel 4a: Synthese von 2,8-Bis(triphenylsilyl)dibenzothiophen

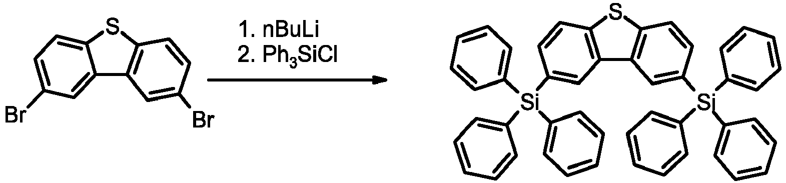

Eine Lösung von 2,8-Dibromdibenzothiophen (3.0 g, 1 eq) in trockenem THF (165 mL) wird bei -78 °C unter Argon langsam mit *n*-Butyllithium (1.6 M in Hexan, 13.7 mL, 2.5 eq) versetzt und 1 h bei -78 °C gerührt. Nach Zugabe einer Lösung von Chlortriphenylsilan (6.5 g, 2.5 eq) in trockenem THF (14 mL) bei -78 °C wird die Mischung un-ter Rühren über Nacht auf Raumtemperatur erwärmt. Überschüssiges Butyllithium wird mit gesättigter Ammoniumchlorid-Lösung hydrolysiert. Das ausgefallene Produkt wird abfiltriert und gründlich mit THF, Wasser und Methyl-*tert*-butylether gewaschen. Die vereinigten THF-Filtrate werden zur Trockne eingeengt und mit wenig Essigester ver-rührt. Der Niederschlag wird abfiltriert und säulenchromatographisch (Kieselgel, Essi-gester/Cyclohexan) aufgereinigt. Ausbeute: 46%. ¹H-NMR (CD₂Cl₂, 400 MHz): *δ* = 7.35-7.41 (m, 12H), 7.44-7.49 (m, 6H), 7.55-7.59 (m, 12H), 7.65 (d, *J*=8.0, 2H), 7.92 (d, *J*=8.0, 2H), 8.16 (s, 2H).

### Beispiel 4b: Synthese von 9-Phenyl-3,6-bis(triphenylsilyl)-carbazol

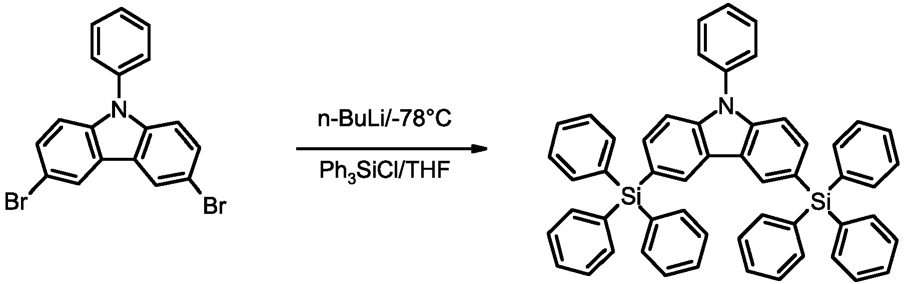

Eine Lösung von 9-Phenyl-3,6-dibrom-9H-carbazol (6.0 g, 1 eq) in trockenem THF (500 mL) wird bei -78 °C unter Argon langsam mit *n*-Butyllithium (1.6 M in Hexan, 28.2 mL, 3.0 eq) versetzt und 1 h bei -78 °C gerührt. Nach Zugabe einer Lösung von Chlortriphenylsilan (13.5 g, 3.0 eq) in trockenem THF (100 mL) bei -78 °C wird die Mi-schung unter Rühren über Nacht auf Raumtemperatur erwärmt. Überschüssiges Butyllithium wird mit gesättigter Ammoniumchlorid-Lösung hydrolysiert. Das ausgefallene Produkt wird abfiltriert und gründlich mit CH₂Cl₂ gewaschen. Das Produkt fällt aus den vereinigten CH₂Cl₂-Filtraten aus. Ausbeute: 40%. Wenn das Produkt nicht ausfällt, wer-den die vereinigten CH₂Cl₂-Filtrate werden zur Trockne eingeengt. Umkristallisation mit Essigester gibt das Produkt. ¹H-NMR (CDCl₃, 400 MHz): *δ* = 7.35 (m, 12H), 7.45 (m, 9H), 7.52-7.6 (m, 18H), 8.2 (s, 2H).

### Beispiel 4c: Synthese von 9-(3,4,5-Trimethoxyphenyl)-3,6-bis(triphenylsilyl)- carbazol

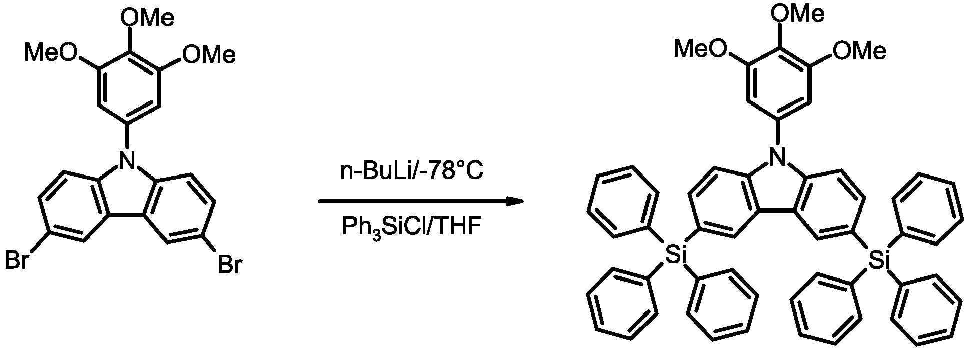

Eine Lösung von 9-(3,4,5-Trimethoxyphenyl)-3,6-dibrom-9H-carbazol (1.5 g, 1 eq) in trockenem THF (100 mL) wird bei -78 °C unter Argon langsam mit *n*-Butyllithium (1.6 M in Hexan, 5.6 mL, 3.0 eq) versetzt und 1 h bei -78 °C gerührt. Nach Zugabe einer Lösung von Chlortriphenylsilan (2.7 g, 3.0 eq) in trockenem THF (40 mL) bei -78 °C wird die Mischung unter Rühren über Nacht auf Raumtemperatur erwärmt. Überschüssiges Butyllithium wird mit gesättigter Ammoniumchlorid-Lösung hydrolysiert. Das ausgefalle-ne Produkt wird abfiltriert und gründlich mit Methylenchlorid gewaschen. Die vereinig-ten Methylenchlorid-Filtrate werden zur Trockne eingeengt. Der Feststoff wird ausge-rührt in Methanol und abfiltriert. Ausbeute: 63%. ¹H-NMR (CDCl₃, 400 MHz): *δ*= 3.8 (s, 6H), 3.9 (s, 3H), 6.75 (s, 2H), 7.3 (m, 12H), 7.4 (m, 8H), 7.6 (m, 14H), 8.2 (s, 2H).

### Beispiel 4d: Synthese von 3,6-Bis-[(3,5-bis(trifluoromethyl)phenyl)-dimethyl-silyl]-9-phenyl-carbazol

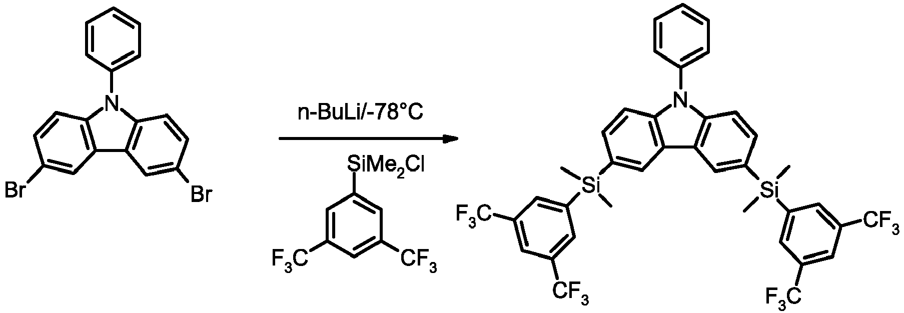

Eine Lösung von 9-Phenyl-3,6-dibrom-9H-carbazol (1.3 g, 1 eq) in trockenem THF (50 mL) wird bei -78 °C unter Argon langsam mit *n*-Butyllithium (1.6 M in Hexan, 5.1 mL, 2.5 eq) versetzt und 1 h bei -78 °C gerührt. Nach Zugabe einer Lösung von 3,5-Bis(trifluormethyl)phenyldimethylchlorsilan (2.5 g, 2.5 eq) in trockenem THF (20 mL) bei -78 °C wird die Mischung unter Rühren über Nacht auf Raumtemperatur erwärmt. Überschüssiges Butyllithium wird mit gesättigter Ammoniumchlorid-Lösung hydrolysiert. Das ausgefallene Produkt wird abfiltriert und gründlich mit CH₂Cl₂ gewa-schen. Die vereinigten Methylenchlorid-Filtrate werden mit Wasser extrahiert und zur Trockne eingeengt. Der Rückstand wird säulenchromatographisch (Kieselgel, Methy-lenchlorid/Cyclohexan) aufgereinigt. Ausbeute 60%. ¹H-NMR (CDCl₃, 400 MHz): *δ* = 0.7 (s, 12H), 7.4-7.7 (m, 9H), 7.8 (s, 2H), 8.0 (s, 4H), 8.3 (s, 2H).

### Beispiel 4e: Synthese von 3,6-Bis-[(4-methoxyphenyl)-dimethyl-silyl]-9-phenyl-carbazol

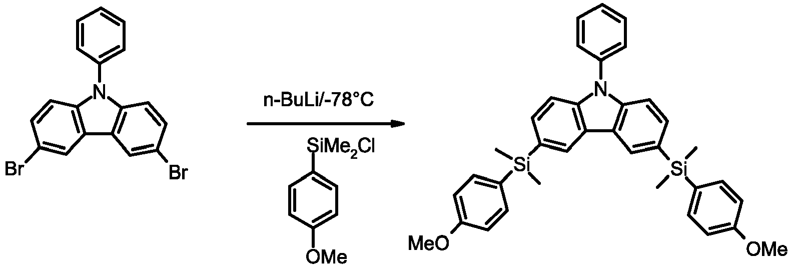

Eine Lösung von 9-Phenyl-3,6-dibrom-9H-carbazol (1.6 g, 1 eq) in trockenem THF (20 mL) wird bei -78 °C unter Argon langsam mit *n*-Butyllithium (1.6 M in Hexan, 6.1 mL, 2.5 eq) versetzt und 1 h bei -78 °C gerührt. Nach Zugabe einer Lösung von 4-Methoxyphenyl-dimethylchlorsilan (2.4 g, 3.1 eq) in trockenem THF (10 mL) bei -78 °C wird die Mischung unter Rühren über Nacht auf Raumtemperatur erwärmt. Überschüs-siges Butyllithium wird mit gesättigter Ammoniumchlorid-Lösung hydrolysiert. Das aus-gefallene Produkt wird abfiltriert und gründlich mit CH₂Cl₂ gewaschen. Die vereinigten Methylenchlorid-Filtrate werden mit Wasser extrahiert und zur Trockne eingeengt. Der Rückstand wird säulenchromatographisch (Kieselgel, Hexan/Essigester) aufgereinigt. Ausbeute 40%. MALDI-MS: m/z= 571. HPLC: 99% Reinheit.

### Beispiel 4f: Synthese von 9-(3-Methoxyphenyl)-3,6-bis(triphenylsilyl)-carbazol

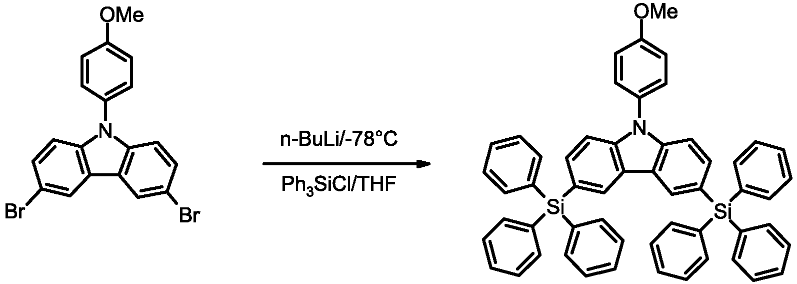

Eine Lösung von 9-(3-Methoxyphenyl)-3,6-dibrom-9H-carbazol (2.5 g, 1 eq) in trockenem THF (100 mL) wird bei -78 °C unter Argon langsam mit *n*-Butyllithium (1.6 M in Hexan, 5.6 mL, 2.5 eq) versetzt und 1 h bei -78 °C gerührt. Nach Zugabe einer Lösung von Chlortriphenylsilan (4.3 g, 2.5 eq) in trockenem THF (40 mL) bei -78 °C wird die Mischung unter Rühren über Nacht auf Raumtemperatur erwärmt. Überschüssiges Butyllithium wird mit gesättigter Ammoniumchlorid-Lösung hydrolysiert. Das ausgefallene Produkt wird abfiltriert und gründlich mit Methylenchlorid gewaschen. Die vereinigten Methylenchlorid-Filtrate werden mit Wasser extrahiert und zur Trockne eingeengt. Der Rückstand wird säulenchromatographisch (Kieselgel, Cyclohexan/Methylenchlorid) aufgereinigt. Ausbeute 41%. ¹H-NMR (CDCl₃, 400 MHz): *δ*= 3.9 (s, 3H), 7.05 (d, 2H), 7.15 (m, 14H), 7.20 (m, 8H), 7.55 (d, 2H), 7.60 (d, 12H), 8.2 (s, 2H).

### Beispiel 4g: Synthese von 2,8-bis(triphenylsilyl)-dibenzofuran

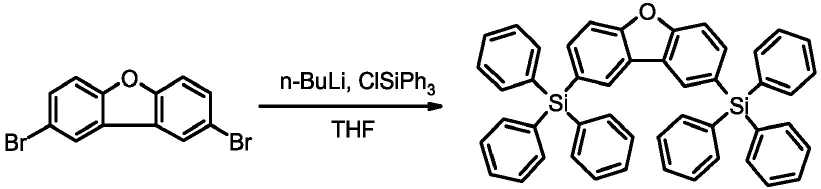

### Reaktionsvorschrift :

6.02 g (18.47 mmol) 2,8-Dibromdibenzofuran werden in 120 ml THF suspendiert und bei -78°C vorsichtig mit 22.9 ml (36.64 mmol) n-BuLi (1,6M in Hexan) versetzt. Im Anschluss wird 3 h bei - 78°C gerührt. Das Reaktionsgemisch wird mit einer Lösung von 10.91 g (37.00 mmol) Chlortriphenylsilan in 120 ml THF versetzt, auf Raumtemperatur aufwärmen lassen und 16 h bei Raumtemperatur gerührt. Es wird vorsichtig mit 10 ml Methanol gequencht und anschließend bis zur Trockene eingeengt. Der Rückstand wird erst in Methanol, dann in Wasser und anschließend wieder in Methanol digeriert, abfiltriert und getrocknet. Das Rohprodukt wird in Methylenchlorid gelöst, über Kieselgel filtriert und durch Überschichten mit Cyclohexan auskristallisiert. Das Kristallisat wird abfiltriert und getrocknet. Man erhält 9.28 g (73 %) weißes Pulver.
**¹H-NMR:** (CD₂Cl₂, 500 MHz):
δ = 7.35-7.38 (m, 12 H, CH_{Ar}), 7.41-7.44 (m, 6 H, CH_{Ar}), 7.56-7.57 (m, 12 H, CH_{Ar}), 7.58-7.63 (m, 4 H, CH_{Ar}), 8.09 (s, 2 H, CH_{Ar}).
**¹³C-NMR** (CD₂Cl₂, 125 MHz):
δ = 111.5 (2C, CH_{Ar}), 124.0 (2C, C_{quart}), 128.1 (12C, CH_{Ar}), 128.3 (2C, C_{quart}), 129.2 (2C, CH_{Ar}), 129.8 (6C, CH_{Ar}), 134.4 (6C, C_{quart}), 135.6 (2C, CH_{Ar}), 136.5 (12C, CH_{Ar}), 157.5 (2C, C_{quart}).
*Masse (EI):* ***m*/*e* = 684 *(M*⁺*)***

### Beispiel 4h: Synthese von 3-Bromo-9-phenyl-6-triphenylsilyl-carbazol

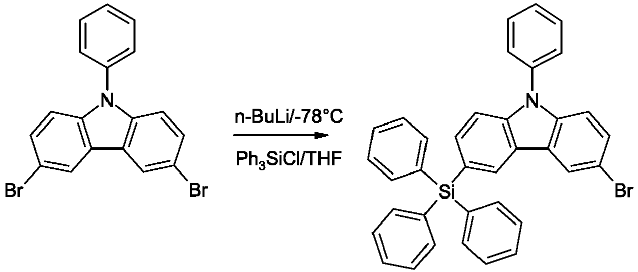

### Reaktionsvorschrift :

Eine Lösung von 9-(3-Methoxyphenyl)-3,6-dibrom-9H-carbazol (26 g, 1 eq) in trockenem THF (700 mL) wird bei -78 °C unter Argon langsam mit *n*-Butyllithium (1.6 M in Hexan, 41 mL, 1 eq) versetzt und 2 h bei -78 °C gerührt. Nach Zugabe einer Lösung von Chlortriphenylsilan (30 g, 1.5 eq) in trockenem THF (150 mL) bei -78 °C wird die Mischung unter Rühren über Nacht auf Raumtemperatur erwärmt. Überschüssiges Butyllithium wird mit gesättigter Ammoniumchlorid-Lösung hydrolysiert. Das ausgefallene Produkt wird abfiltriert und gründlich mit Methylenchlorid gewaschen. Die vereinigten Methylenchlorid-Filtrate werden mit Wasser extrahiert und zur Trockne eingeengt. Der Rückstand mit Aceton ausgerührt und abfiltriert. Ausbeute 74%.
**¹H-NMR** (CDCl₃, 400 MHz):
*δ*= 8.15 (s, 1H), 8.28 (s, 1H), 7.3-7.7 (m, 24H).

### Beispiel 4i: Synthese von 3,6-Bis-(methyl-diphenyl-silyl)-9-phenyl-carbazol

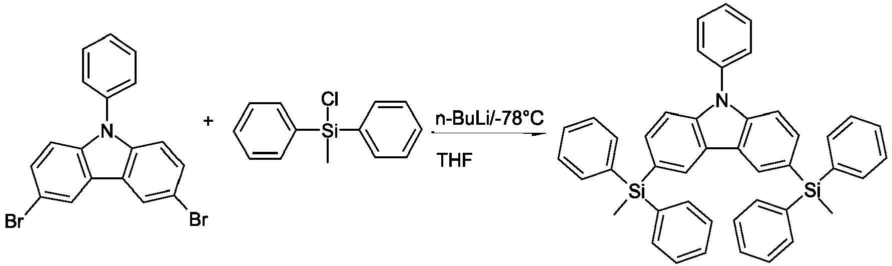

### Reaktionsvorschrift:

Eine Lösung von 9-Phenyl-3,6-dibrom-9H-carbazol (3.1 g, 1 eq) in trockenem THF (150 mL) wird bei -78 °C unter Argon langsam mit *n*-Butyllithium (1.6 M in Hexan, 12.2 mL, 2.5 eq) versetzt und 1 h bei -78 °C gerührt. Nach Zugabe einer Lösung von Diphenylmethylchlorsilan (5.6 g, 3.0 eq) in trockenem THF (10 mL) bei -78 °C wird die Mischung unter Rühren über Nacht auf Raumtemperatur erwärmt. Überschüssiges Butyllithium wird mit gesättigter Ammoniumchlorid-Lösung hydrolysiert. Das ausgefallene Produkt wird abfiltriert und gründlich mit CH₂Cl₂ gewaschen. Säulenchromatographie (SiO₂, Cyclohexan/CH₂Cl₂ 15:1) gibt das Produkt. Ausbeute 60%.
**¹H-NMR** (CDCl₃, 400 MHz):
*δ*= 0.9 (s, 6H), 7.3-7.4 (m, 14H), 7.4-7.5 (m, 3H), 7.5-7.6 (m, 12H), 8.25 (s, 2H).

### Beispiel 4j: Synthese von 3,6-Bis-(dimethyl-pentafluorophenyl-silyl)-9-phenyl-carbazol

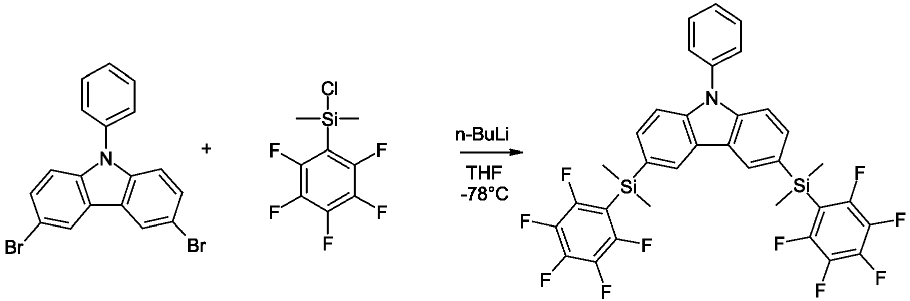

### Reaktionsvorschrift:

Eine Lösung von 9-Phenyl-3,6-dibrom-9H-carbazol (3.1 g, 1 eq) in trockenem THF (150 mL) wird bei -78 °C unter Argon langsam mit *n*-Butyllithium (1.6 M in Hexan, 12.2 mL, 2.5 eq) versetzt und 1 h bei -78 °C gerührt. Nach Zugabe einer Lösung von Flophemesylchlorid (6.1 g, 3.0 eq) in trockenem THF (10 mL) bei -78 °C wird die Mischung unter Rühren über Nacht auf Raumtemperatur erwärmt. Überschüssiges Butyllithium wird mit gesättigter Ammoniumchlorid-Lösung hydrolysiert. Das ausgefallene Produkt wird abfiltriert und gründlich mit CH₂Cl₂ gewaschen. Säulenchromatographie (C18-SiO₂, MeCN) gibt das Produkt. Ausbeute 65%.
**¹H-NMR** (CDCl₃, 400 MHz):
*δ*= 0.8 (s, 12H), 7.38 (d, 2H), 7.5 (m, 3H), 7.6 (m, 4H), 8.35 (s, 2H).

### Beispiel 4k: Synthese von Bis(9-Phenyl-3-triphenylsilyl-carbazolyl)dimethylsilan

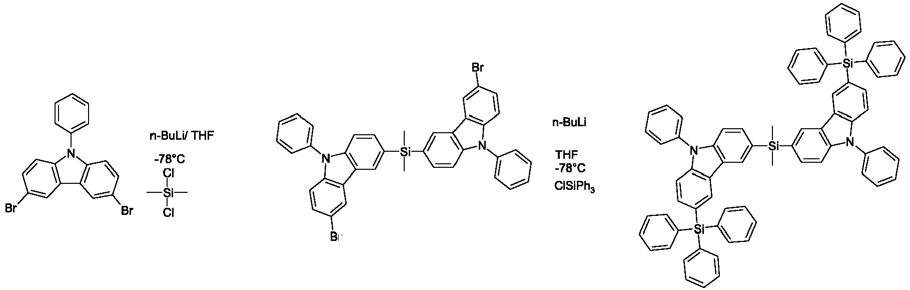

### Reaktionsvorschrift:

### Schritt 1:

Eine Lösung von 9-Phenyl-3,6-dibrom-9H-carbazol (12 g, 1 eq) in trockenem THF (230 mL) wird bei -78 °C unter Argon langsam mit *n*-Butyllithium (1.6 M in Hexan, 18.8 mL, 1 eq) versetzt und 1,5 h bei -78 °C gerührt. Nach Zugabe einer Lösung von Dichlordimethylsilan (1.9 g, 0.5 eq) in trockenem THF (20 mL) bei -78 °C wird die Mischung unter Rühren über Nacht auf Raumtemperatur erwärmt. Überschüssiges Butyllithium wird mit gesättigter Ammoniumchlorid-Lösung hydrolysiert. Das ausgefallene Produkt wird abfiltriert und gründlich mit CH₂Cl₂ gewaschen. Säulenchromatographie (SiO₂, Hexan/EtOAc 10:1) gibt das Produkt. Ausbeute 74%. ¹H-NMR (CDCl₃, 400 MHz): *δ*= 0.7 (s, 6H), 7.25 (dd, 4H), 7.40 (d, 2H), 7.42-7.50 (m, 6H), 7.55-7.65 (m, 6H) 8.22 (s, 2H), 8.30 (s, 2H).

### Schritt 2:

Eine Lösung von Produkt von Schritt 1 (3.5 g, 1 eq) in trockenem THF (100 mL) wird bei -78 °C unter Argon langsam mit *n*-Butyllithium (1.6 M in Hexan, 7.8 mL, 2.5 eq) ver-setzt und 1,5 h bei -78 °C gerührt. Nach Zugabe einer Lösung von Chlortriphenylsilan (3.6 g, 2.5 eq) in trockenem THF (20 mL) bei -78 °C wird die Mischung unter Rühren über Nacht auf Raumtemperatur erwärmt. Überschüssiges Butyllithium wird mit gesät-tigter Ammoniumchlorid-Lösung hydrolysiert. Das ausgefallene Produkt wird abfiltriert und gründlich mit CH₂Cl₂ gewaschen. Aufkochen mit Aceton und abfiltrieren gibt das Produkt. Ausbeute: 45%.
**¹H-NMR** (CDCl₃, 400 MHz):
*δ*= 0.65 (s, 6H), 7.28-7.38 (m, 22H), 7.42 (t, 2H), 7.51 (m, 12H), 7.61 (d, 12H) 8.20 (s, 2H), 8.32 (s, 2H).

### Beispiel 5

### Kupplung von bromierten Carbazolderivaten (X = N-H) mit Silylverbindungen

### Beispiel 5a: Synthese von 3,6-Bis(triphenylsilyl)-9H-carbazol ohne Zwischenisolierung (Möglichkeit 1)

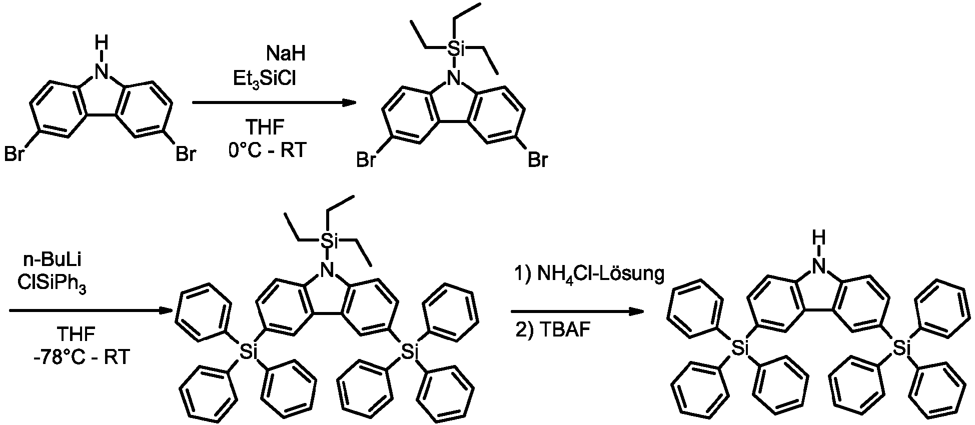

Eine Lösung von 3,6-Dibromcarbazol (9.1 g, 1 eq) in trockenem THF (400 mL) wird bei 0°C unter Argon langsam mit NaH (60% in Mineralöl, 1.3 g, 1.2 eq) versetzt und 2 h bei 0°C gerührt. Nach Zugabe einer Lösung von Chlortriethylsilan (5.1 g, 1.2 eq) in tro-ckenem THF (80 mL) wird die Lösung 1h bei RT (Raumtemperatur) gerührt. Die Lö-sung wird auf -78°C gekühlt und mit *n*-Butyllithium (1.6 M in Hexan, 43.8 mL, 2.5 eq) versetzt und 1h bei -78°C gerührt. Nach Zugabe einer Lösung von Chlortriphenylsilan (29.8 g, 3.5 eq) in trockenem THF (100 mL) bei -78 °C wird die Mischung unter Rühren über Nacht auf Raumtemperatur erwärmt. Überschüssiges Butyllithium wird mit gesät-tigter Ammoniumchlorid-Lösung hydrolysiert. Das ausgefallene Produkt wird abfiltriert und gründlich mit Methylenchlorid gewaschen. Die vereinigten Methylenchlorid-Filtrate werden mit Wasser ausgeschüttelt und über Natriumsulfat getrocknet. Die organische Phase wird mit Tetra-n-butylammoniumfluorid (TBAF,1 M in THF, 2 mL) versetzt und 4h bei RT gerührt. Die Mischung wird bis auf 100 mL eingeengt. Der entstandene Nie-derschlag wird filtriert und mit n-Hexan gewaschen. Ausbeute 66%.
¹H-NMR (CDCl₃, 400 MHz): *δ*= 7.4 (m, 20H), 7.6 (m, 14H), 8.18 (s, 2H), 8.20 (s, 1H).

### Beispiel 5b: Synthese von 3,6-Bis(triphenylsilyl)-9H-carbazol mit Zwischenisolierung

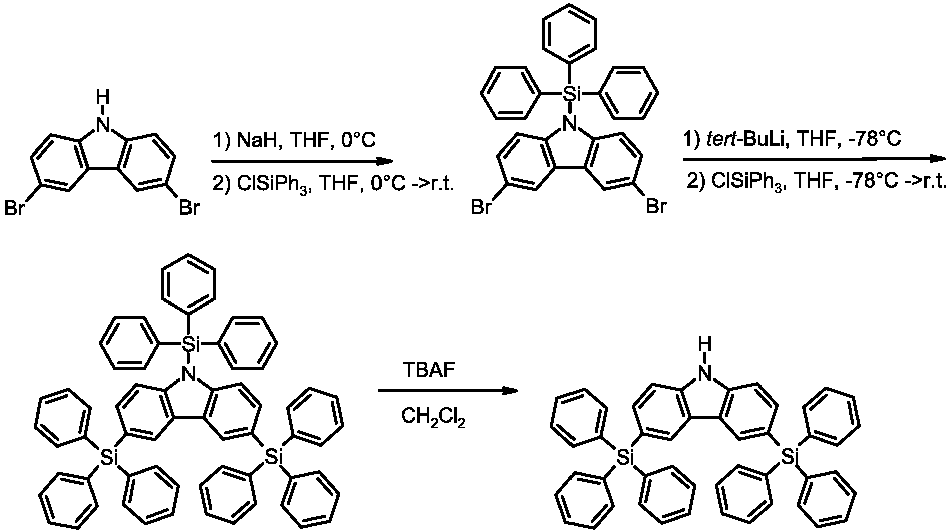

### i) 3,6-Dibromo-9-triphenylsilyl-carbazol

Zu einer kalten Lösung (0°C) von 3,6-Dibromocarbazol (10.4 g, 31 mmol) in trockenem THF (1000 ml) wird langsam NaH (60%ige Dispersion in Öl, 1.5 g, 37 mmol) zugegeben. Nach zweistündigem Rühren wird eine Lösung von ClSiPh₃ (18.7 g, 62 mmol) in trockenem THF (200 ml) bei 0°C zugegeben. Die Mischung wird über Nacht gerührt, und gesättigte NH₄Cl-Lösung wird zugegeben. Das entstandene Salz wird abfiltriert, und die organische Phase wird von der wässrigen Phase getrennt. Die wässrige Phase wird mit CH₂Cl₂ extrahiert. Die organische Phase wird zweimal mit Wasser gewaschen. Die vereinigten organischen Phasen werden über Na₂SO₄ getrocknet. Filtration über SiO₂ ergibt das gewünschte Produkt (quantitativ).

### ii) 3,6,9-Tris-triphenylsilyl-carbazol

Zu einer kalten Lösung (-78°C) von 3,6-Dibromo-9-triphenylsilyl-carbazol (90% Rein-heit, siehe i), 13 g, 22 mmol) in trockenem THF (1000 ml) wird langsam *tert*-BuLi (1.7 M in Pentan, 58 ml, 99 mmol) zugegeben. Nach zweistündigem Rühren bei -78°C wird ClSiPh₃ (34 g, 115 mmol) in trockenem THF zugegeben. Die Mischung wird über Nacht gerührt, und gesättigte NH₄Cl-Lösung wird zugegeben. Das entstandene Salz wird ab-filtriert, und die organische Phase wird von der wässrigen Phase getrennt. Die wässri-ge Phase wird mit CH₂Cl₂ extrahiert. Die organische Phase wird zweimal mit Wasser gewaschen. Die vereinigten organischen Phasen werden über Na₂SO₄ getrocknet. Nach Säulenchromatographie (SiO₂; Cyclohexan:CH₂Cl₂ 5:1) wird das reine Produkt erhalten (R_{f} ∼ 0.3, 11 g, 53%).

### iii) 3,6-Bis-triphenylsilyl-9H-carbazol

Zu einer Lösung von 3,6,9-Tris-triphenylsilyl-carbazol (8.7 g, 9.2 mmol) in CH₂Cl₂ (150 ml) wird eine TBAF-Lösung (1M in THF, 4.6 ml, 4.6 mmol) zugegeben. Nach einstündi-gem Rühren zeigt die Dünnschichtchromatographie eine vollständige Entschützung. Das Lösungsmittel wird im Vakuum entfernt, und der Rückstand wird in Cyclohexan (150 ml) unter Rückfluss erhitzt. Nach Abkühlung wird die Suspension filtriert. Der Rückstand wird über SiO₂ filtriert (Cyclohexan:EtOAc 10:1), wobei das gewünschte Produkt erhalten wird (7.6 g, 97%). ¹H-NMR (CDCl₃, 400 MHz): δ = 7.4(m, 20H), 7.6 (m, 14H), 8.18 (s, 2H), 8.20 (s, 1H).

### Beispiel 6

### N-Arylierung von 3,6-silylierten Carbazolderivaten (X = N-H)

Allgemeine Arbeitsvorschrift:

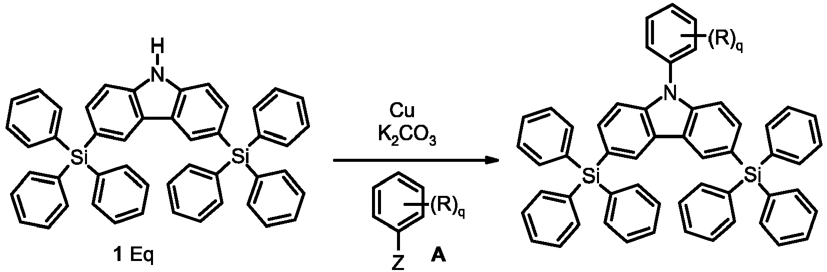

3,6-Bis(triphenylsilyl)-9H-carbazol (1 Eq), Phenylhalogenid **A,** Kaliumcarbonat und Kupferpulver werden auf 150-200 °C erhitzt und übernacht bei dieser Temperatur ge-rührt. Nach dem Abkühlen auf Raumtemperatur wird mit Methylenchlorid extrahiert. Der Niederschlag wird abfiltriert und säulenchromatographisch (Kieselgel, Methylenchlorid/Cyclohexan) aufgereinigt.

In der nachfolgenden Tabelle sind die Daten verschiedener N-Arylierungen von silylier-ten Carbazolderivaten, die entsprechend der allgemeinen Vorschrift durchgeführt wur-den, zusammengefasst:

| 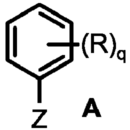 | Equiv. **A** | Equiv. K₂CO₃ | Mol% Cu | T (°C) | Ausbeute | Analytik |
|---|---|---|---|---|---|---|
| 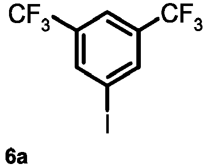 | 14.5 | 2.5 | 21 | 170 | 61 | ¹H-NMR (CDCl₃, 400 MHz): *δ*= 7.3 (dd, 12H), 7.4 (m, 8H), 7.6 (d, 12H), 7.65 (d, 2H), 7.95 (s, 1H), 8.1 (s, 2H), 8.2 (s, 2H). |
| 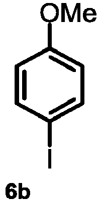 | 6 | 2.5 | 21 | 160 | 71 | ¹H-NMR (CDCl₃, 400 MHz): *δ*= 3.9 (s, 3H), 7.1 (d, 2H), 7.35 (m , 14H), 7.45 (m, 8H), 7.55 (d, 2H), 7.6 (d, 12H), 8.2 (s, 2H). |
| 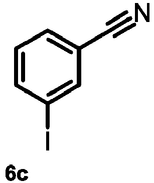 | 4 | 2.5 | 21 | 170 | 50 | ¹H-NMR (DMF-d7, 400 MHz): *δ*= 7.5 (m, 18H), 7.6 (d, 12H), 7.7 (m, 4H), 8.0 (t, 1H), 8.1 (d, 1H), 8.2 (d, 1H), 8.3 (s, 2H), 8.35 (s, 1H). |
| 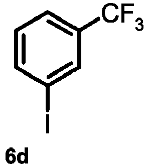 | 4 | 2.5 | 21 | 155 | 80 | ¹H-NMR (CDCl₃, 400 MHz): *δ*= 7.3-7.5 (m, 20H), 7.6 (d, 14H), 7.7 (d, 2H), 7.8 (m, 1H), 7.9 (s, 1H), 8.2 (d, 2H). |
| 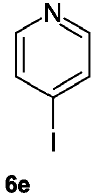 | 1.0 | Cs₂CO₃ 1.75 | 10 (Cul) | 110 | 62 In Nitrobenzol mit 0.2 Equiv. Phenanthrolin | ¹H-NMR (CD₂Cl₂, 400 MHz): *δ* = 7.38 (dd, 12H), 7.43 (m, 6H), 7.58 (d, 12H), 7.65 (m, 6H), 8.2 (s, 2H), 8.8 (br, 2H). |
| 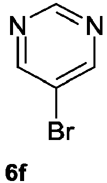 | 2.0 | 2.5 | 20 | 160 | 73 | ¹H-NMR (CD₂Cl₂, 400 MHz): *δ*= 7.38 (m, 12H), 7.42 (m, 8H), 7.58 (d, 12H), 7.61 (d, 2H), 8.2 (s, 2H), 9.05 (s, 2H), 9.15 (s, 1H). |
| 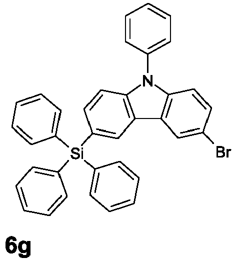 | 2.0 | 2.5 | 22 | 190 | 56 | ¹H-NMR (CDCl₃, 400 MHz): *δ =* 7.3-7.7 (m, 58H), 8.15 (s, 1H), 8.22 (s, 2H), 8.28 (s, 1H). |
| 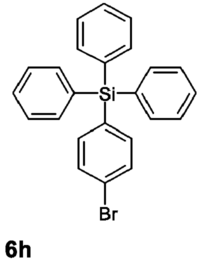 | 3.9 | 2.5 | 20 | 180 | 40 | ¹H-NMR (CDCl₃, 400 MHz): *δ =* 7.32 (t, 12H), 7.35-7.50 (m, 17H), 7.6 (m, 22H), 7.75 (d, 2H), 8.2 (s, 2H). |
| 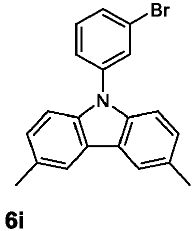 | 3.9 | 2.5 | 20 | 170 | 45 | ¹H-NMR (CDCl₃, 400 MHz): *δ =* 2.5 (s, 6H), 7.20 (d, 2H), 7.3-7.5 (m, 20H), 7.50-7.61 (m, 16H), 7.65 (d, 2H), 7.8 (dd, 2H), 7.9 (d, 2H), 8.2 (s, 2H). |
| 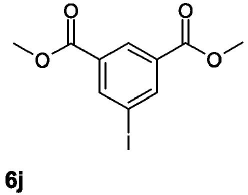 | 8.0 | 2.5 | 20 | 130 | 55 | ¹H-NMR (CDCl₃, 400 MHz): *δ* = 4.0 (s, 6H), 7.30-7.45 (m, 20H), 7.6 (2x d, gesammt 14H), 8.2 (s, 2H), 8.45 (s, 2H), 8.75 (s, 1H). |
| 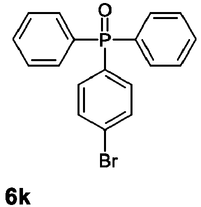 | 3.1 | 2.5 | 20 | 160 | 40 | ¹H-NMR (CDCl₃, 400 MHz): *δ =* 7.35 (dd, 12H), 7.42, (m, 6H), 7.50 (m, 6H), 7.58 (m, 16H), 7.72 (m, 6H), 7.88 (dd, 2H), 8.2 (s, 2H). |
| 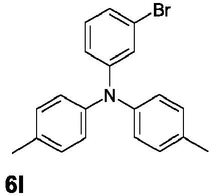 | 3.0 | 2.5 | 20 | 180 | 65 | ¹H-NMR (CDCl₃, 400 MHz): *δ* = 2.25 (s, 6H), 7.05 (m, 10H), 7.14, (s, 1H), 7.35 (m, 13H), 7.42 (m, 8H), 7.5 (d, 2H), 7.6 (d, 12H), 8.18 (s, 2H). |
| 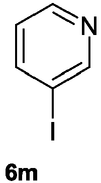 | 6.0 | 2.5 | 20 | 155 | 85 | ¹H-NMR (CD₂Cl₂, 400 MHz): *δ* = 7.30-7.50 (m, 20H), 7.60 (m, 15H), 7.95 (br, 1H), 8.2 (s, 2H), 8.7 (br, 1H), 8.8 (br, 1H). |
| 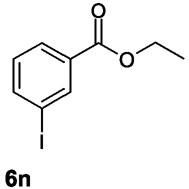 | 3.5 | 2.5 | 20 | 140 | 57 | ¹H-NMR (CDCl₃, 400 MHz): *δ* = 1.35 (t, 3H), 4.40 (q, 2H), 7.4 (m, 20H), 7.60 (m, 14H), 7.63 (t, 1H), 7.75 (d, 1H), 8.12 (d, 1H), 8.19 (s, 2H), 8.22 (s, 1H). |
| 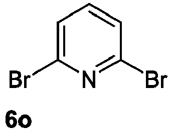 | 0.5 | 1.5 | 20 | 200 | 37 | ¹H-NMR (CDCl₃, 400 MHz): *δ =* 7.32 (dd, 24H), 7.40 (t, 12H), 7.56 (m, 28H), 7.62 (d, 2H), 7.94 (d, 4H), 8.10 (t, 1H), 8.18 (s, 4H). |
| 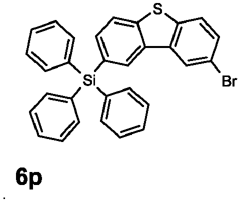 | 2 | 5 | 40 | 210 | 54 | MALDI-MS (m/z)= 1124 |
| Synthese von Edukt analog an **4g** | | | | | | |

### Beispiel 7

### Dreifachsubstitution von 2,4,6-Trichloro-1,3,5-triazin (Cyanurchlorid) zur Darstellung von 2,4,6-Tris-(3,6-bis(triphenylsilanyl)carbazol-9-yl)-1,3,5-triazin

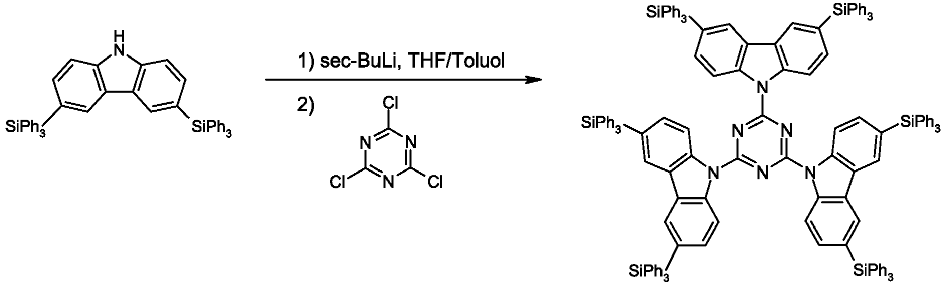

*Allg. Vorschrift:* 1.70 g (2.50 mmol) 3,6-Bis-triphenylsilanyl-9H-carbazol werden in ei-nem 100 ml 2-Halskolben, bestückt mit Stickstoffeinlass und Septum, in 50 ml absolu-tem Toluol unter Stickstoffatmosphäre, gelöst. Anschließend wird die Lösung bei Raumtemperatur über einen Zeitraum von 10 Minuten mit 1.92 ml (2.50 mmol) sec-Bu-thyllithium (1.3M in Cyclohexan) versetzt und weitere 10 Minuten gerührt. In einem 250 ml 3-Halskolben, bestückt mit Stickstoffeinlass, Rückflusskühler und Septum, werden 0.14 g (0.75 mmol) Cyanurchlorid in einer Mischung aus 10ml absolutem THF und 20ml absolutem Toluol unter Stickstoffatmosphäre, gelöst. Die Carbazol-Lösung wird der Cyanurchlorid-Lösung mittels einer Transferkanüle über einen Zeitraum von 20 Mi-nuten zugetropft. Das Reaktionsgemisch wird anschließend 4 Stunden unter Rückfluss gekocht. Nach Abkühlen auf Raumtemperatur wird das Lösungsmittel verdampft und der Rückstand 10 Minuten in 200 ml heißem Hexan gerührt. Der durch Filtration gewonnene Feststoff wird mit Diethylether gewaschen, in heißem Ethanol aufgeschlämmt und heiß filtriert. Die Reinigung des Produkts erfolgt mittels Säulechromatographie mit einem Hexan/Toluol Elutionsmittelgemisch (1/4, V/V), wobei
0.84 g (53%) 2,4,6-Tris-(3,6-bis(triphenylsilanyl)carbazol-9-yl)-1,3,5-triazin als weißer Feststoff erhalten werden.
**¹H-NMR** (250 MHz, CDCl₃)
δ (ppm): 8.97(d, 6H), 8.08 (s, 6H), 7.63 (d, 6H), 7.60-7.53 (m, 30H), 7.42-7.16 (m, 60H).
**MALDI-TOF:** m/z = 2126.53 (M⁺)

### B Anwendungsbeispiele

### Beispiel 1:

### Typische Arbeitsvorschrift für die Herstellung einer OLED:

Das als Anode verwendete ITO-Substrat wird zuerst mit kommerziellen Reinigungsmit-teln für die LCD-Produktion (Deconex® 20NS und Neutralisationsmittel 25ORGAN-ACID®) und anschließend in einem Aceton/Isopropanol-Gemisch im Ultraschallbad ge-säubert. Zur Beseitigung möglicher organischer Rückstände wird das Substrat in einem Ozonofen weitere 25 Minuten einem kontinuierlichen Ozonfluss ausgesetzt. Diese Be-handlung verbessert auch die Lochinjektionseigenschaften des ITOs.

Danach werden die nachfolgend genannten organischen Materialien mit einer Rate von ca. 0.5-5 nm/min bei etwa 10⁻⁸ mbar auf das gereinigte Substrat aufgedampft. Als Lochleiter und Excitonenblocker wird Ir(dpbic)₃ mit einer Dicke von 45 nm auf das Sub-strat aufgebracht. 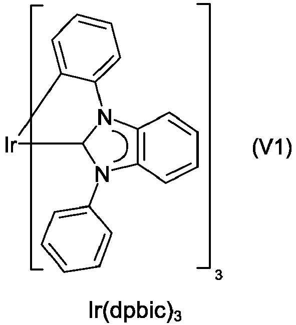 (zur Herstellung siehe Ir-Komplex (7) in der Anmeldung WO 2005/019373).

Anschließend wird eine Mischung aus 7,5 Gew.-% der Verbindung V5 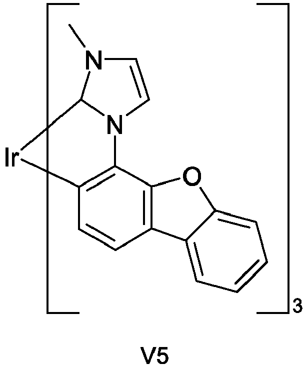 und 92,5 Gew.-% der Verbindung 9-Phenyl-3,6-bis(triphenylsilyl)-carbazol (Beispiel 4b) in einer Dicke von 40 nm aufgedampft, wobei erstere Verbindung als Emitter, letztere als Matrixmaterial fungiert.

Anschließend wird das Material 9-Phenyl-3,6-bis(triphenylsilyl)-carbazol (Beispiel 4b) mit einer Dicke von 10 nm als Excitonen- und Lochblocker aufgedampft.

Als nächstes wird ein Elektronentransporter BCP (2,9-Dimethyl-4,7-diphenyl-1,10-phenanthrolin) in einer Dicke von 50 nm, eine 0,75 nm dicke Lithiumfluorid-Schicht und abschließend eine 110 nm dicke AI-Elektrode aufgedampft.

Zur Charakterisierung der OLED werden Elektrolumineszenz-Spektren bei verschiede-nen Strömen bzw. Spannungen aufgenommen. Weiterhin wird die Strom-Spannungs-Kennlinie in Kombination mit der abgestrahlten Lichtleistung gemessen. Die Lichtleis-tung kann durch Kalibration mit einem Luminanzmeter in photometrische Größen um-gerechnet werden.

In der nachfolgenden Tabelle 1 sind die Elektrolumineszenzdaten (Stromeffizienz, Quanteneffizienz (QE) und Spannung) für verschiedene OLEDs angegeben. Der Auf-bau der jeweiligen OLED ist in der rechten Spalte von Tabelle 1 (Device Aufbau) ange-geben. Die Herstellung der jeweiligen OLED erfolgt entsprechend der vorstehend ge-nannten typischen Arbeitsvorschrift. Die in Tabelle 1 angegebenen Werte für die Stro-meffizienz, Quanteneffizienz (QE) und Spannung sind relative Werte, jeweils bezogen auf eine Referenz-OLED, die im Aufbau mit der jeweiligen OLED identisch ist und sich darin von der jeweiligen OLED unterscheidet, dass sowohl als Matrix als auch als Excitonen- und Lochblocker 9-Phenyl-3,6-bis(triphenylsilyl)-carbazol (Beispiel 4b) einge-setzt werden. Die in Tabelle 1 genannten Werte für die Stromeffizienz, Quanteneffizi-enz (QE) und Spannung werden für die jeweilige Referenz-OLED als 100 % definiert. Ein Vergleich einer Diode, worin 9-Phenyl-3,6-bis(triphenylsilyl)-carbazol (Beispiel 4b) sowohl als Matrix als auch als Excitonen- und Lochblocker eingesetzt wird, mit einer Diode mit einem identischen Aufbau, worin jeweils anstelle von 9-Phenyl-3,6-bis(triphe-nylsilyl)-carbazol (Beispiel 4b) (das synthetisch wesentlich schwerer zugängliche) 9-(4-tert-Butylphenyl)-3,6-bis(triphenylsilyl)-carbazol eingesetzt wird, zeigt, dass die Elektro-lumineszenzdaten beider OLEDs nur unwesentlich voneinander abweichen. Somit dient die jeweils als Referenz-OLED verwendete OLED, die sowohl als Matrix als auch als Excitonen- und Lochblocker 9-Phenyl-3,6-bis(triphenylsilyl)-carbazol (Beispiel 4b) aufweist, auch als Referenz für eine OLED, die 9-(4-tert-Butylphenyl)-3,6-bis(triphenylsilyl)-carbazol sowohl als Matrix als auch als Excitonen- und Lochblocker aufweist.

| **Mate-rial** | **Struktur** | **Funktion** | **(Stromeffi-zienz (max)/ Stro-meffizienz (max, stan-dard)) 100%** | **QE (max)/ QE (max, Standard)) 100%** | **Span-nung bei 300 nits/Spa nnung bei 300 nits (stan-dard)** 100% | **Device Aufbau (ITO 125 +/-20nm)** (analog zur typischen Arbeitsvor-schrift, Austausch von **4b** durch neues Material) |
|---|---|---|---|---|---|---|
| **4b (stan-dard)** | 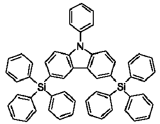 | Matrix+Blocker | 100 | 100 | 100 | siehe vorstehend genannte Arbeits-vorschrift |
| **6a** | 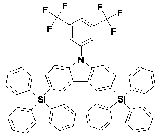 | Blocker | 121 | 123 | 99 | V1 (45nm)//8,5%V5:**4b** (40nm)//**6a** (10nm)//BCP (50nm)//LiF//Alu |
| | | Matrix | 106 | 100 | 103 | V1 (45nm)//8,5%V5:**6a** (40nm)//**4b** |
| | | | | | | (10nm)//BCP (50nm)//LiF//Alu |
| | | Matrix+Blocker | 111 | 100 | 93 | V1 (45nm)//8,5%V5:**6a** (40nm)//**6a** (10nm)//BCP (50nm)//LiF//Alu |
| **6b** | 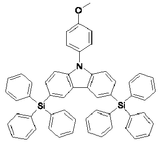 | Blocker | 99 | 98 | 100 | V1 (45nm)//8,5%V5:**4b** (40nm)//**6b** (10nm)//BCP (50nm)//LiF//Alu |
| | | Matrix | 109 | 108 | 95 | V1 (45nm)//8,5%V5:**6b** (40nm)//**4b** (10nm)//BCP (50nm)//LiF//Alu |
| | | Matrix+Blocker | 106 | 106 | 98 | V1 (45nm)//8,5%V5:**6b** (40nm)//**6b** (10nm)//BCP (50nm)//LiF//Alu |
| **6c** | 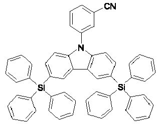 | Matrix+Blocker | Keine ver-gleichbaren Referenz-werte ver-fügbar | Keine ver-gleichbaren Referenz-werte ver-fügbar | 75 | V1 (40nm)//7,5%V5:**6c** (40nm)//**6c** (5nm)//BCP (50nm)//LiF//Alu |
| **6d** | 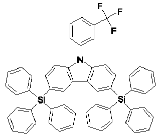 | Matrix+Blocker | Keine ver-gleichbaren Referenz-werte ver-fügbar | Keine ver-gleichbaren Referenz-werte ver-fügbar | 87 | V1 (40nm)//7,5%V5:**6d** (40nm)//**6d** (5nm)//BCP (50nm)//LiF//Alu |
| **6e** | 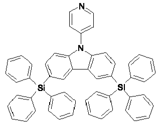 | Blocker | 124 | 130 | 94 | 10%MoO₃:V1 (35nm)//V1 (10nm)//7,5%V5:**4b** (40nm)//**6e** (10nm)//BCP (50nm)//LiF//Alu |
| | | Matrix | 118 | 109 | 84 | 10%MoO₃:V1 (35nm)/V1 (10nm)//7,5%V5:**6e** (40nm)//**4b** (10nm)//BCP (50nm)//LiF//Alu |
| | | Matrix+Blocker | 144 | 139 | 78 | 10%MoO₃:V1 (35nm)/V1 (10nm)//7,5%V5:**6e** (40nm)//**6e** (10nm)//BCP (50nm)//LiF//Alu |
| **6f** | 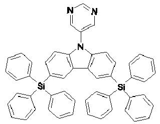 | Blocker | 158 | 177 | 92 | V1 (45nm)//7,5%V5:V1 (10nm)//7,5%V5:**4b** (40nm)//**6f** (10nm)//BCP (40nm)//LiF//Alu |
| | | Matrix | 125 | 117 | 74 | V1 (45nm)//8,5%V5:V1 (10nm)//8,5%V5:**6f** (40nm)//**4b** (10nm)//BCP (40nm)//LiF//Alu |
| | | Matrix+Blocker | 138 | 137 | 65 | V1 (45nm)//8,5%V5:V1 (10nm)//8,5%V5:**6f** (40nm)//**6f** (10nm)//BCP (40nm)//LiF//Alu |
| **6m** | 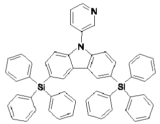 | Blocker | 113 | 113 | 86 | NPD (45nm)//8,5%V5:**4b** (40nm)//**6m** (10nm)//BCP (40nm)//LiF//Alu |
| | | Matrix | 126 | 124 | 87 | NPD (45nm)//8,5%V5:**6m** (40nm)//**4b** (10nm)//BCP (40nm)//LiF//Alu |
| | | Matrix+Blocker | 151 | 149 | 94 | NPD (45nm)//8,5%V5:**6m** |
| | | | | | | (40nm)//**6m** (10nm)//BCP (40nm)//LiF//Alu |
| | | Matrix+Blocker | 130 | 135 | 78 | V1 (45nm)//8,5%V5:V1 (10nm)//8,5%V5:**6m** (40nm)//**6m** (10nm)//BCP (40nm)//LiF//Alu |

### Beispiel 2

Die nachstehend in Tabelle 2 genannten OLEDs werden entsprechend der in Beispiel 1 genannten typischen Arbeitsvorschrift hergestellt. In der nachfolgenden Tabelle sind die Elektrolumineszenzdaten (Stromeffizienz, Quanteneffizienz (QE) und Spannung) für verschiedene OLEDs angegeben. In den nachstehend genannten OLEDs wurde anstelle des Materials 9-Phenyl-3,6-bis(triphenylsilyl)-carbazol (Beispiel **4b)** gemäß der typischen Arbeitsvorschrift gemäß Beispiel 1 jeweils eine andere Verbindung der Formel II als Matrixmaterial und/oder Blockermaterial eingesetzt. Sonst unterscheiden sich die nachstehend genannten OLEDs nicht von der vorstehend beschriebenen OLED.

**Tabelle 2**

| | **Material** | **Funktion** | **Stromeffizi-enz (max) (cd/A)** | **QE (max) (%)** | **V bei 300 nits (V)** | **Device Aufbau (ITO 125 +/-20nm)** (Analog an typische Arbeitsvor-schrift, Austausch von **4b** durch neues Material) |
|---|---|---|---|---|---|---|
| **4a** | 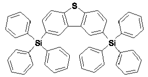 | Matrix+Blocker | 10,8 | 7,2 | 8,7 | V1 (45nm)//8,5%V5:**4a**(40nm)//**4a** (10nm)//TPBI (50nm)//LiF//Alu |
| 4g | 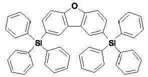 | Blocker | 7,9 | 7,0 | 9,7 | 10%MoO₃:V1 (35nm)/V1 (10nm)//7,5%V5:**4b** (40nm)//**4g** (10nm)//BCP (40nm)//LiF//Alu |
| | | Matrix | 8,4 | 7,1 | 8,9 | 10%MoO₃:V1 (35nm)//V1 (10nm)//7,5% V5:**4g** (40nm)//**4b** (10nm)//BCP (40nm)//LiF//Alu |
| | | Matrix+Blocker | 9,7 | 8,2 | 8,1 | 10%MoO₃:V1 (35nm)//V1 (10nm)//7,5% V5:**4g** (40nm)//**4g** (10nm)//BCP (40nm)//LiF//Alu |

## Patentansprüche

1. Verbindung der allgemeinen Formel I: 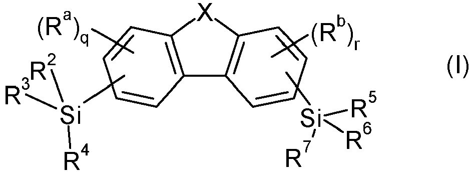 worin bedeuten:
X NR¹, S, O, PR¹, SO₂ oder SO
R¹ substituiertes oder unsubstituiertes C₆-C₃₀-Aryl, bevorzugt substituiertes oder unsubstituiertes C₆-C₁₀-Aryl, besonders bevorzugt substituiertes oder unsubstituiertes Phenyl, wobei der Substituent ausgewählt ist aus der Gruppe bestehend aus C₁-C₂₀-Alkyl, unsubstituiertes C₆-C₃₀-Aryl, C₆-C₃₀-Aryl, welches mit einer oder mehreren C₁-C₂₀-Alkoxygruppen, CN, CF₃, F, C₆-C₃₀-Aryloxy-, oder C₆-C₃₀-Alkylthiogruppen substituiert ist, oder C₁-C₂₀-Alkoxy, C₆-C₃₀-Aryloxy, C₁-C₂₀-Alkylthio, C₆-C₃₀-Arylthio, SiR¹⁴R¹⁵R¹⁶, Halogenresten, halogenierten C₁-C₂₀-Alkylresten, Carbonyl (-CO(R¹⁴)), Carbonylthio (- C = O (SR¹⁴)), Carbonyloxy (- C = O(OR¹⁴)), Oxycarbonyl (- OC = O(R¹⁴)), Thiocarbonyl (- SC = O(R¹⁴)), Amino (-NR¹⁴R¹⁵), OH, Pseudohalogenresten, Amido (- C = O (NR¹⁴)), -NR¹⁴C = O (R¹⁵), Phosphonat (- P(O) (OR¹⁴)₂, Phosphat (-OP(O) (OR¹⁴)₂), Phosphin (-PR¹⁴R¹⁵), Phosphinoxid (-P(O)R¹⁴₂), Sulfat (-OS(O)₂OR¹⁴), Sulfoxid (-S(O)R¹⁴), Sulfonat (-S(O)₂OR¹⁴), Sulfonyl (-S(O)₂R¹⁴), Sulfonamid (-S(O)₂NR¹⁴R¹⁵), NO₂, Boronsäureestern (-OB(OR¹⁴)₂), Imino (-C = NR¹⁴R¹⁵)), Boranresten, Stannanresten, Hydrazinresten, Hydrazonresten, Oximresten, Nitroso-Gruppen, Diazo-Gruppen, Vinylgruppen, (=Sulfonat) und Boronsäuregruppen, Sulfoximine, Alane, Germane, Boroxime und Borazine; oder substituiertes oder unsubstituiertes Heteroaryl mit 5 bis 30 Ringatomen;
R², R³, R⁴, R⁵, R⁶, R⁷ unabhängig voneinander substituiertes oder unsubstituiertes C₁-C₂₀-Alkyl oder substituiertes oder unsubstituiertes C₆-C₃₀-Aryl oder eine Struktur der allgemeinen Formel (c) 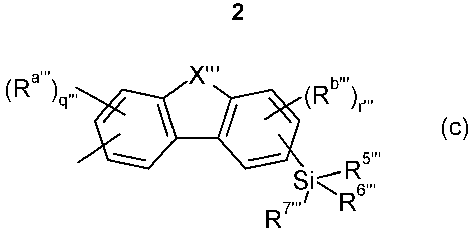
R^{a}, R^{b} unabhängig voneinander substituiertes oder unsubstituiertes C₁-C₂₀-Alkyl, substituiertes oder unsubstituiertes C₆-C₃₀-Aryl, substituiertes oder unsubstituiertes Heteroaryl mit 5 bis 30 Ringatomen oder ein Substituent mit Donor- oder Akzeptorwirkung ausgewählt aus der Gruppe bestehend aus: C₁-C₂₀-Alkoxy, C₆-C₃₀-Aryloxy, C₁-C₂₀-Alkylthio, C₆-C₃₀-Arylthio, SiR¹⁴R¹⁵R¹⁶, Halogenresten, halogenierten C₁-C₂₀-Alkylresten, Carbonyl (-CO(R¹⁴)), Carbonylthio (- C = O (SR¹⁴)), Carbonyloxy (- C = O(OR¹⁴)), Oxycarbonyl (- OC = O(R¹⁴)), Thiocarbonyl (- SC = O(R¹⁴)), Amino (-NR¹⁴R¹⁵), OH, Pseudohalogenresten, Amido (- C = O (NR¹⁴)), -NR¹⁴C = O (R¹⁵), Phosphonat (-P(O) (OR¹⁴)₂, Phosphat (-OP(O) (OR¹⁴)₂), Phosphin (-PR¹⁴R¹⁵), Phosphinoxid (-P(O)R¹⁴₂), Sulfat (-OS(O)₂OR¹⁴), Sulfoxid (-S(O)R¹⁴), Sulfonat (-S(O)₂OR¹⁴), Sulfonyl (-S(O)₂R¹⁴, Sulfonamid (-S(O)₂NR¹⁴R¹⁵), NO₂, Boronsäureestern (-OB(OR¹⁴)₂), Imino (-C = NR¹⁴R¹⁵)), Boranresten, Stannanresten, Hydrazinresten, Hydrazonresten, Oximresten, Nitroso-Gruppen, Diazo-Gruppen, Vinylgruppen, , Sulfoximinen, Alanen, Germanen, Boroximen und Borazinen.
R¹⁴, R¹⁵, R¹⁶ unabhängig voneinander substituiertes oder unsubstituiertes C₁-C₂₀-Alkyl oder substituiertes oder unsubstituiertes C₆-C₃₀-Aryl;
q, r unabhängig voneinander 0, 1, 2 oder 3; wobei in dem Fall, wenn q bzw. r 0 bedeuten, alle substituierbaren Positionen des Arylrests mit Wasserstoff substituiert sind,
wobei die Reste und Indices in der Gruppe der Formel (c) X''', R⁵''', R⁶''', R⁷''', R^{a}''', R^{b}''', q''' und r''' unabhängig voneinander die für die Reste und Indices der Verbindungen der allgemeinen Formel (I) X, R⁵, R⁶, R⁷, R^{a}, R^{b}, q und r genannten Bedeutungen aufweisen,
wobei die folgenden Verbindungen ausgenommen sind:

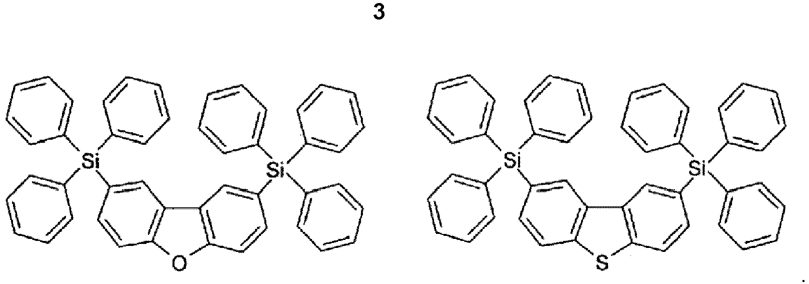

2. Verbindung nach Anspruch 1, **dadurch gekennzeichnet, dass** mindestens einer der Reste R², R³ oder R⁴ und/oder mindestens einer der Reste R⁵, R⁶ oder R⁷ substituiertes oder unsubstituiertes C₆-C₃₀-Aryl bedeuten.

3. Verbindung nach Anspruch 1 oder 2, **dadurch gekennzeichnet, dass** die Verbin-dung der allgemeinen Formel (I) eine 3,6-Disilyl-substituierte Verbindung der all-gemeinen Formel (Ia) ist: 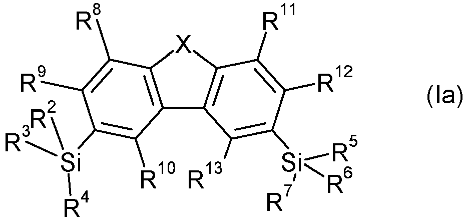 worin bedeuten:
X NR¹, S, O, PR¹, SO₂ oder SO; bevorzugt NR¹, S oder O; besonders bevorzugt NR¹
R¹ substituiertes oder unsubstituiertes C₁-C₂₀-Alkyl, substituiertes oder unsubstituiertes C₆-C₃₀-Aryl oder substituiertes oder unsubstituiertes Heteroaryl mit 5 bis 30 Ringatomen; bevorzugt substituiertes oder unsubstituiertes C₆-C₃₀-Aryl oder unsubstituiertes C₁-C₂₀-Alkyl, besonders bevorzugt substituiertes oder unsubstituiertes C₆-C₁₀-Aryl oder unsubstituiertes C₁-C₂₀-Alkyl, ganz besonders bevorzugt substituiertes oder unsubstituiertes Phenyl;
R², R³, R⁴, R⁵, R⁶, R⁷ unabhängig voneinander substituiertes oder unsubstituiertes C₁-C₂₀-Alkyl oder substituiertes oder unsubstituiertes C₆-C₃₀-Aryl oder eine Struktur der allgemeinen Formel (c); bevorzugt bedeutet mindestens einer der Reste R², R³ oder R⁴ und/oder mindestens einer der Reste R⁵, R⁶ oder R⁷ substituiertes oder unsubstituiertes C₆-C₃₀-Aryl, besonders bevorzugt substituiertes oder unsubstituiertes C₆-C₁₀-Aryl, ganz besonders bevorzugt substituiertes oder unsubstituiertes Phenyl und/oder einer der Reste R², R³ oder R⁴ und/oder einer der Reste R⁵, R⁶ oder R⁷ ist ein Rest der Struktur (c);
R⁸, R⁹, R¹⁰, R¹¹, R¹², R¹³ unabhängig voneinander Wasserstoff oder die für R^{a} und R^{b} genannten Bedeutungen, das heißt unabhängig voneinander substituiertes oder unsubstituiertes C₁-C₂₀-Alkyl, substituiertes oder unsubstituiertes C₆-C₃₀-Aryl, substituiertes oder unsubstituiertes Heteroaryl mit 5 bis 30 Ringatomen oder ein Substituent mit Donor- oder Akzeptorwirkung; bevorzugt Wasserstoff, substituiertes oder unsubstituiertes C₁-C₆-Alkyl, substituiertes oder unsubstituiertes C₆-C₁₀-Aryl oder SiR¹⁴R¹⁵R¹⁶; wobei R¹⁴, R¹⁵ und R¹⁶ bevorzugt unabhängig voneinander substituiertes oder unsubstituiertes C₁-C₂₀-Alkyl oder substituiertes oder unsubstituiertes Phenyl bedeuten; besonders bevorzugt ist mindestens einer der Reste R¹⁴, R¹⁵ oder R¹⁶ substituiertes oder unsubstituiertes Phenyl, ganz besonders bevorzugt ist mindestens einer der Reste R¹⁴,R¹⁵ und R¹⁶ substituiertes Phenyl, wobei geeignete Substituenten vorstehend genannt sind.

4. Verbindung nach einem der Ansprüche 1 bis 3, **dadurch gekennzeichnet, dass** die Reste R¹ bis R⁷, R^{a} und R^{b} sowie die Gruppe X die folgenden Bedeutungen aufweisen
X NR¹;
R¹ substituiertes oder unsubstituiertes C₆-C₃₀-Aryl, bevorzugt substituiertes oder unsubstituiertes C₆-C₁₀-Aryl, besonders bevorzugt substituiertes oder unsubstituiertes Phenyl;
R², R³, R⁴, R⁵, R⁶, R⁷ unabhängig voneinander substituiertes oder unsubstituiertes C₁-C₂₀-Alkyl oder substituiertes oder unsubstituiertes C₆-C₃₀-Aryl, oder eine Struktur der allgemeinen Formel (c) bevorzugt unabhängig voneinander substituiertes oder unsubstituiertes C₁-C₆-Alkyl oder substituiertes oder unsubstituiertes C₆-C₁₀-Aryl, besonders bevorzugt substituiertes oder unsubstituiertes C₁-C₆-Alkyl oder substituiertes oder unsubstituiertes Phenyl; wobei in einer Ausführungsform mindestens einer der Reste R², R³ oder R⁴ und/oder mindestens einer der Reste R⁵, R⁶ oder R⁷ substituiertes oder unsubstituiertes C₆-C₃₀-Aryl, bevorzugt substituiertes oder unsubstituiertes C₆-C₁₀-Aryl, besonders bevorzugt substituiertes oder unsubstituiertes Phenyl, bedeutet;
R⁸, R⁹, R¹⁰, R¹¹, R¹², R¹³ unabhängig voneinander Wasserstoff oder die für R^{a} und R^{b} genannten Bedeutungen, das heißt unabhängig voneinander substituiertes oder unsubstituiertes C₁-C₂₀-Alkyl, substituiertes oder unsubstituiertes C₆-C₃₀-Aryl, substituiertes oder unsubstituiertes Heteroaryl mit 5 bis 30 Ringatomen oder ein Substituent mit Donor- oder Akzeptorwirkung; bevorzugt Wasserstoff, substituiertes oder unsubstituiertes C₁-C₆-Alkyl, substituiertes oder unsubstituiertes C₆-C₁₀-Aryl oder SiR¹⁴R¹⁵R¹⁶; besonders bevorzugt Wasserstoff, Methyl, Ethyl, Phenyl, CF₃ oder SiR¹⁴R¹⁵R¹⁶;
R¹⁴, R¹⁵, R¹⁶ unabhängig voneinander substituiertes oder unsubstituiertes C₁-C₂₀-Alkyl oder substituiertes oder unsubstituiertes C₆-C₃₀-Aryl, bevorzugt substituiertes oder unsubstituiertes C₁-C₆-Alkyl oder substituiertes oder unsubstituiertes C₆-C₁₀-Aryl, wobei R¹⁴, R¹⁵ und R¹⁶ besonders bevorzugt unabhängig voneinander substituiertes oder unsubstituiertes C₁-C₂₀-Alkyl oder substituiertes oder unsubstituiertes Phenyl bedeuten; besonders bevorzugt ist mindestens einer der Reste R¹⁴,R¹⁵ oder R¹⁶ substituiertes oder unsubstituiertes Phenyl, ganz besonders bevorzugt ist mindestens einer der Reste R¹⁴,R¹⁵ und R¹⁶ substituiertes Phenyl.

5. Verbindung nach einem der Ansprüche 1 bis 4, **dadurch gekennzeichnet, dass** der Rest R¹, und/oder mindestens einer der Reste aus der Gruppe R², R³ und R⁴ und/oder mindestens einer der Reste aus der Gruppe R⁵, R⁶ und R⁷ unabhängig voneinander substituiertes oder unsubstituiertes C₆-Aryl der folgenden Formel bedeuten: 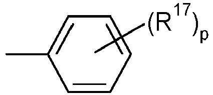 worin bedeuten
p 0, 1, 2, 3, 4 oder 5, bevorzugt 0, 1, 2 oder 3, besonders bevorzugt 0, 1 oder 2;
R¹⁷ Wasserstoff, substituiertes oder unsubstituiertes C₁-C₂₀-Alkyl, substituiertes oder unsubstituiertes C₆-C₃₀-Aryl, substituiertes oder unsubstituiertes Heteroaryl mit 5 bis 30 Ringatomen, ein Substituent mit Donor- oder Akzeptorwirkung, oder ein Rest der allgemeinen Formel a oder b 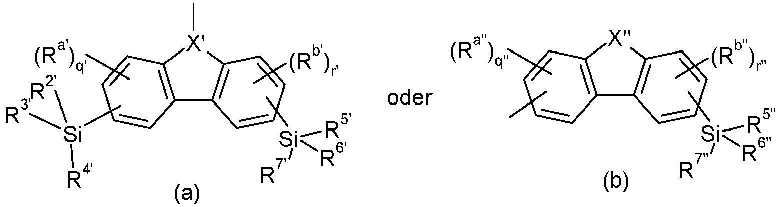
worin
X' N oder P bedeutet, und
die Reste und Indizes X", R^{2'}, R^{3'}, R^{4'}, R^{5'}, R^{5"}, R^{6'}, R^{6"}, R^{7'}, R^{7"}, R^{a'}, R^{a"}, R^{b'}, R^{b"}, q', q", r' und r" unabhängig voneinander für die Reste und Indizes X, R², R³, R⁴, R⁵, R⁶, R⁷, R^{a}, R^{b}, q und r genannten Bedeutungen aufweisen;
oder
einer der Reste R², R³ oder R⁴ und/oder einer der Reste R⁵, R⁶ oder R⁷ ein Rest der allgemeinen Formel c ist 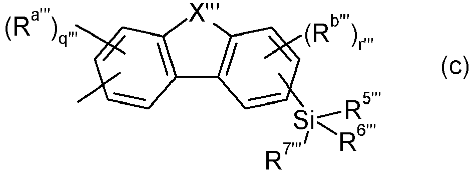
worin die Reste und Indizes X^{"'}, R^{5"'}, R^{6'}, R^{7"'}, R^{a"'}, R^{b"'}, q^{'"} und r^{'"} unabhängig voneinander die für die Reste und Indizes X, R⁵, R⁶, R⁷, R^{a}, R^{b}, q und r genannten Bedeutungen aufweisen.

6. Verbindung nach einem der Ansprüche 1 bis 5, **dadurch gekennzeichnet, dass** wenn in der Verbindung der allgemeinen Formel (I) die Gruppe X NR¹ bedeutet, mindestens einer der Reste R¹ bis R⁷, R^{a} oder R^{b} in den Verbindungen der Formel (I) mindestens ein Heteroatom enthält.

7. Verbindung nach einem der Ansprüche 1 bis 6, **dadurch gekennzeichnet, dass** die Verbindung der Formel (I) mindestens einen Rest R¹, R², R³, R⁴, R⁵, R⁶ oder R⁷ aufweist, der ausgewählt ist aus der Gruppe bestehend aus substituiertem oder unsubstituiertem C₁-C₂₀-Alkyl, Heteroaryl mit 5 bis 30 Ringatomen, unsubstituiertem C₆-C₃₀-Aryl, alkyl-substituiertem C₆-C₃₀-Aryl, mit mindestens einem Substituenten mit Donorwirkung substituiertem C₆-C₃₀-Aryl, mit Heteroaryl mit 5 bis 30 Ringatomen substituiertem C₆-C₃₀-Aryl und einem Substituenten mit Donorwirkung.

8. Verbindung nach einem der Ansprüche 1 bis 6, **dadurch gekennzeichnet, dass** die die Verbindung der Formel (I) mindestens einen Rest R¹, R², R³, R⁴, R⁵, R⁶ oder R⁷ aufweist, der ausgewählt ist aus der Gruppe bestehend aus mit mindestens einem Substituenten mit Akzeptorwirkung substituiertem C₁- bis C₂₀-Alkyl, mit mindestens einem Substituenten mit Akzeptorwirkung substituiertem C₆-C₃₀-Aryl, mit mindestens einem Heteroarylrest mit 5 bis 30 Ringatomen substituiertem C₆-C₃₀-Aryl und einem Substituenten mit Akzeptorwirkung.

9. Schicht enthaltend mindestens eine Verbindung der Formel (I) gemäß einem der Ansprüche 1 bis 8, wobei die Schicht ausgewählt ist aus der Gruppe bestehend aus: mindestens einer Licht-emittierenden Schicht, mindestens einer Blockschicht für Elektronen/Excitonen, mindestens einer Blockschicht für Löcher/Excitonen, mindestens einer Loch-Injektionsschicht, mindestens einer Lochleiterschicht, mindestens einer Elektroneninjektionsschicht und mindestens einer Elektronenleiterschicht, wobei
in dem Fall, wenn die Verbindung der allgemeinen Formel (I) ausschließlich in der Licht-emittierenden Schicht oder in der Licht-emittierenden Schicht und in der Lochleiterschicht vorliegt und die Gruppe X NR¹ bedeutet, mindestens einer der Reste R¹ bis R⁷, R^{a} oder R^{b} in den Verbindungen der Formel (I) mindestens ein Heteroatom enthält.

10. Verwendung von Verbindungen der allgemeinen Formel (I) gemäß einem der Ansprüche 1 bis 8 als Matrixmaterial, Loch-/Excitonenblockermaterial und/oder Elektronen-/Excitonenblockermaterial und/oder Loch-Injektionsmaterial und/oder Elektronen-Injektionsmaterial und/oder Lochleitermaterial und/oder Elektronenleitermaterial in einer organischen Leuchtdiode, wobei
in dem Fall, wenn die Verbindung der allgemeinen Formel (I) ausschließlich in der Licht-emittierenden Schicht oder in der Licht-emittierenden Schicht und in der Lochleiterschicht vorliegt und die Gruppe X NR¹ bedeutet, mindestens einer der Reste R¹ bis R⁷, R^{a} oder R^{b} in den Verbindungen der Formel (I) mindestens ein Heteroatom enthält.

11. Vorrichtung ausgewählt aus der Gruppe bestehend aus stationären Bildschirmen wie Bildschirmen von Computern, Fernsehern, Bildschirmen in Druckern, Küchengeräten sowie Reklametafeln, Beleuchtungen, Hinweistafeln und mobilen Bildschirmen wie Bildschirmen in Handys, Laptops, Digitalkameras, Fahrzeugen sowie Zielanzeigen an Bussen und Bahnen und Beleuchtungseinheiten enthaltend mindestens eine Verbindung gemäß einem der Ansprüche 1 bis 8.

## Claims

1. Compound of the general formula I: 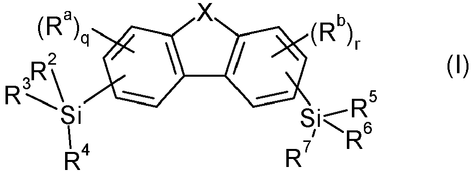 wherein:
X is NR¹, S, O, PR¹, SO₂ or SO;
R¹ is substituted or unsubstituted C₆-C₃₀ aryl, preferably substituted or unsubstituted C₆-C₁₀ aryl, more preferably substituted or unsubstituted phenyl, wherein the substituent is selected from the group consisting of: C₁-C₂₀ alkyl, unsubstituted C₆-C₃₀ aryl, C₆-C₃₀ aryl, which is substituted with one or more C₁-C₂₀ alkyloxy, CN, CF₃, F, C₆-C₃₀ aryloxy, or C₆-C₃₀ alkylthio, or C₁-C₂₀ alkyloxy, C₆-C₃₀ aryloxy, C₁-C₂₀ alkylthio, C₆-C₃₀ arylthio, SiR¹⁴R¹⁵R¹⁶, halogen radicals, halogenated C₁-C₂₀ alkyl radicals, carbonyl (-CO(R¹⁴)), carbonylthio (-C = O(SR¹⁴)), carbonyloxy (C = O(OR¹⁴)), oxycarbonyl (-OC = O(R¹⁴)), thiocarbonyl (-SC = O(R¹⁴), amino (-NR¹⁴R¹⁵), OH, pseudo halogen radicals, amido (-C = O (NR¹⁴)), -NR¹⁴C = O (R¹⁵), phosphonate (-P(O) (OR¹⁴)₂), phosphate (-OP(O) (OR¹⁴)₂), phosphine (-PR¹⁴R¹⁵), phosphine oxide (-P(O) (OR¹⁴)₂), sulfate (-OS(O)₂OR¹⁴), sulfoxide (-S(O)R¹⁴), sulfonate (-S(O)₂OR¹⁴), sulfonyl (-S(O)₂R¹⁴), sulfonamide (-S(O)₂NR¹⁴R¹⁵), NO₂, boronic esters (-OB(OR¹⁴)₂), imino (-C = NR¹⁴R¹⁵), borane radicals, stannane radicals, hydrazine radicals, hydrazone radicals, oxime radicals, nitroso groups, diazo groups, vinyl groups, (=sulfonate) and boronic acid groups, sulfoximes, alanes, germanes, boroximines and borazines; or substituted or unsubstituted heteroaryl having 5 - 30 ring atoms;
R², R³, R⁴, R⁵, R⁶, R⁷ are each independently of each other substituted or unsubstituted C₁-C₂₀ alky, substituted or unsubstituted C₆-C₃₀ aryl, or a structure of the general Formula (c) 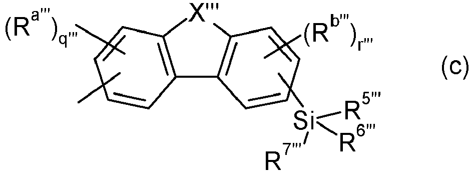
R^{a}, R^{b} are each independently of each other substituted or unsubstituted C₁-C₂₀ alky, substituted or unsubstituted C₆-C₃₀ aryl, substituted or unsubstituted heteroaryl having 5 to 30 ring atoms or a substituent with donor or acceptor action, selected from the group consisting of: C₁-C₂₀ alkyloxy, C₆-C₃₀ aryloxy, C₁-C₂₀ alkylthio, C₆-C₃₀ arylthio, SiR¹⁴R¹⁵R¹⁶, halogen radicals, halogenated C₁-C₂₀ alkylradicals, carbonyl (-CO(R¹⁴)), carbonylthio (-C = O(SR¹⁴)), carbonyloxy (C = O(OR¹⁴)), oxycarbonyl (-OC = O(R¹⁴)), thiocarbonyl (-SC = O(R¹⁴), amino (-NR¹⁴R¹⁵), OH, pseudo halogen radicals, amido (-C = O(NR¹⁴)), -NR¹⁴C = O(R¹⁵), phosphonate (-P(O)(OR¹⁴)₂), phosphate (-OP(O)(OR¹⁴)₂), phosphine (-PR¹⁴R¹⁵), phosphine oxide (-P(O)(OR¹⁴)₂), sulfate (-OS(O)₂OR¹⁴), sulfoxide (-S(O)R¹⁴), sulfonate (-S(O)₂OR¹⁴), sulfonyl (-S(O)₂R¹⁴), sulfonamide (-S(O)₂NR¹⁴R¹⁵), NO₂, boronic esters (-OB(OR¹⁴)₂), imino (-C = NR¹⁴R¹⁵), borane radicals, stannane radicals, hydrazine radicals, hydrazone radicals, oxime radicals, nitroso groups, diazo groups, vinyl groups, sulfoximes, alanes, germanes, boroximines and borazines;
R¹⁴, R¹⁵, R¹⁶ are each independently of each other substituted or unsubstituted C₁-C₂₀ alky, or substituted or unsubstituted C₆-C₃₀ aryl;
q, r are each independently 0, 1, 2 or 3; wherein when q or r, respectively, are 0, all substitutable positions of the aryl radical are substituted by hydrogen,
wherein in the group of Formula (c) the radicals and indices X"', R⁵'" , R⁶'", R⁷'", R^{a}"', R^{b}"', q'" and r'" are each independently of each other as defined for the radicals and indices of compounds of the general Formula (I) X, R⁵, R⁶, R⁷, R^{a}, R^{b}, q and r,
wherein following compounds are excluded:

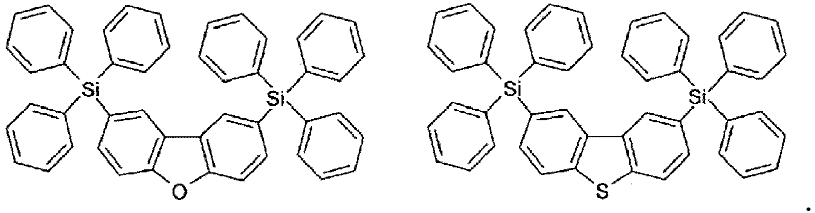

2. Compound according to claim 1, **characterized in that** at least one of the R², R³ or R⁴ radicals and/or at least one of the R⁵, R⁶ or R⁷ radicals is substituted or unsubstituted C₆-C₃₀ aryl.

3. Compound according to claim 1 or 2, **characterized in that** the compound of the general formula (I) is a 3,6-disilyl-substituted compound of the general Formula (la): 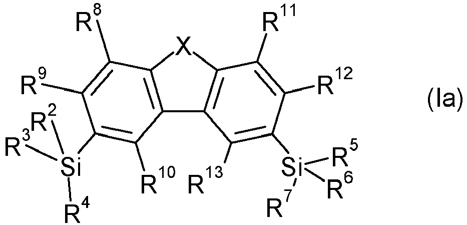 wherein:
X is NR¹, S, O, PR¹, SO₂ or SO; preferably NR¹, S or O; more preferably
NR¹; R¹ is substituted or unsubstituted C₁-C₂₀ alkyl, substituted or unsubstituted C₆-C₃₀ aryl or substituted or unsubstituted heteroaryl having 5 to 30 ring atoms; preferably substituted or unsubstituted C₆-C₃₀ aryl or unsubstituted C₁-C₂₀ alkyl, more preferably substituted or unsubstituted C₆-C₁₀ aryl or unsubstituted C₁-C₂₀ alkyl, most preferably substituted or unsubstituted phenyl;
R², R³, R⁴, R⁵, R⁶, R⁷ are each independently of each other substituted or unsubstituted C₁-C₂₀ alkyl, or substituted or unsubstituted C₆-C₃₀ aryl, or a structure of the general Formula (c); preferably at least one of the radicals R², R³, R⁴ and/or at least one of the radicals R⁵, R⁶ or R⁷ is substituted or unsubstituted C₆-C₃₀ aryl, more preferably substituted or unsubstituted C₆-C₁₀ aryl, most preferably substituted or unsubstituted phenyl and /or one of the radicals R², R³ or R⁴ and/or one of the radicals R⁵, R⁶ or R⁷ is a radical of structure (c);
R⁸, R⁹, R¹⁰, R¹¹, R¹², R¹³ are each independently of each other hydrogen or are as defined for R^{a} and R^{b}, i.e. are each independently of each other substituted or unsubstituted C₁-C₂₀ alkyl, or substituted or unsubstituted C₆-C₃₀ aryl, substituted or unsubstituted heteroaryl having 5 to 30 ring atoms, or a substituent with donor or acceptor action; preferably hydrogen, substituted or unsubstituted C₁-C₆ alkyl, or substituted or unsubstituted C₆-C₁₀ aryl, or SiR¹⁴R¹⁵R¹⁶; wherein R¹⁴, R¹⁵ and R¹⁶ are preferably each independently of each other substituted or unsubstituted C₁-C₂₀ alkyl or substituted or unsubstituted phenyl; more preferably at least one of the radicals R¹⁴, R¹⁵ and R¹⁶ is substituted or unsubstituted phenyl, most preferably at least one of the radicals R¹⁴, R¹⁵ and R¹⁶ is substituted phenyl, wherein suitable substituents are those mentioned above.

4. Compound according to any one of the claims 1 to 3, **characterized in that** the radicals R¹ to R⁷, R^{a} and R^{b} and group X are each defined as follows:
X is NR¹;
R¹ is substituted or unsubstituted C₆-C₃₀ aryl, preferably substituted or unsubstituted C₆-C₁₀ aryl, most preferably substituted or unsubstituted phenyl;
R², R³, R⁴, R⁵, R⁶, R⁷ are each independently of each other substituted or unsubstituted C₁-C₂₀ alkyl, substituted or unsubstituted C₆-C₃₀ aryl, or a structure of the general formula (c), preferably each independently substituted or unsubstituted C₁-C₆ alkyl or substituted or unsubstituted C₆-C₁₀ aryl, more preferably substituted or unsubstituted C₁-C₆ alkyl substituted or unsubstituted phenyl; wherein, in one embodiment, at least one of the radicals R², R³ or R⁴ and/or at least one of the radicals R⁵, R⁶ or R⁷ is substituted or unsubstituted C₆-C₃₀ aryl, preferably substituted or unsubstituted C₆-C₁₀ aryl, more preferably substituted or unsubstituted phenyl;
R⁸, R⁹, R¹⁰, R¹¹, R¹², R¹³ are each independently of each other hydrogen or are as defined for R^{a} and R^{b}, i.e. are each independently substituted or unsubstituted C₁-C₂₀ alkyl, or substituted or unsubstituted C₆-C₃₀ aryl, substituted or unsubstituted heteroaryl having 5 to 30 ring atoms, or a substituent with donor or acceptor action; preferably hydrogen, substituted or unsubstituted C₁-C₆ alkyl, or substituted or unsubstituted C₆-C₁₀ aryl, or SiR¹⁴R¹⁵R¹⁶, most preferably methyl, ethyl, phenyl, CF₃ or SiR¹⁴R¹⁵R¹⁶;
R¹⁴, R¹⁵, R¹⁶ are each independently of each other substituted or unsubstituted C₁-C₂₀ alkyl, or substituted or unsubstituted C₆-C₃₀ aryl, preferably substituted or unsubstituted C₁-C₆ alkyl, or substituted or unsubstituted C₆-C₁₀ aryl, wherein R¹⁴, R¹⁵ and R¹⁶ are more preferably each independently of each other substituted or unsubstituted C₁-C₂₀ alkyl or substituted or unsubstituted phenyl; more preferably at least one of the radicals R¹⁴, R¹⁵ and R¹⁶ is substituted or unsubstituted phenyl; most preferably at least one of the radicals R¹⁴, R¹⁵ and R¹⁶ is substituted phenyl.

5. Compound of any of claims 1 to 4, **characterized in that** the radical R¹ and/or at least one of the radicals from the group R², R³ and R⁴ and/or at least one radical from the group of R⁵, R⁶ and R⁷ are independently substituted or unsubstituted C₆ aryl of the following Formula: 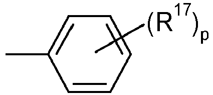 wherein:
p is 0, 1, 2, 3, 4 or 5, preferably 0, 1, 2 or 3, more preferably 0, 1, 2;
R¹⁷ is hydrogen, substituted or unsubstituted C₁-C₂₀ alkyl, substituted or unsubstituted C₆-C₃₀ aryl, substituted or unsubstituted heteroaryl having 5 to 30 ring atoms, a substituent with donor or acceptor action, or a radical of the general Formula a or b 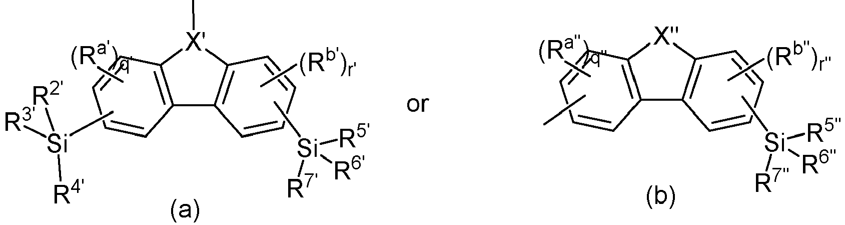
wherein,
X' is N or P, and the radicals and indices X", R^{2'}, R^{3'}, R^{4'}, R^{5'}, R^{5"}, R^{6'}, R^{6"}, R^{7'}, R^{7"}, R^{a'}, R^{a"}, R^{b'}, R^{b"}, q', q", r' and r" are each independently as defined for the radicals and indices X, R², R³, R⁴, R⁵, R⁶, R⁷, R^{a}, R^{b}, q and r; or one of the radicals R², R³ or R⁴ and/or one of the radicals R⁵, R⁶ or R⁷ is a radical of the general formula (c) 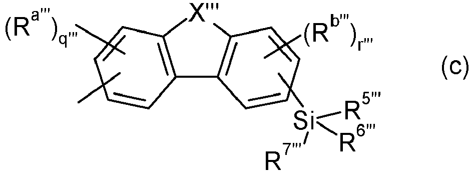 wherein, the radicals and indices X"', R^{5"'}, R^{6"'}, R^{7"'}, R^{a"'}, R^{b"}, q'" and r'" are each independently of each other as defined for the radicals and indices X, R⁵, R⁶, R⁷, R^{a}, R^{b}, q and r.

6. Compound according to any one of claims 1 to 5, **characterized in that**, if in a compound of general Formula (I) the group X is NR¹, at least one of the radicals R¹ to R⁷, R^{a} or R^{b} in the compounds according to Formula (I) contains at least one hetero atom.

7. Compound according to any one of claims 1 to 6, **characterized in that** the compound of general Formula (I) has at least one radical R¹, R², R³, R⁴, R⁵, R⁶ or R⁷ which is selected from the group consisting of substituted or unsubstituted C₁-C₂₀ alkyl, heteroaryl having 5 to 30 ring atoms, unsubstituted C₆-C₃₀ aryl, alkyl-substituted C₆-C₃₀ aryl, C₆-C₃₀ aryl substituted with at least one substituent with donor action, C₆-C₃₀ aryl substituted with a heteroaryl having 5 to 30 ring atoms and a substituent with donor action.

8. Compound according to any one of claims 1 to 6, **characterized in that** the compound of general Formula (I) has at least one radical R¹, R², R³, R⁴, R⁵, R⁶ or R⁷ which is selected from the group consisting of C₁-C₂₀ alkyl substituted with at least one substituent with acceptor action, C₆-C₃₀ aryl substituted with at least one substituent with acceptor action, C₆-C₃₀ aryl substituted with at least one substituent with acceptor action, C₆-C₃₀ aryl substituted with a heteroaryl having 5 to 30 ring atoms and a substituent with acceptor action.

9. Layer comprising at least one compound of Formula (I) according to any one of claims 1 to 8, wherein the layer is selected from the group consisting of: at least one light-emitting layer, at least one electron/exciton blocking layer, at least one hole/exciton blocking layer, at least one hole injection layer, at least one hole transporting layer, at least one electron injection layer, and at least one electron transporting layer, wherein when the compound of Formula (I) is present only in the light-emitting layer or in the light-emitting layer and the hole transporting layer and the group X is NR¹, at least one of the radicals R¹ to R⁷, R^{a} or R^{b} in the compound of general Formula (I) comprises at least one heteroatom.

10. A use of compounds of the general Formula (I) according to any one of claims 1 to 8 as a matrix material, hole/exciton blocking material and/or electron/exciton blocking material and/or hole injection material and/or electron injection material and/or hole transporting material and/or electron transporting material in an organic light-emitting diode, wherein
when the compound of general Formula (I) is present only in the light-emitting layer or in the light-emitting layer and in the hole transporting layer and group X is NR¹, at least one of the radicals R¹-R⁷, R^{a} or R^{b} in the compounds of Formula (I) comprises at least one heteroatom.

11. A device selected from the group consisting of stationary visual display units such as display units of computers, televisions, visual display units in printers, kitchen appliances and billboards, illuminations, signs and mobile visual displays, such as visual display units in mobile phones, digital cameras, vehicles and destination displays on busses and trains, and illumination units comprising at least one of the compounds according to any one of claims 1 to 8.

## Revendications

1. Composé de formule générale I : 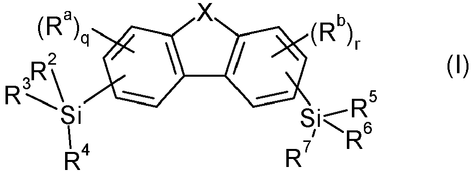 dans laquelle :
X représente NR¹, S, O, PR¹, SO₂ ou SO,
R¹ représente un aryle en C₆ à C₃₀ substitué ou non substitué, de manière préférée un aryle en C₆ à C₁₀ substitué ou non substitué, de manière particulièrement préférée un phényle substitué ou non substitué, dans lequel le substituant est choisi parmi le groupe constitué d'un alkyle en C₁ à C₂₀, d'un aryle en C₆ à C₃₀ non substitué, d'un aryle en C₆ à C₃₀ qui est substitué par un ou plusieurs groupes alcoxy en C₁ à C₂₀, CN, CF₃, F, des groupes aryloxy en C₆ à C₃₀ ou des groupes alkylthio en C₆ à C₃₀, ou un alcoxy en C₁ à C₂₀, un aryloxy en C₆ à C₃₀, un alkylthio en C₁ à C₂₀, un arylthio en C₆ à C₃₀, SiR¹⁴R¹⁵R¹⁶, des radicaux halogène, des radicaux alkyle en C₁ à C₂₀ halogénés, un carbonyle (-CO(R¹⁴)), un carbonylthio (- C = O (SR¹⁴)), un carbonyloxy (- C = O(OR¹⁴)), un oxycarbonyle (- OC = O(R¹⁴)), un thiocarbonyle (- SC = O(R¹⁴)), un amino (-NR¹⁴R¹⁵), OH, des radicaux pseudohalogène, un amido (- C = O (NR¹⁴)), -NR¹⁴C = O (R¹⁵), un phosphonate (-P(O) (OR¹⁴)₂, un phosphate (-OP(O) (OR¹⁴)₂), une phosphine (-PR¹⁴R¹⁵), un oxyde de phosphine (-P(O)R¹⁴₂), un sulfate (-OS(O)₂OR¹⁴), un sulfoxyde (-S(O)R¹⁴), un sulfonate (-S(O)₂OR¹⁴), un sulfonyle (-S(O)₂R¹⁴), un sulfonamide (-S(O)₂NR¹⁴R¹⁵), NO₂, des esters d'acide borique (-OB(OR¹⁴)₂), un imino (-C = NR¹⁴R¹⁵)), des radicaux boranne, des radicaux stannane, des radicaux hydrazine, des radicaux hydrazone, des radicaux oxime, des groupes nitroso, des groupes diazo, des groupes vinyle, (=sulfonate) et des groupes d'acide borique, une sulfoximine, un alane, un germane, une boroxime et une borazine, ou un hétéroaryle substitué ou non substitué possédant 5 à 30 atomes de cycle,
R², R³, R⁴, R⁵, R⁶, R⁷ représentent, indépendamment les uns des autres, un alkyle en C₁ à C₂₀ substitué ou non substitué ou un aryle en C₆ à C₃₀ substitué ou non substitué ou une structure de formule générale (c), 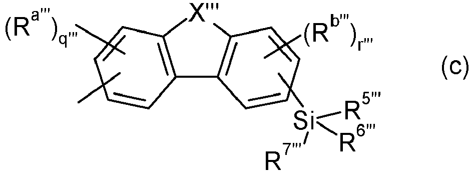
R^{a}, R^{b} représentent, indépendamment l'un de l'autre, un alkyle en C₁ à C₂₀ substitué ou non substitué, un aryle en C₆ à C₃₀ substitué ou non substitué, un hétéroaryle substitué ou non substitué possédant 5 à 30 atomes de cycle ou un substituant à effet donneur ou accepteur, choisi parmi le groupe constitué de : alcoxy en C₁ à C₂₀, aryloxy en C₆ à C₃₀, alkylthio en C₁ à C₂₀, arylthio en C₆ à C₃₀, SiR¹⁴R¹⁵R¹⁶, radicaux halogène, radicaux alkyle en C₁ à C₂₀ halogénés, carbonyle (-CO(R¹⁴)), carbonylthio (- C = O (SR¹⁴)), carbonyloxy (-C = O(OR¹⁴)), oxycarbonyle (- OC = O(R¹⁴)), thiocarbonyle (- SC = O(R¹⁴)), amino (-NR¹⁴R¹⁵), OH, radicaux pseudo-halogène, amido (- C = O (NR¹⁴)),-NR¹⁴C = O (R¹⁵), phosphonate (-P(O) (OR¹⁴)₂, phosphate (-OP(O) (OR¹⁴)₂), phosphine (-PR¹⁴R¹⁵), oxyde de phosphine (-P(O)R¹⁴₂), sulfate (-OS(O)₂OR¹⁴), sulfoxyde (-S(O)R¹⁴), sulfonate (-S(O)₂OR¹⁴), sulfonyle (-S(O)₂R¹⁴, sulfonamide (-S(O)₂NR¹⁴R¹⁵), NO₂, esters d'acide borique (-OB(OR¹⁴)₂), imino (-C = NR¹⁴R¹⁵)), radicaux borane, radicaux stannane, radicaux hydrazine, radicaux hydrazone, radicaux oxime, groupes nitroso, groupes diazo, groupes vinyle, sulfoximines, alanes, germanes, boroximes et borazines,
R¹⁴, R¹⁵, R¹⁶ représentent, indépendamment les uns des autres, un alkyle en C₁ à C₂₀ substitué ou non substitué ou un aryle en C₆ à C₃₀ substitué ou non substitué,
q, r représentent, indépendamment l'un de l'autre, 0, 1, 2 ou 3, dans lequel dans le cas où q ou r représente 0, toutes les positions substituables du radical alkyle sont substituées par l'hydrogène,
dans lequel les radicaux et les indices dans le groupe de formule (c) X"', R⁵"', R⁶"', R⁷"', R^{a}"', R^{b}'", q'" et r'" ont, indépendamment les uns des autres, les significations indiquées pour les radicaux et les indices des composés de formule générale (I) X, R⁵, R⁶, R⁷, R^{a}, R^{b}, q et r,
dans lequel les composés suivants sont exclus :

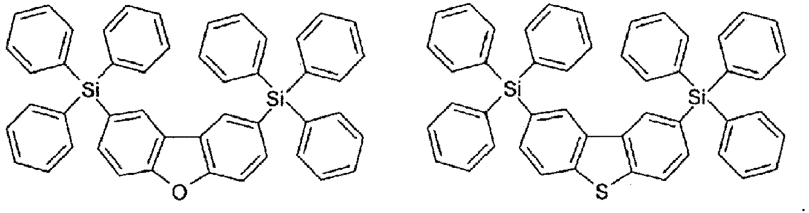

2. Composé selon la revendication 1, **caractérisé en ce qu'**au moins un des radicaux R², R³ ou R⁴ et/ou au moins un des radicaux R⁵, R⁶ ou R⁷ représente un aryle en C₆ à C₃₀ substitué ou non substitué.

3. Composé selon la revendication 1 ou 2, **caractérisé en ce que** le composé de formule générale (I) est un composé 3,6-disilyl-substitué de formule générale (la) : 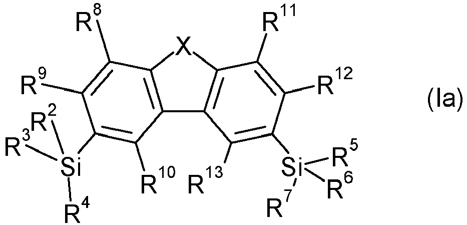 dans laquelle :
X représente NR¹, S, O, PR¹, SO₂ ou SO, de manière préférée NR¹, S ou O, de manière particulièrement préférée NR¹,
R¹ représente un alkyle en C₁ à C₂₀ substitué ou non substitué, un aryle en C₆ à C₃₀ substitué ou non substitué ou un hétéroaryle substitué ou non substitué possédant 5 à 30 atomes de cycle, de manière préférée un aryle en C₆ à C₃₀ substitué ou non substitué ou un alkyle en C₁ à C₂₀ non substitué, de manière particulièrement préférée un aryle en C₆ à C₁₀ substitué ou non substitué ou un alkyle en C₁ à C₂₀ non substitué, de manière tout particulièrement préférée un phényle substitué ou non substitué,
R², R³, R⁴, R⁵, R⁶, R⁷ représentent, indépendamment les uns des autres, un alkyle en C₁ à C₂₀ substitué ou non substitué ou un aryle en C₆ à C₃₀ substitué ou non substitué ou une structure de formule générale (c), de manière préférée, au moins un des radicaux R², R³ ou R⁴ et/ou au moins un des radicaux R⁵, R⁶ ou R⁷ représente un aryle en C₆ à C₃₀ substitué ou non substitué, de manière particulièrement préférée un aryle en C₆ à C₁₀ substitué ou non substitué, de manière tout particulièrement préférée un phényle substitué ou non substitué et/ou un des radicaux R², R³ ou R⁴ et/ou un des radicaux R⁵, R⁶ ou R⁷ est un radical de la structure (c),
R⁸, R⁹, R¹⁰, R¹¹, R¹², R¹³ représentent, indépendamment les uns des autres, l'hydrogène ou ont les significations indiquées pour R^{a} et R^{b}, c'est-à-dire qu'ils représentent, indépendamment les uns des autres, un alkyle en C₁ à C₂₀ substitué ou non substitué, un aryle en C₆ à C₃₀ substitué ou non substitué, un hétéroaryle substitué ou non substitué possédant 5 à 30 atomes de cycle ou un substituant à effet donneur ou accepteur, de préférence l'hydrogène, un alkyle en C₁ à C₆ substitué ou non substitué, un aryle en C₆ à C₁₀ substitué ou non substitué ou SiR¹⁴R¹⁵R¹⁶, dans lequel R¹⁴, R¹⁵ et R¹⁶ représentent de manière préférée, indépendamment les uns des autres, un alkyle en C₁ à C₂₀ substitué ou non substitué ou un phényle substitué ou non substitué, de manière particulièrement préférée, au moins un des radicaux R¹⁴, R¹⁵ ou R¹⁶ est un phényle substitué ou non substitué, de manière tout particulièrement préférée, au moins un des radicaux R¹⁴,R¹⁵ et R¹⁶ est un phényle substitué, dans lequel des substituants adaptés sont indiqués ci-dessus.

4. Composé selon l'une des revendications 1 à 3, **caractérisé en ce que** les radicaux R¹ à R⁷, R^{a} et R^{b} ainsi que le groupe X ont les significations suivantes :
X représente NR¹,
R¹ représente un aryle en C₆ à C₃₀ substitué ou non substitué, de préférence un aryle en C₆ à C₁₀ substitué ou non substitué, de manière particulièrement préférée un phényle substitué ou non substitué,
R², R³, R⁴, R⁵, R⁶, R⁷ représentent, indépendamment les uns des autres, un alkyle en C₁ à C₂₀ substitué ou non substitué ou un aryle en C₆ à C₃₀ substitué ou non substitué, ou une structure de formule générale (c), de manière préférée, indépendamment les uns des autres, un alkyle en C₁ à C₆ substitué ou non substitué ou un aryle en C₆ à C₁₀ substitué ou non substitué, de manière particulièrement préférée, un alkyle en C₁ à C₆ substitué ou non substitué ou un phényle substitué ou non substitué, dans lequel dans un mode de réalisation, au moins un des radicaux R², R³ ou R⁴ et/ou au moins un des radicaux R⁵, R⁶ ou R⁷ représente un aryle en C₆ à C₃₀ substitué ou non substitué, de préférence un aryle en C₆ à C₁₀ substitué ou non substitué, de manière particulièrement préférée un phényle substitué ou non substitué,
R⁸, R⁹, R¹⁰, R¹¹, R¹², R¹³ représentent, indépendamment les uns des autres, l'hydrogène ou ont les significations indiquées pour R^{a} et R^{b}, c'est-à-dire qu'ils représentent, indépendamment les uns des autres, un alkyle en C₁ à C₂₀ substitué ou non substitué, un aryle en C₆ à C₃₀ substitué ou non substitué, un hétéroaryle substitué ou non substitué possédant 5 à 30 atomes de cycle ou un substituant à effet donneur ou accepteur, de manière préférée l'hydrogène, un alkyle en C₁ à C₆ substitué ou non substitué, un aryle en C₆ à C₁₀ substitué ou non substitué ou SiR¹⁴R¹⁵R¹⁶, de manière particulièrement préférée l'hydrogène, un méthyle, un éthyle, un phényle, CF₃ ou SiR¹⁴R¹⁵R¹⁶,
R¹⁴, R¹⁵, R¹⁶ représentent, indépendamment les uns des autres, un alkyle en C₁ à C₂₀ substitué ou non substitué ou un aryle en C₆ à C₃₀ substitué ou non substitué, de manière préférée un alkyle en C₁ à C₆ substitué ou non substitué ou un aryle en C₆ à C₁₀ substitué ou non substitué, dans lequel R¹⁴, R¹⁵ et R¹⁶ représentent de manière particulièrement préférée, indépendamment les uns des autres, un alkyle en C₁ à C₂₀ substitué ou non substitué ou un phényle substitué ou non substitué, de manière particulièrement préférée, au moins un des radicaux R¹⁴,R¹⁵ ou R¹⁶ représente un phényle substitué ou non substitué, de manière tout particulièrement préférée, au moins un des radicaux R¹⁴,R¹⁵ et R¹⁶ représente un phényle substitué.

5. Composé selon l'une des revendications 1 à 4, **caractérisé en ce que** le radical R¹, et/ou au moins un des radicaux des groupes R², R³ et R⁴ et/ou au moins un des radicaux des groupes R⁵, R⁶ et R⁷ représentent, indépendamment les uns des autres, un aryle en C₆ substitué ou non substitué de formule générale : 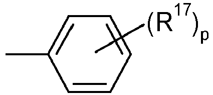 dans laquelle :
p représente 0, 1, 2, 3, 4 ou 5, de manière préférée 0, 1, 2 ou 3, de manière particulièrement préférée 0, 1 ou 2,
R¹⁷ représente l'hydrogène, un alkyle en C₁ à C₂₀ substitué ou non substitué, un aryle en C₆ à C₃₀ substitué ou non substitué ou un hétéroaryle substitué ou non substitué possédant 5 à 30 atomes de cycle, un substituant à effet donneur ou accepteur, ou un radical de formule générale a ou b 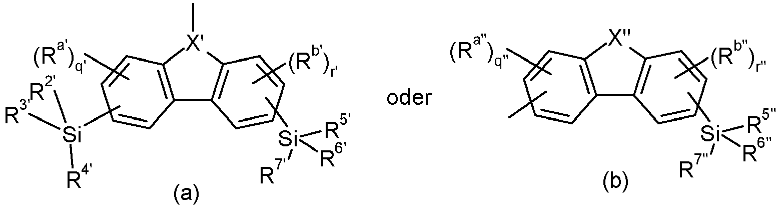
dans laquelle
X' représente N ou P, et
les radicaux et les indices X", R^{2'}, R^{3'}, R^{4'}, R^{5'}, R^{5"}, R^{6'}, R^{6"}, R^{7'}, R^{7"}, R^{a'}, R^{a"}, R^{b'}, R^{b"}, q', q", r' et r" ont, indépendamment les uns des autres, les significations indiquées pour les radicaux et les indices X, R², R³, R⁴, R⁵, R⁶, R⁷, R^{a}, R^{b}, q et r,
ou
l'un des radicaux R², R³ ou R⁴ et/ou l'un des radicaux R⁵, R⁶ ou R⁷ représente un radical de formule générale c, 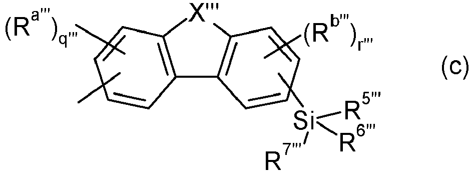
dans laquelle les radicaux et les indices X"', R^{5"'}, R^{6"'}, R^{7"'} , R^{a'}, R^{b"'}, q'" et r'" ont, indépendamment les uns des autres, les significations indiquées pour les radicaux et les indices X, R⁵, R⁶, R⁷, R^{a}, R^{b}, q et r.

6. Composé selon l'une des revendications 1 à 5, **caractérisé en ce que** lorsque, dans le composé de formule générale (I), le groupe X représente NR¹, au moins un des radicaux R¹ à R⁷, R^{a} ou R^{b} dans les composés de formule (I) contient au moins un hétéroatome.

7. Composé selon l'une des revendications 1 à 6, **caractérisé en ce que** le composé de formule (I) comporte au moins un radical R¹, R², R³, R⁴, R⁵, R⁶ ou R⁷ choisi parmi le groupe constitué d'un alkyle en C₁ à C₂₀ substitué ou non substitué, un hétéroaryle possédant 5 à 30 atomes de cycle, un aryle en C₆ à C₃₀ non substitué, un aryle en C₆à C₃₀ substitué par un alkyle, un aryle en C₆ à C₃₀ substitué par au moins un substituant à effet donneur, un aryle en C₆ à C₃₀ substitué par un hétéroaryle possédant 5 à 30 atomes de cycle et un substituant à effet donneur.

8. Composé selon l'une des revendications 1 à 6, **caractérisé en ce que** le composé de formule (I) comporte au moins un radical R¹, R², R³, R⁴, R⁵, R⁶ ou R⁷ choisi parmi le groupe constitué d'un alkyle en C₁ à C₂₀ substitué par au moins un substituant à effet accepteur, un aryle en C₆ à C₃₀ substitué par au moins un substituant à effet accepteur, un aryle en C₆ à C₃₀ substitué par au moins un radical hétéroaryle possédant 5 à 30 atomes de cycle et un substituant à effet accepteur.

9. Couche contenant au moins un composé de formule (I) selon l'une des revendications 1 à 8, dans laquelle la couche est choisie parmi le groupe constitué d'au moins une couche électroluminescente, d'au moins une couche de blocage pour des électrons/excitons, d'au moins une couche de blocage pour des trous/excitons, d'au moins une couche d'injection de trous, d'au moins une couche de transport de trous, d'au moins une couche d'injection d'électrons et d'au moins une couche de transport d'électrons, dans laquelle dans le cas où le composé de formule générale (I) se trouve uniquement dans la couche électroluminescente ou dans la couche électroluminescente et dans la couche de transport de trous et où le groupe X représente NR¹, au moins un des radicaux R¹ à R⁷, R^{a} ou R^{b} dans les composés de formule (I) contient au moins un hétéroatome.

10. Utilisation de composés de formule générale (I) selon l'une des revendications 1 à 8 en tant que matériau de matrice, matériau de blocage de trous/excitons et/ou matériau de blocage d'électrons/excitons et/ou matériau d'injection de trous et/ou matériau d'injection d'électrons et/ou matériau de transport de trous et/ou matériau de transport d'électrons dans une diode électroluminescente organique, dans laquelle dans le cas où le composé de formule générale (I) se trouve uniquement dans la couche électroluminescente ou dans la couche électroluminescente et dans la couche de transport de trous et où le groupe X représente NR¹, au moins un des radicaux R¹ à R⁷, R^{a} ou R^{b} dans les composés de formule (I) contient au moins un hétéroatome.

11. Dispositif choisi parmi le groupe constitué d'écrans fixes tels que des écrans d'ordinateurs, des téléviseurs, des écrans d'imprimantes, des appareils de cuisine ainsi que des panneaux publicitaires, des éclairages, des panneaux d'information et des écrans mobiles tels que des écrans de téléphones portables, d'ordinateurs portables, d'appareils photo numériques, de véhicules ainsi que des affichages de destination dans des bus et des trains et des unités d'éclairage contenant au moins un composé selon l'une des revendications 1 à 8.
